(19) Europäisches Patentamt
European Patent Office
Office européen des brevets

(11) Publication number: 0 374 952 A2

(12) EUROPEAN PATENT APPLICATION

(21) Application number: 89123799.2

(51) Int. Cl.5: C07D 493/08, A61K 31/41

(22) Date of filing: 22.12.89

Claims for the following Contracting States: ES + GR.

(30) Priority: 23.12.88 US 288826

(43) Date of publication of application: 27.06.90 Bulletin 90/26

(84) Designated Contracting States: AT BE CH DE ES FR GB GR IT LI LU NL SE

(71) Applicant: E.R. SQUIBB & SONS, INC.
P.O.Box 4000
Princeton New Jersey 08543-4000(US)

(72) Inventor: Sher, Philip M.
1808 Fox Run Drive
Plainsboro New Jersey(US)
Inventor: Stein, Philip D.
64 Carter Road
Princeton New Jersey(US)
Inventor: Floyd, David
R.R. 1, Box 404 M
Pennington New Jersey(US)
Inventor: Hall, Steven E.
57 Colleen Circle
Trenton New Jersey(US)

(74) Representative: Vossius & Partner
Siebertstrasse 4 P.O. Box 86 07 67
D-8000 München 86(DE)

# (54) 7-Oxabicycloheptyl substituted heterocyclic amide prostaglandin analogs useful in the treatment of thrombotic and vasospastic disease.

(57) 7-Oxabicycloheptane substituted prostaglandin analogs which are thromboxane $A_2$ ($TxA_2$) receptor antagonists or combined thromboxane $A_2$ receptor antagonists/thromboxane synthase inhibitors useful, for example, in treating thrombotic and vasospastic disease have the structural formula

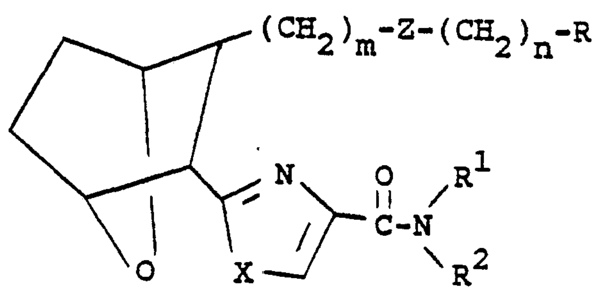

wherein m is 1, 2 or 3; n is 0, 1, 2, 3 or 4; Z is $-(CH_2)_2-$ or $-CH=CH-$, with the proviso when Z is $-CH=CH-$, n is 1, 2, 3 or 4; R is $CO_2H$, $CO_2$lower alkyl, $CH_2OH$, $CO_2$alkali metal, $CONHSOR^3$, $CONHR^{3a}$ or $-CH_2$-5-tetrazolyl, X is O, S or NH; and where $R^1$, $R^2$, $R^3$ and $R^{3a}$ are as defined herein.

EP 0 374 952 A2

# 7-OXABICYCLOHEPTYL SUBSTITUTED HETEROCYCLIC AMIDE PROSTAGLANDIN ANALOGS USEFUL IN THE TREATMENT OF THROMBOTIC AND VASOSPASTIC DISEASE

The present invention relates to 7-oxabicycloheptyl substituted heterocyclic amide prostaglandin analogs which are thromboxane $A_2$ ($TXA_2$) receptor antagonists or combined thromboxane $A_2$ receptor antagonists/thromboxane synthase inhibitors useful, for example, in the treatment of thrombotic and/or vasospastic disease, and have good duration of action. These compounds have the structural formula

I

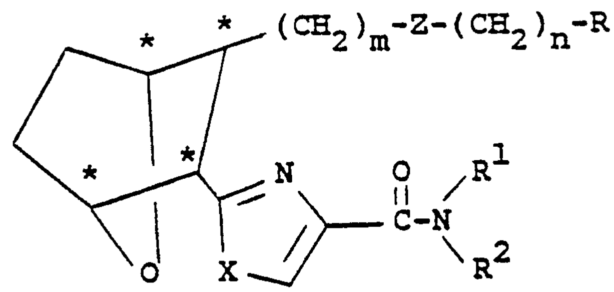

and including all stereoisomers thereof, wherein
m is 1, 2 or 3; n is 0, 1, 2, 3 or 4;
Z is $-(CH_2)_2-$ or $-CH=CH-$
and when Z is $-CH=CH-$, n is 1,2,3, or 4;
R is $CO_2H$, $CO_2$lower alkyl, $CO_2$alkali metal, $CH_2OH$, $CONHSO_2R^3$, $CONHR^{3a}$, or

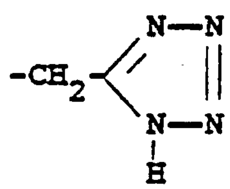

($-CH_2$-5-tetrazolyl),
X is O, S or NH;
$R^1$ is lower alkyl, lower alkenyl, lower alkynyl, aralkyl, aryl, cycloalkyl, or cycloalkylalkyl each of $R^1$ being unsubstituted or optionally substituted with an alkyl, aryl, cycloalkyl, or cycloalkylalkyl group;
$R^2$ is hydrogen, lower alkyl, aryl, or aralkyl; or
$R^1$ and $R^2$ together with the nitrogen to which they are linked may form a 5- to 8- membered ring;
$R^3$ is lower alkyl, aryl or aralkyl; and
$R^{3a}$ is hydrogen, lower alkyl, aryl or aralkyl.

Thus, the compounds of the invention encompass the following types of compounds:

IA

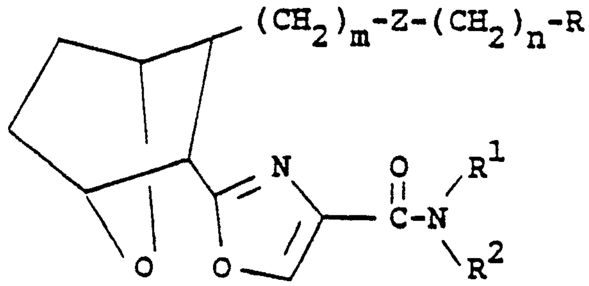

,

IB

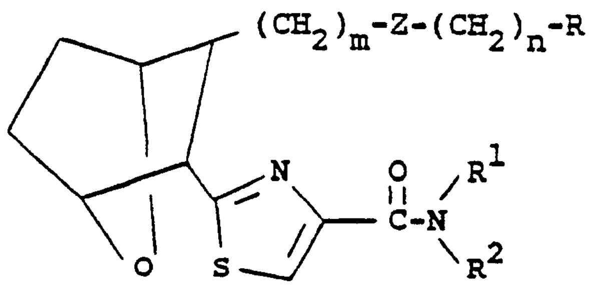

and

IC

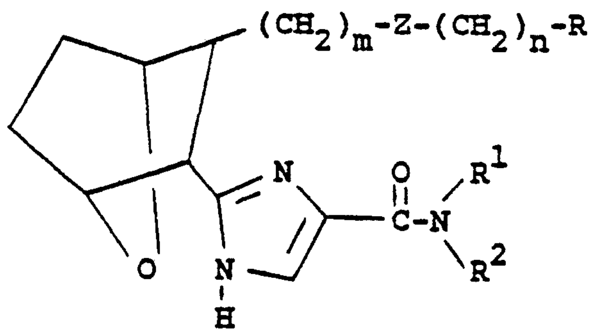

The term "lower alkyl" or "alkyl" as employed herein includes both straight and branched chain radicals of up to 16 carbons, preferably 1 to 8 carbons, such as methyl, ethyl, propyl, isopropyl, butyl, t-butyl, isobutyl, pentyl, hexyl, isohexyl, heptyl, 4, 4-dimethylpentyl, octyl, 2,2,4-trimethylpentyl, nonyl, decyl, undecyl, dodecyl, the various branched chain isomers thereof, and the like as well as such groups including 1, 2 or 3 halo substituents, an aryl substituent, an alkyl-aryl substituent, a haloaryl substituent, a cycloalkyl substituent or an alkylcycloalkyl substituent.

The term "cycloalkyl" includes saturated cyclic hydrocarbon groups containing 3 to 12 carbons, preferably 3 to 8 carbons, which include cyclopropyl, cyclobutyl, cyclopentyl, cyclohexyl, cycloheptyl, cyclooctyl, cyclodecyl and cyclododecyl, any of which groups may be substituted with substituents such as halogen, lower alkyl, lower alkoxy and/or a hydroxy group.

The term "aryl" or "Ar" as employed herein refers to monocyclic or bicyclic aromatic groups containing from 6 to 10 carbons in the ring portion, such as phenyl, naphthyl. Aryl (or Ar), phenyl or naphthyl may include substituted aryl, substituted phenyl or substituted naphthyl, which may include 1 or 2 substituents on either the phenyl or naphthyl such as lower alkyl, trifluoromethyl, halogen (Cl, Br, I or F), lower alkoxy, aralkoxy, hydroxy, alkylthio, alkylsulfinyl, alkylsulfonyl, arylthio, arylsulfinyl, and/or arylsulfonyl.

The term "aralkyl", "aryl-alkyl" or "aryl-lower alkyl" as used herein refers to lower alkyl groups as discussed above having an aryl substituent, such as benzyl.

The term "lower alkoxy", "alkoxy" or "aralkoxy" includes any of the above lower alkyl, alkyl or aralkyl groups linked to an oxygen atom.

The term "halogen" or "halo" as used herein refers to chlorine, bromine, fluorine or iodine with chlorine being preferred.

The term "lower alkenyl" or "alkenyl" as employed herein with respect to the $R^1$ substituent includes a carbon chain of up to 16 carbons, preferably 3 to 10 carbons, containing one double bond which will be separated from "N" by at least one saturated carbon moiety such as $-(CH_2)_q$where q can be 1 to 14, such as 2-propenyl, 2-butenyl, 3-butenyl, 2-pentenyl, 4-pentenyl and the like, and may include a halogen substituent such as I, Cl, or F.

The term "lower alkynyl" or "alkynyl" as employed herein with respect to the $R^1$ substituent includes a carbon chain of up to 16 carbons, preferably 3 to 10 carbons, containing one triple bond which will be separated from "N" by at least one saturated carbon moiety such as $-(CH_2)_{q'}$-where $q'$ can be 1 to 14, such as 2-propynyl, 2-butynyl, 3-butynyl and the like.

Preferred are compounds of formula I wherein Z is -CH=CH- in the cis configuration, m is 1, n is 2 or 3, R is $CO_2H$, $R^1$ is substituted phenylalkyl or cyclohexylalkyl and $R^2$ is H or methyl.

The compounds of formula I of the invention wherein Z is -CH=CH or $-(CH_2)_2-$ may be prepared as follows.

Compounds of the invention where Z is -CH=CH- and preferably in the cis form, and X is O are prepared starting with the hydroxymethyl compound II

II

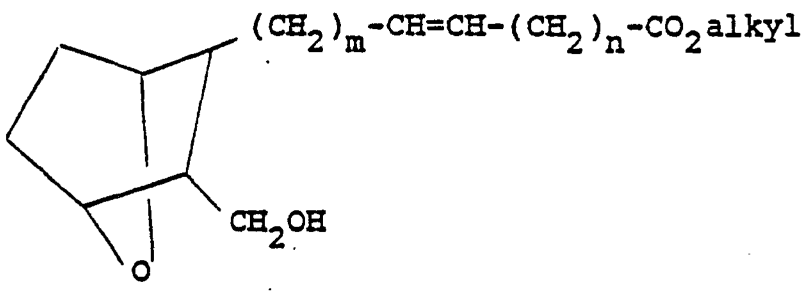

(which is prepared as described in U.S. Patent No. 4,143,054) which is subjected to a Jones oxidation wherein II is reacted with Jones' Reagent ($CrO_3$ dissolved or suspended in aqueous sulfuric acid, prepared as described in Fieser & Fieser, "Reagents for Organic Synthesis", Vol I, p. 142 (1967)), in the presence of acetone, under an inert atmosphere such as argon at a temperature within the range of from about -10 to about 20 °C, to form the corresponding carboxylic acid III

III

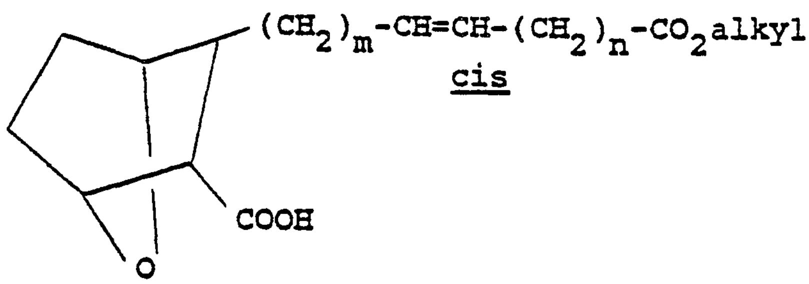

Acid III, in an inert organic solvent, such as tetrahydrofuran, is then made to undergo a carbodiimide coupling reaction with amide A

A

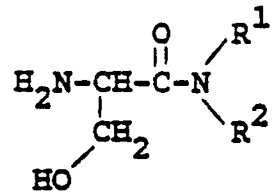

in the presence of dicyclohexylcarbodiimide (DCC) or 1-(3-dimethylaminopropyl)-3-ethylcarbodiimide hydrochloride (WSC) and 1-hydroxybenzotriazole under an inert atmosphere such as argon employing a molar ratio of A:III of within the range of from about 1.2:1 to about 1:1, to form hydroxybisamide IV

IV

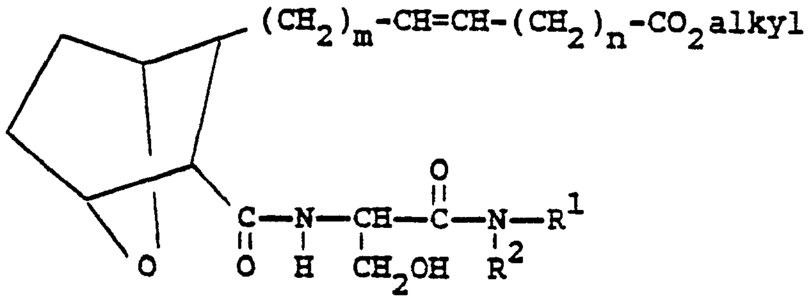

Hydroxybisamide IV is then subjected to cyclodehydration wherein a solution of IV in an inert organic solvent such as tetrahydrofuran, acetonitrile or chloroform , under an inert atmosphere such as argon, is treated with triphenylphosphine and carbon tetrachloride in the presence of an amine base such as triethylamine, to form oxazoline V.

V

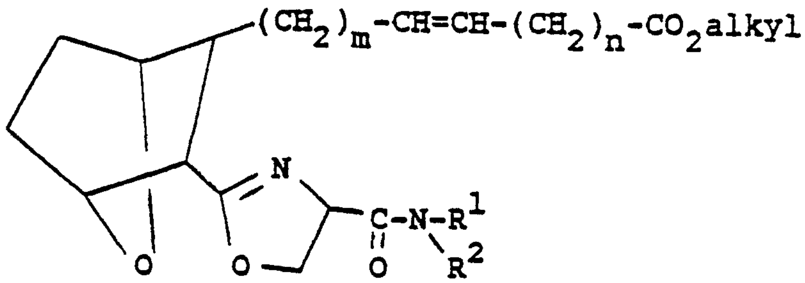

Alternatively, hydroxybisamide IV is treated with a sulfonyl chloride, such as methane sulfonyl chloride, and an amine base such as triethylamine followed by treatment with potassium carbonate in acetone to form oxazoline V.

Oxazoline V is oxidized by treatment with manganese dioxide or nickel peroxide, preferably nickel peroxide, to form the oxazole IA′

IA′

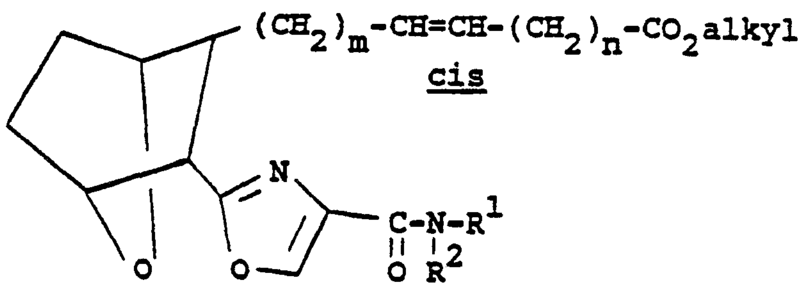

Alternatively, oxazole IA′ can be prepared from acid III

III

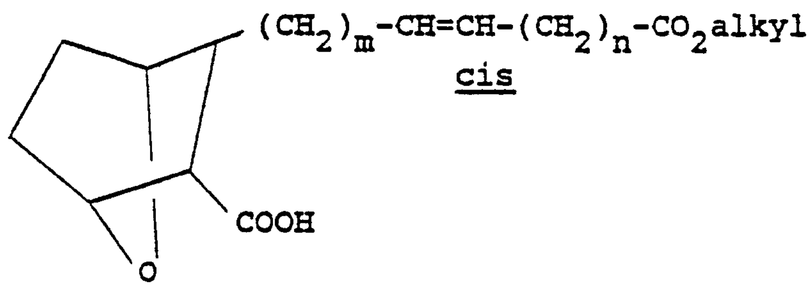

by a carbodiimide coupling as described previously except substituting <u>A</u>′ for <u>A</u> to obtain VI.

<u>A</u>'

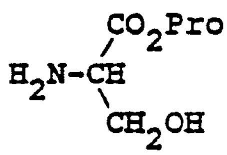

VI

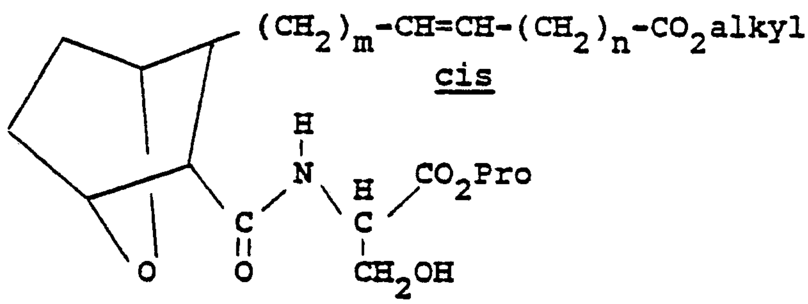

where Pro is a conventional protecting group. Hydroxyamide VI is then subjected to a cyclodehydration and oxidation as described for IV and V to form VII

VII

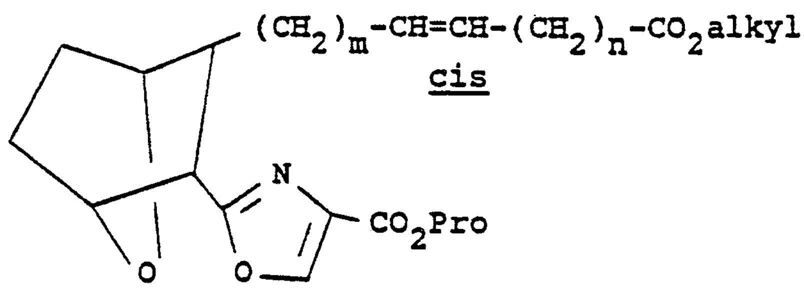

The protecting group of VII can be removed to form the corresponding acid VIII which is treated with excess

VIII

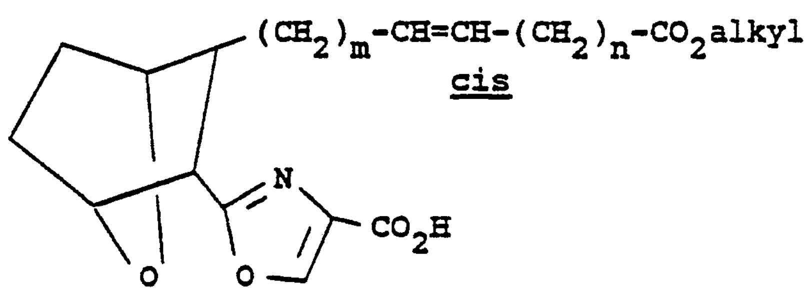

oxalyl chloride in the presence of an inert organic solvent such as toluene, methylene chloride or chloroform, and optionally a catalytic amount of dimethylformamide, while stirring under an inert atmosphere such as argon, to form the crude acid chloride IX

IX 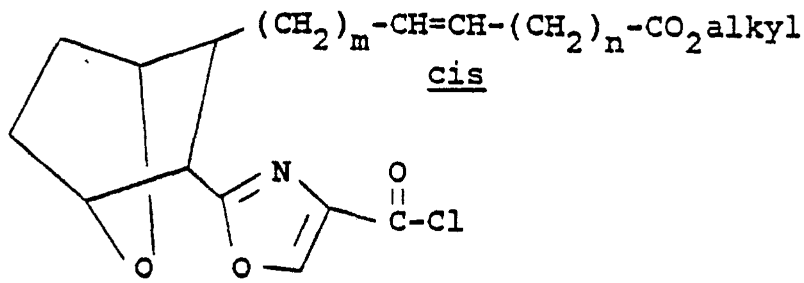

which is treated with amine hydrochloride A''

A'' 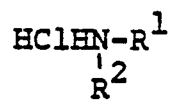

in the presence of an organic base such as triethylamine under an inert atmosphere such as argon, employing a molar ratio of IX:A'' of within the range of from about 0.5:1 to about 1:1 and preferably from about 0.8:1 to about 1:1, to form IA'.

IA' 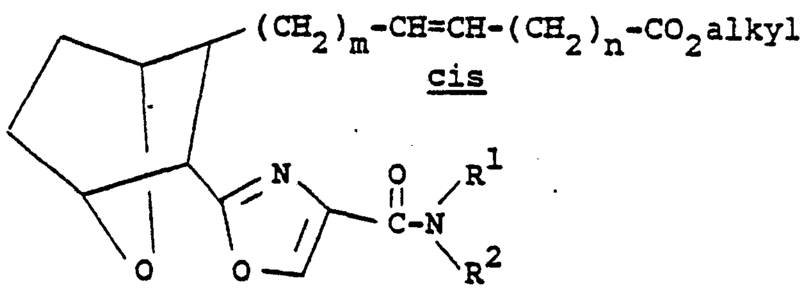

Compounds of formula I wherein Z represents -CH = CH- which is the trans double bond isomer may be prepared starting with hydroxymethyl compound II which includes a cis double bond. Compound II is treated with a protecting compound such as t-butyldimethylsilyl chloride or other silyl protecting group as described hereinbefore in the presence of imidazole or triethylamine and an inert organic solvent such as methylene chloride or tetrahydrofuran, to form the protected compound X

X 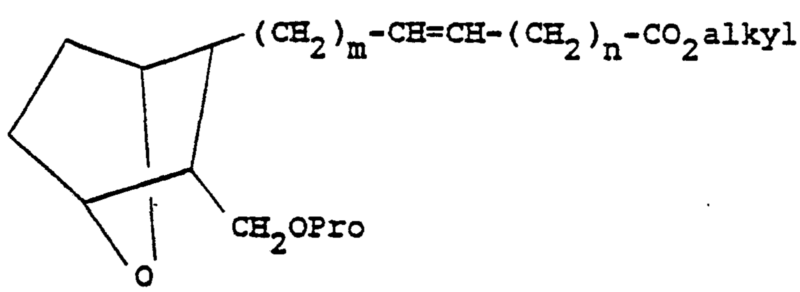

A solution of the protected alcohol X in an inert organic solvent such as methylene chloride or acetone is treated with excess ozone at reduced temperature of from about -78 °C to about -60 °C followed by treatment with excess dimethyl sulfide (molar ratio of X:$(CH_3)_2S$ of within the range of from about 1:100 to about 1:5), or triphenylphosphine, to form the aldehyde XI

XI

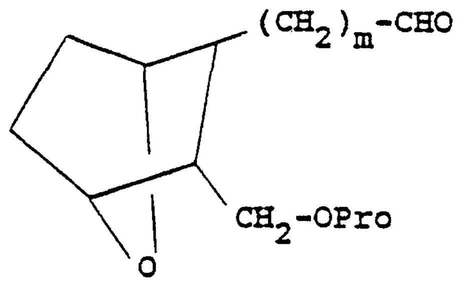

For compounds of the invention where Z = -CH = CH-in the trans form and n = 2, aldehyde XI is then treated with a mixture of lithium bromide or lithium chloride and trimethylphosphonoacetate and triethylamine in an inert organic solvent such as methylene chloride or chloroform to form the ester XII

XII

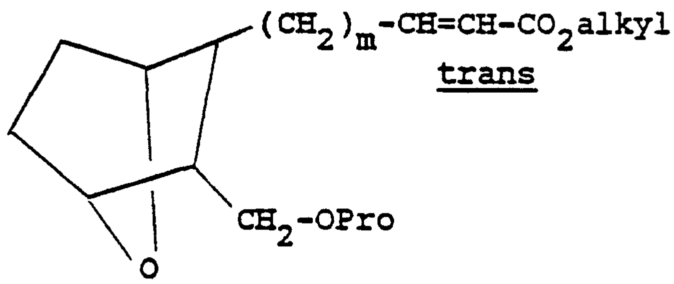

A solution of ester XII in an inert organic solvent such as tetrahydrofuran, ether or dimethoxyethane is cooled to a temperature of from about $-78^\circ$ C to $0^\circ$ C and reacted with diisobutylaluminum hydride in an aromatic solvent such as toluene for a period of from about 0.5 to about 4 hours to form alcohol XIII

XIII

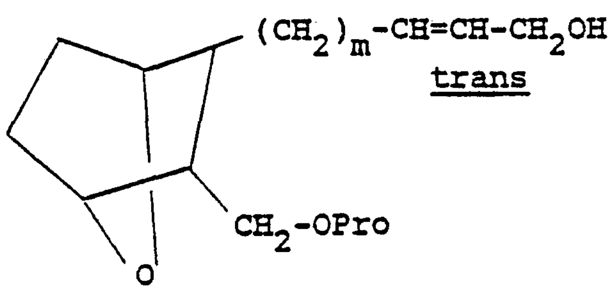

Alcohol XIII is treated with bromotriphenylphosphonium bromide (formed by adding bromine to triphenylphosphine in toluene or other aromatic solvent under an inert atmosphere such as argon, at a reduced temperature of from about -10 to about $10^\circ$ C) in the presence of pyridine and toluene, at a reduced temperature of from about -10 to about $10^\circ$ C, to form bromide XIV

XIV

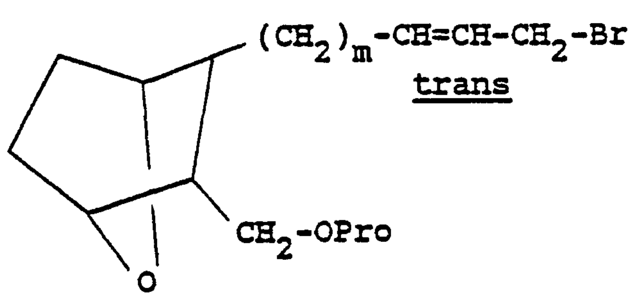

An acetic acid alkyl ester such as t-butyl acetate or ethyl acetate is treated with a solution of LDA (lithium diisopropylamide) in an inert organic solvent such as tetrahydrofuran at a reduced temperature of from about -78 to about $-60^\circ$ C for a period of from about 0.5 to about 2 hours followed by addition of a

solution of bromide XIV in an inert organic solvent such as tetrahydrofuran to form ester XV (where n = 2)

XV 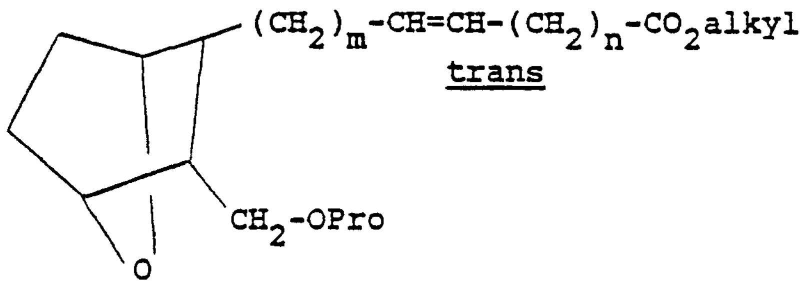

For compounds of the invention where Z = -CH = CH- in the trans form and n is 1, 3, or 4, aldehyde XI is allowed to react with a phosphonium salt of formula P

$$\underline{P} \quad (C_6H_5)_3\overset{\oplus}{P}-(CH_2)_{n+1}-CH_2OH \quad Br^{\ominus}$$

in the presence of a strong base such as potassium t-amylate in toluene or NaH/dimethylsulfoxide to give XIII′

XIII' 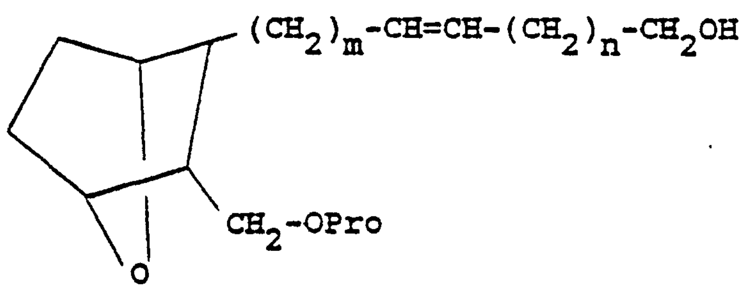

which is oxidized and esterified using procedures known to those skilled in the art to form ester XV (where n = 1, 3, or 4).

Ester XV is then deprotected by treating XV in methanol under an inert atmosphere such as argon with hydrochloric acid in methanol (prepared by adding acetyl chloride to methanol) to form alcohol XVI

XVI 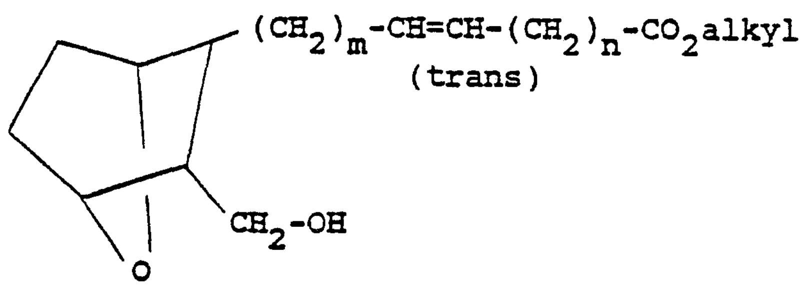

XVI may then be used in place of II as a starting material following the procedure hereinbefore described to form acid IIIA

IIIA

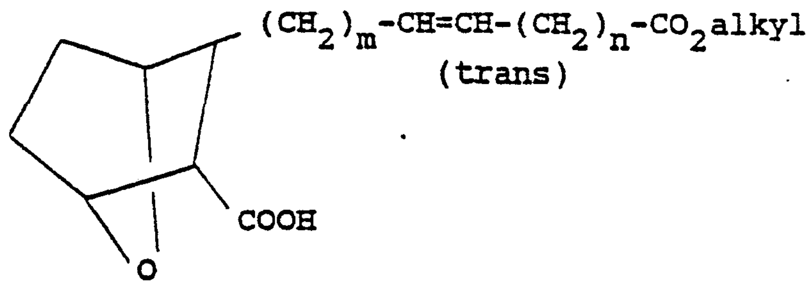

and subsequently to form the trans compound of the invention IA"

IA"

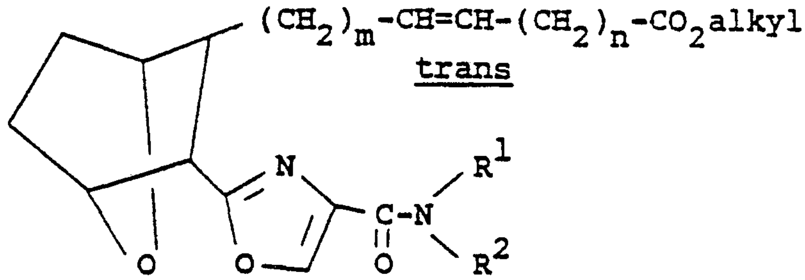

Compounds of the invention IB wherein Z is -CH=CH- and X is S may be prepared starting with acid III or IIIA as follows:

Acid III or IIIA is reacted with oxalyl chloride, optionally in the presence of catalytic amounts of dimethylformamide, in methylene chloride, to form the corresponding acid chloride which is amidated by reacting with ammonia to form the amide XVII

XVII

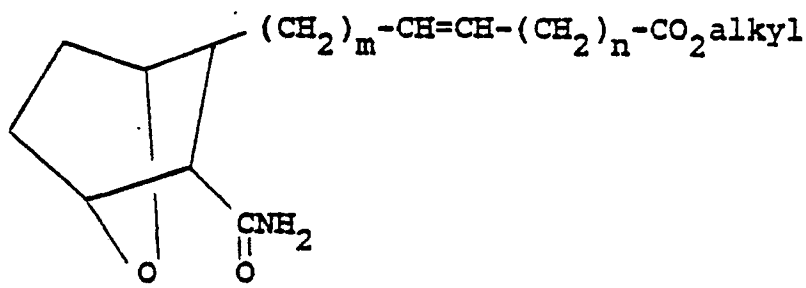

Alternatively, acid III or IIIA is reacted with an alkylchloroformate in the presence of an amine such as triethylamine to form the mixed anhydride which is amidated by reacting with methanol-ammonia solution or concentrated aqueous ammonia solution to form amide XVII.

Amide XVII is then treated with phosphorus pentasulfide ($P_2S_5$) or Lawesson's Reagent (2,4-bis(4-methoxyphenyl)-1,3-dithia-2,4-diphosphetane-2,4-disulfide) to form the corresponding thioamide XVIII

XVIII

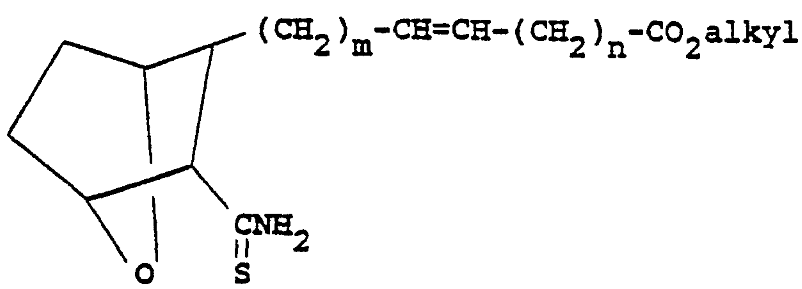

which is treated with bromopyruvic acid

$(Br-CH_2-\overset{O}{\overset{\|}{C}}-CO_2H)$

in a polar solvent such as dimethylformamide in the presence of a weak base such as $K_2CO_3$ employing a molar ratio of XVIII: bromopyruvic acid of within the range of from about 1:1 to about 1:1.5 to form thiazoline XIX

XIX 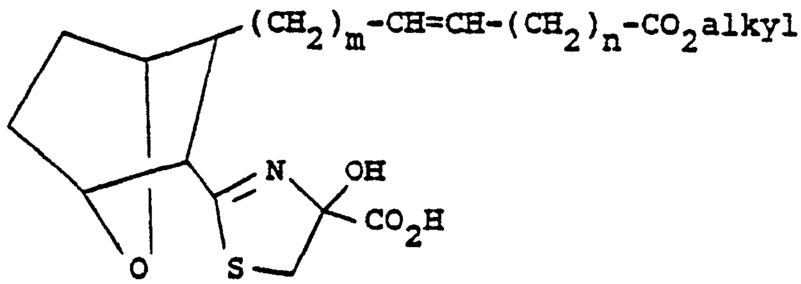

Thiazoline XIX is then dehydrated by treatment with a sulfonyl chloride such as methanesulfonyl chloride in the presence of a base such as triethylamine to form thiazole acid XX

XX 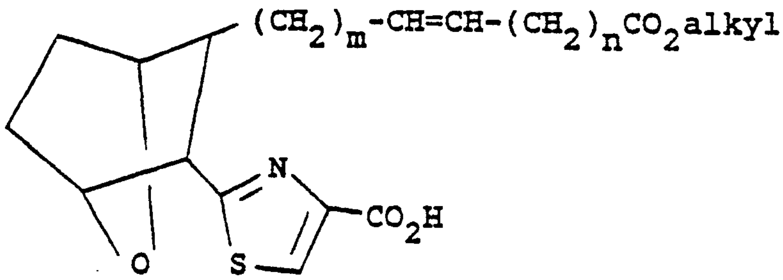

which is then made to undergo a carbodiimide coupling reaction with amine

A''' 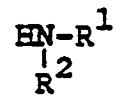

in the presence of DCC or WSC under an inert atmosphere such as argon employing a molar ratio of A''':XX of within the range of from about 1:1 to about 2:1, to form amide $IB_a$

$IB_a$ 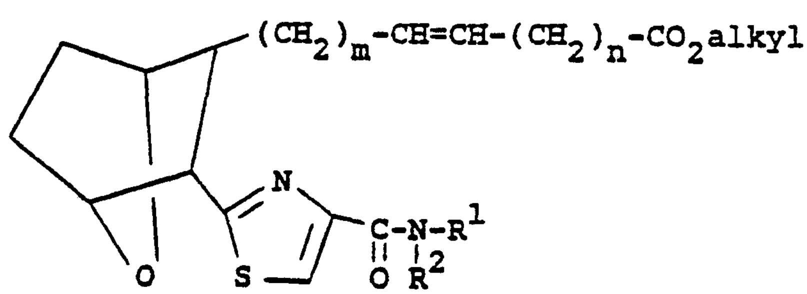

Compounds of the invention IC where X is NH are prepared starting with acid III or IIIA which is made to undergo a coupling reaction with amine B

B 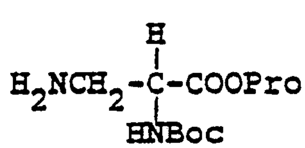

where Boc is t-butyloxycarbonyl and Pro is a protecting group such as preferably $-CH_2CH_2Si(CH_3)_3$, in the presence of a coupling agent such as 1-(3-dimethylaminopropyl)-3-ethylcarbodiimide hydrochloride (WSC) and 1-hydroxybenzotriazole (HOBT) and methylene chloride employing a molar ratio of III or IIIA:B of within the range of from about 1.2:1 to about 1:1, for a period of from about 12 to about 90 hours. The resulting amide is made to undergo a thionation reaction by treating the amide with Lawesson's reagent in the presence of benzene at a temperature of from about 50 to about 75 °C for a period of from about 1 to about 4 hours, to form the ester XXI

XXI 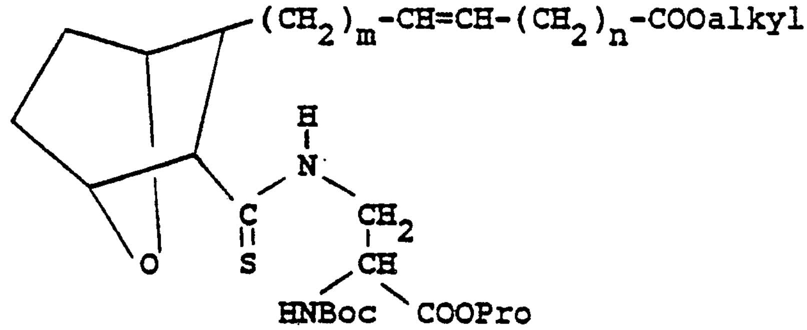

The ester XXI is cyclized by treating a solution of XXI in an inert solvent such as acetonitrile, chloroform or tetrahydrofuran, with triphenylphosphine (employing a molar ratio of XXI:triphenylphosphine of from about 0.8:1 to about 1:1) and carbon tetrachloride in the presence of an amine base such as triethylamine or diisopropylethylamine, to form imidazoline XXII

XXII 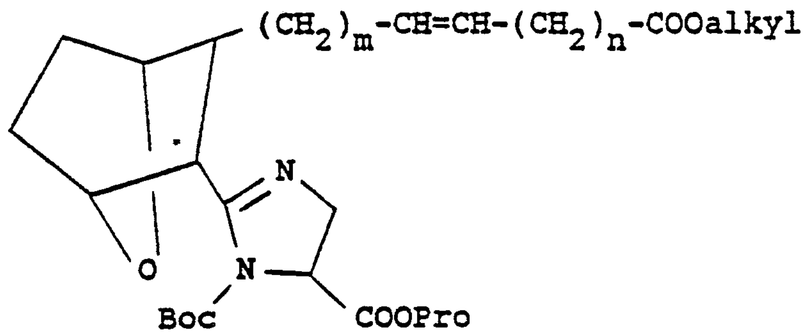

Imidazoline XXII is then deprotected to remove the Pro protecting group, using conventional procedures, for example, by treatment with trifluoroacetic acid in the presence of methylene chloride, to form the acid XXIII

XXIII 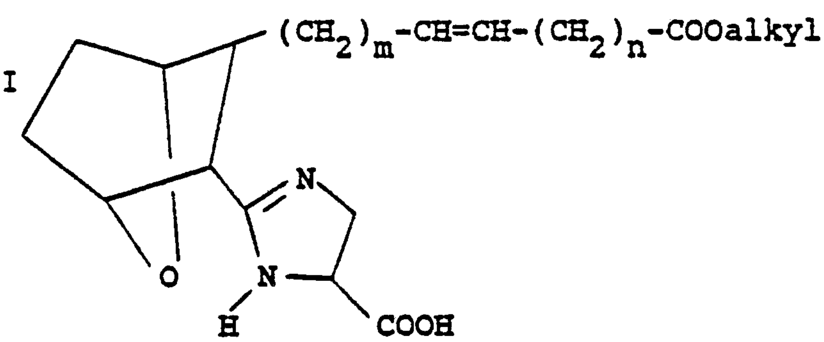

Next, the acid XXIII is made to undergo a coupling reaction with amine A‴ in the presence of an amine base such as pyridine or triethylamine under an inert atmosphere such as argon in the presence of a coupling agent such as WSC and HOBT and chloroform, employing a molar ratio of A‴:XXIII of within the range of from about 0.8:1 to about 1.2:1 to form amide XXIV

XXIV

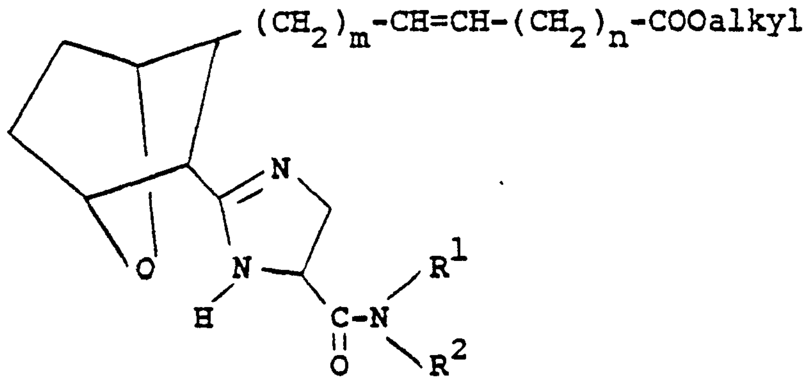

Amide XXIV is oxidized by treatment with an oxidizing agent such as manganese dioxide in the presence of an inert solvent such as chloroform to form ester ICa

ICa

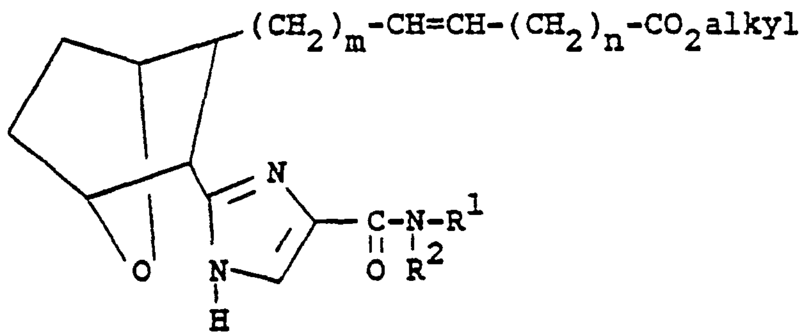

The esters IA′, IA″, IBa, and ICa may be converted to the corresponding acids, that is $I^1$

$I^1$

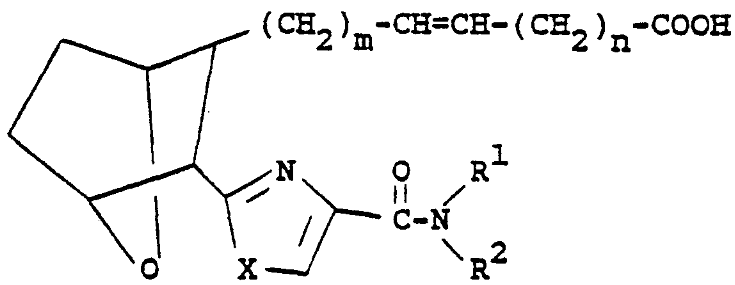

by treating the esters with a base, such as lithium hydroxide, sodium hydroxide or potassium hydroxide to form the corresponding alkali metal salt, followed by neutralization with an acid, such as dilute hydrochloric acid or oxalic acid to form the acid compounds of the invention.

Compounds of formula I wherein Z is $-(CH_2)_2-$may be prepared from acid $I^1$ by subjecting $I^1$ to hydrogenation using, for example, a hydrogenation catalyst, such as palladium on charcoal, in the presence of an inert organic solvent such as ethyl acetate (EtOAc) or acetic acid (AcOH) to form acid of the invention $I^2$

$I^2$

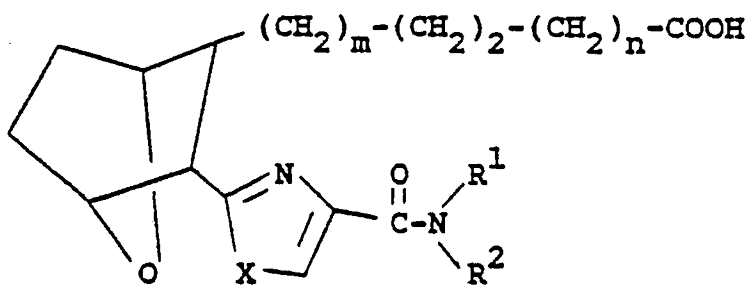

Compounds of the invention wherein R is $CONHSO_2R^3$, that is $I^3$

$I^3$

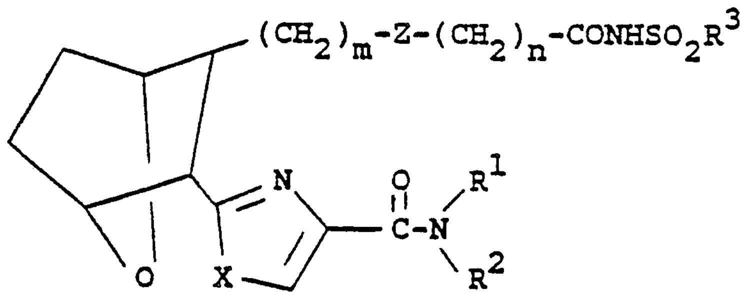

are prepared by treating acid $I^1$ or $I^2$ with a sulfonamide of the structure C

C

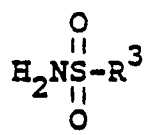

in the presence of a coupling agent such as carbonyldiimidazole or WSC in the presence of an amine such as 4-dimethyl aminopyridine under an inert atmosphere such as argon employing a molar ratio of C:$I^1$ or $I^2$ of within the range of from about 0.8:1 to about 1.2:1, to form sulfonamide $I^3$.

Acids $I^1$ or $I^2$ may be converted to the corresponding alkyl esters by treating the acids $I^1$ and $I^2$ with the appropriate alcohol under acid catalysis to form the esters.

Compounds of the invention wherein R is $-CH_2$-5-tetrazolyl, and Z is $-(CH_2)_2-$ that is $I^4$

$I^4$

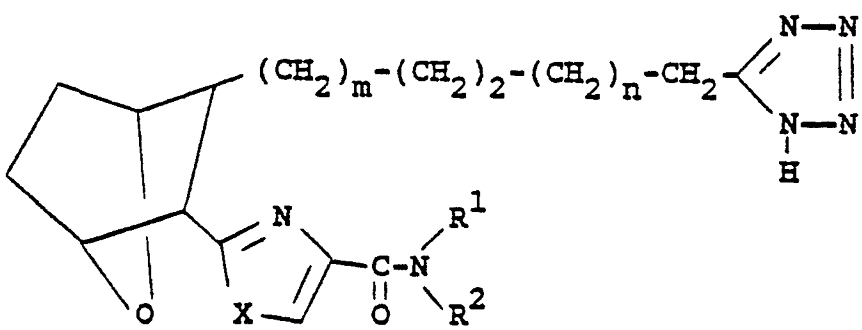

are prepared by subjecting esters IA′, IA″, IBa and ICa or the esters of $I^1$ to reduction with a hydride reagent such as lithium borohydride or sodium borohydride to afford alcohol XXV

XXV

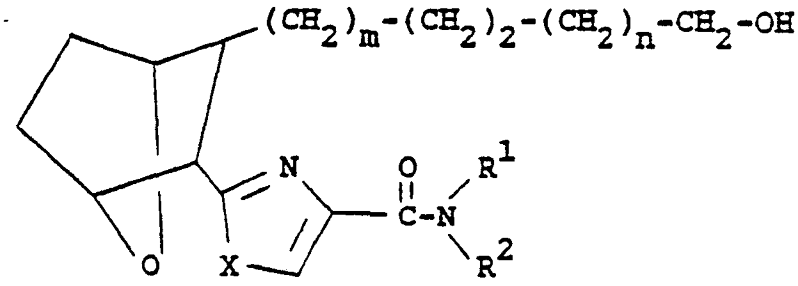

which is converted to the bromide on treatment with triphenylphosphonium dibromide in an inert solvent such as toluene. The bromide is then converted to nitrile XXVI on treatment with an alkali metal cyanide in a polar solvent such as methanol/water.

XXVI

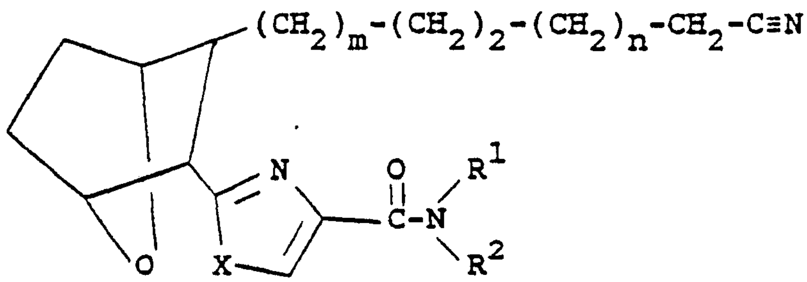

The nitrile XXVI is then subjected to a cycloaddition reaction by treating XXVI with sodium azide in the presence of ammonium chloride, dimethylformamide and lithium chloride at a temperature of from about 100 °C to about 130 °C to form $I^4$.

Compounds of the invention wherein R is $-CH_2$-5-tetrazolyl, and Z is -CH = CH- that is $I^5$

$I^5$

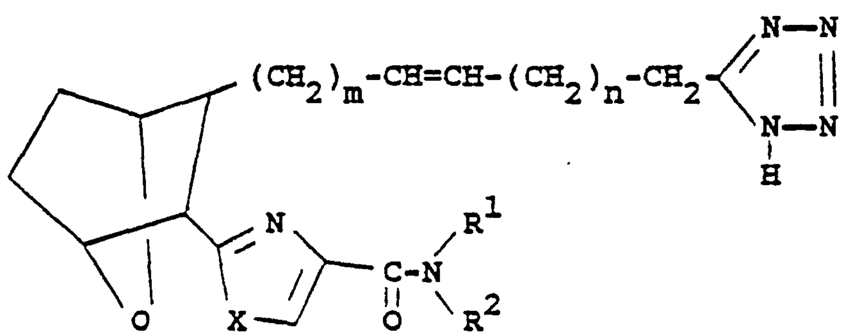

are prepared by treating hemiacetal D (prepared as described in U.S. Patent No. 4,654,356)

D

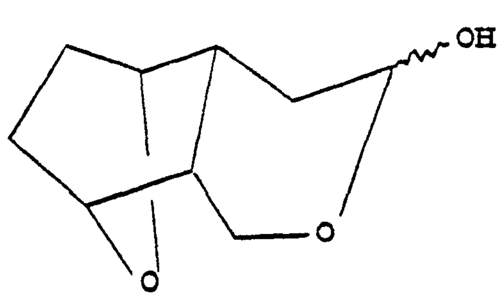

with a Wittig reagent of the structure E

E .

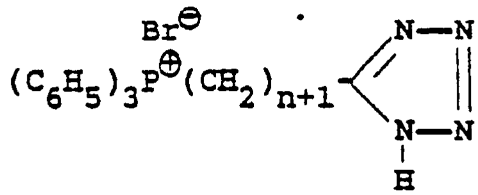

in the presence of a base, such as potassium t-butoxide or sodium hydride-dimethyl sulfoxide employing a molar ratio of D:E of within the range of from about 1:1 to about 0.2:1 to form the hydroxymethyl compound XXVII

XXVII

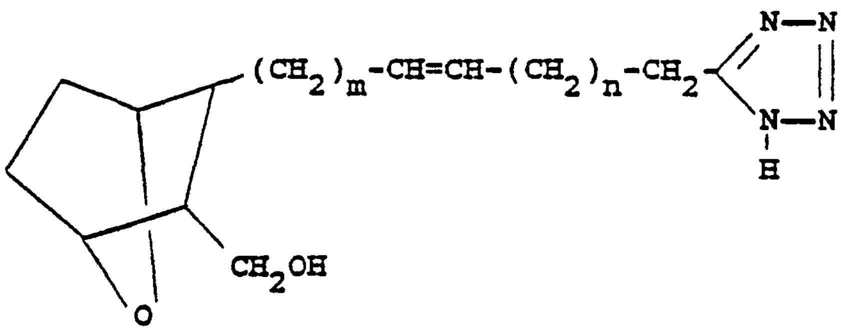

which is treated with protecting compound F

F Pro-Halide

for example, bromomethyl methyl ether in the presence of base to form the protected tetrazole XXVIII

XXVIII

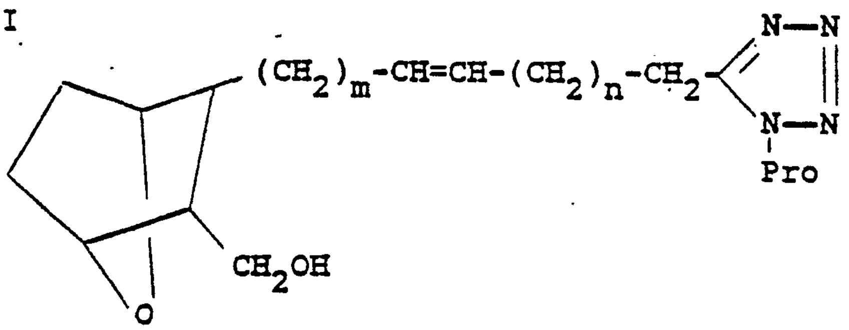

The protected tetrazole XXVIII may then be used in place of hydroxymethyl compound II to form the various compounds of the formula XXIX wherein X is O, S or NH

XXIX

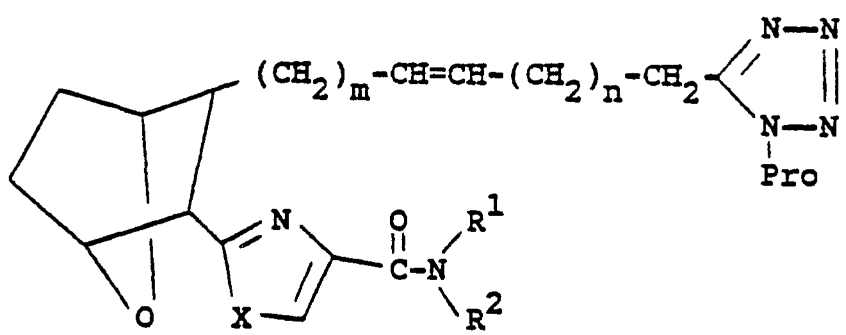

which is deprotected by treatment with aqueous acid such as aqueous hydrochloric acid to form compounds of the invention $I^5$.

Compounds of formula I wherein R is $CONHR^{3a}$ wherein $R^{3a}$ is other than H may be prepared from the corresponding acid $I^6$

$I^6$

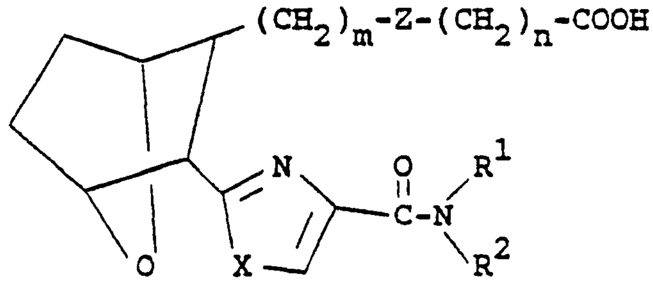

by treating acid $I^6$ with 1(3-dimethylaminopropyl)-3-ethylcarbodiimide hydrochloride in the presence of dimethylformamide, 1-hydroxybenzotriazole and an organic base such as triethylamine and amine G

G HNHR$^{3a}$

to form the amide of the invention $I^7$

$I^7$

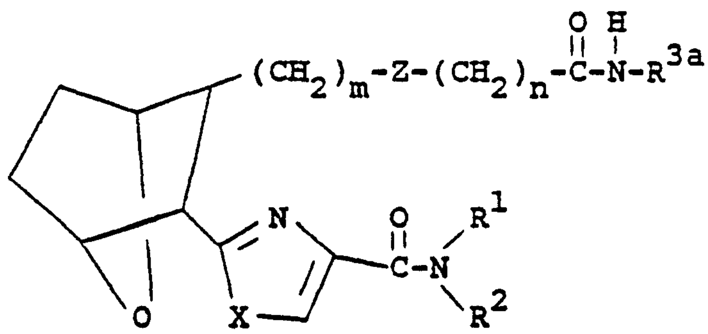

where $R^{3a}$ is lower alkyl, aryl or aralkyl.

Compounds of formula I wherein R is $CONH_2$ may be prepared from the corresponding acid $I^6$, employing the procedure as described above for making amide $I^7$ except that ammonium chloride is employed in place of amine G to form the amide of the invention $I^8$

$I^8$

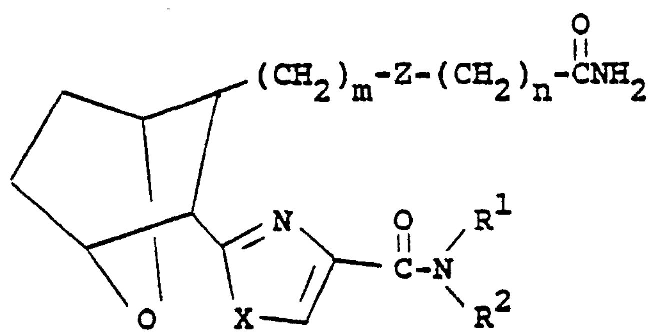

Compounds of formula I wherein R is $CH_2OH$ may be prepared from the corresponding ester $I^9$

$I^9$

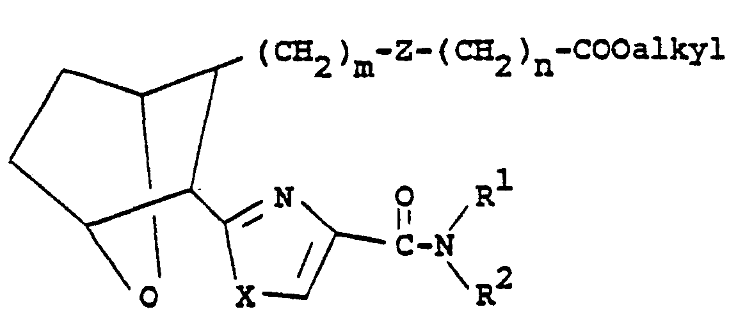

which is treated with a reducing agent such as lithium borohydride ($LiBH_4$) in an inert solvent such as diethyl ether or tetrahydrofuran to form the alcohol $I^{10}$

$I^{10}$

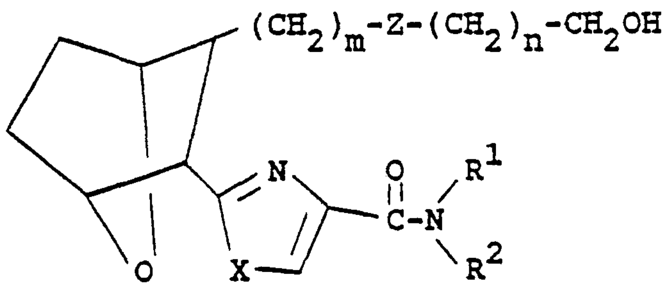

The compounds of this invention have four centers of asymmetry as indicated by the asterisks in formula I. However, it will be apparent that each of the formulae set out above which do not include asterisks still represent all of the possible stereoisomers thereof. All of the various stereoisomeric forms are within the scope of the invention.

The various stereoisomeric forms of the compounds of the invention, namely, cis-exo, cis-endo and all trans forms and stereoisomeric pairs may be prepared by employing starting materials and following the procedures as outlined in U.S. Patent No. 4,143,054. Examples of such stereoisomers are set out below.

Ia

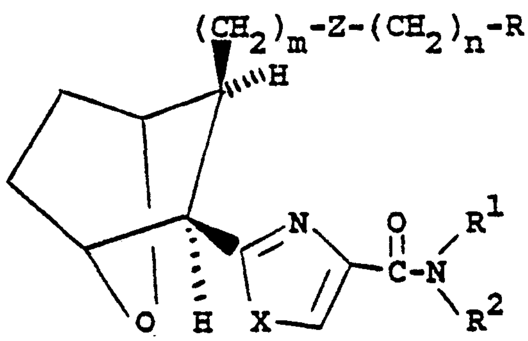

(cis-endo)

Ib

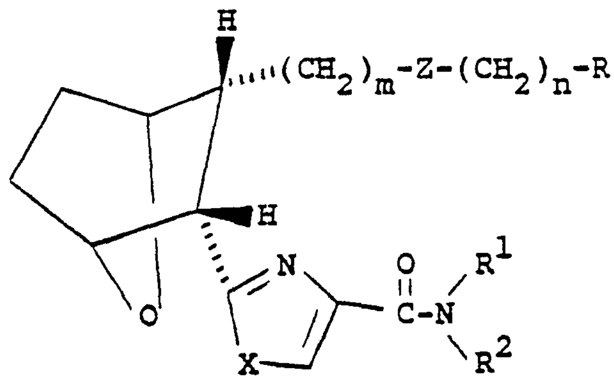

(cis-exo)

Ic

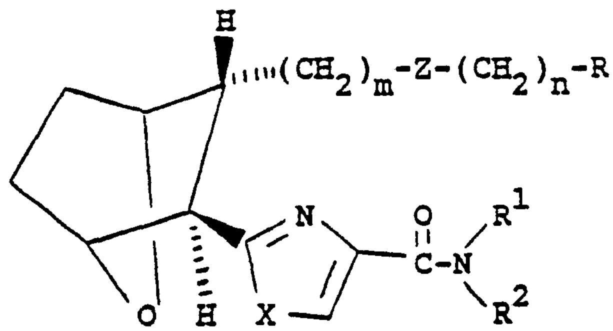

(trans)

Id

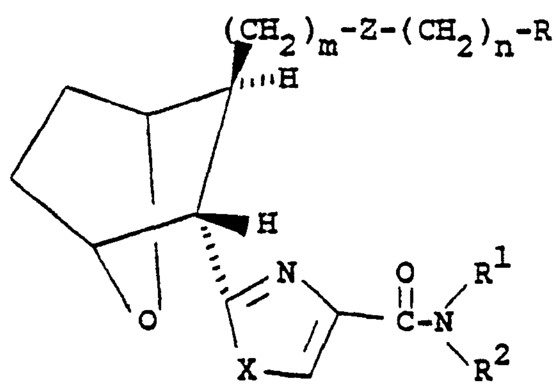

(trans)

The nucleus in each of the compounds of the invention is depicted as

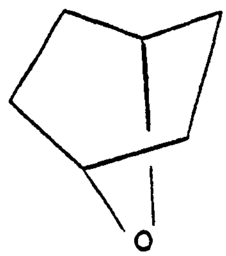

for matter of convenience; it will also be appreciated that the nucleus in the compounds of the invention may be depicted as

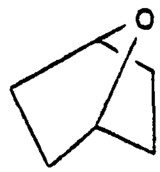

The compounds of this invention are thromboxane receptor antagonists and as such are useful as inhibitors of thromboxane receptor mediated actions. The term "thromboxane receptor antagonist" includes compounds which are so-called thromboxane $A_2$ receptor antagonists, thromboxane $A_2$ antagonists, thromboxane $A_2$/prostaglandin endoperoxide antagonists, TP-receptor antagonists, or thromboxane antagonists.

The compounds of the invention are also thromboxane synthetase inhibitors and thus are useful as inhibitors of thromboxane production.

The compounds of this invention are useful as inhibitors of platelet function, i.e., for the prevention and treatment of thrombotic vascular occlusive disorders, whether complete or partial, for example, arterial thrombosis, including that of the coronary, cerebral, ophthalmic, hepatic, mesenteric, renal, peripheral arteries or vascular or organ grafts, unstable angina, transient ischemic attacks, or intermittent claudication. They may be useful to prevent thrombosis following vascular injury produced in the course of diagnostic or therapeutic procedures such as endarterectomy or angiography. The compounds may be useful in the treatment or prevention of disorders characterized by platelet consumption and/or activation, including, platelet activation, dysfunction, and/or loss during extracorporeal circulation, the use of radiographic contrast agents, thrombotic thrombocytopenia purpura, disseminated intravascular coagulation, purpura fulminans, hemolytic transfusion reaction, or hemolytic uremic syndrome, systemic lupus, cyclosporine-induced renal toxicity, pulmonary hypertension, side effects from dialysis, or abdominal aortic aneurism repair. The compounds may be used in the treatment of venous thrombosis or embolism, including pulmonary embolism, deep venous thrombosis, hepatic vein thrombosis, and renal vein thrombosis.

The compounds of this invention are useful as inhibitors of arterial or venous vasoconstriction. Accordingly, they may be useful to prevent vasoconstriction associated with unstable angina, chronic stable angina, and variant, or Prinzmetal's angina, Raynaud's syndrome, migraine headache, vasospasm of the coronary, cerebral, ophthalmic, hepatic, mesenteric, renal, peripheral arteries or vascular grafts, vascular injury such as that associated with surgery or trauma. Hypertension of pregnancy, the hepato-renal syndrome, and pulmonary hypertension are additional examples of vasoconstrictive disorders treatable by the compounds of this invention.

The compounds of this invention are useful as inhibitors of bronchoconstriction, i. e., airway hyperresponsiveness, allergic bronchospasm, asthma, and bronchoconstrictive responses to environmental, infectious, noxious or mechanical stimuli.

The compounds of this invention are useful as inhibitors of ischemic and reperfusion injury to various tissues, including, myocardium, skin, brain, bowel, or kidney, alone or in combination with other agents intended to restore blood flow. For example, these compounds may be useful for improving postischemic myocardial function and decreasing myocardial infarct size. Ischemia caused by reduced blood flow during diagnostic or therapeutic procedures may benefit by treatment with these compounds, for example, they reduce the myocardial stunning observed after bypass surgery. In addition, they may be useful for reducing the tissue injury caused by a stroke.

The compounds of this invention may be useful in the prevention or treatment of other conditions including burns, diabetic retinopathy, tumor metastases and tardive dyskinesia. The compounds may be

useful in potentiating diuretic-induced diuresis.

In addition, the thromboxane receptor antagonists of the invention may be used with a thrombolytic agent such as t-PA, streptokinase, urokinase, prourokinase or anisoyiated plasminogenstreptokinase activator complex (APSAC) within 6 hours of a myocardial infarction. In such case, the thrombolytic agent may be used in amounts conventionally employed, for example, as disclosed in the Physicians' Desk Reference for reducing post-ischemic myocardial injury.

The compounds of the invention can be administered orally or parenterally to various mammalian species known to be subject to such maladies, e.g., humans, cats, dogs and the like in an effective amount within the dosage range of about 0.1 to about 100 mg/kg, preferably about 0.2 to about 50 mg/kg and more preferably about 0.5 to about 25 mg/kg (or from about 1 to about 2500 mg, preferably from about 5 to about 2000 mg) on a regimen in single or 2 to 4 divided daily doses.

The oxazole derivatives of the invention, that is compounds of formula I where X is O have particularly long duration of action and these may, if desired, be administered in the above dosages once daily, once every other day, or if desired once daily two times a week.

The active substance can be utilized in a composition such as tablet, capsule, solution or suspension containing about 5 to about 500 mg per unit of dosage of a compound or mixture of compounds of formula I or in topical form for wound healing (0.01 to 5% by weight compound of formula I, 1 to 5 treatments per day). They may be compounded in conventional matter with a physiologically acceptable vehicle or carrier, excipient, binder, preservative, stabilizer, flavor, etc., or with a topical carrier such as Plastibase (mineral oil gelled with polyethylene) as called for by accepted pharmaceutical practice. Also as indicated in the discussion above, certain members additionally serve as intermediates for other members of the group.

The compounds of the invention may also be administered topically to treat peripheral vascular diseases and as such may be formulated as a cream or ointment.

The following Examples represent preferred embodiments of the present invention. Unless otherwise indicated, all temperatures are expressed in degrees Centigrade.

## Example 1

[1S-[1α,2α(Z),3α,4α]]-6-[3[4[[(4-Cyclohexylbutyl) amino]carbonyl]-2-oxazolyl]-7-oxabicyclo[2.2.1]hept-2-yl]-4-hexenoic acid

A. [(1,1-Dimethylethoxy)carbonyl]-N-(4-cyclohexylbutyl)-L-serinamide

To a solution of 575 mg of 4-cyclohexylbutyl amine hydrochloride (3.0 mmol), 615 mg t-butyloxy carbonyl-L-serine (3.0 mmol, 1.0 equiv), 405 mg 1-hydroxybenzotriazole hydrate (3.0 mmol, 1.0 equiv), and 387 mg diisopropylethylamine (3.0 mmol, 1.0 equiv) in 10 mL dry tetrahydrofuran (TXF) stirring under argon at 0°, was added 618 mg 1,3-dicyclohexylcarbodiimide (3.0 mmol, 1.0 equiv) in a single portion. A precipitate slowly formed. After 1 hour the mixture was warmed to room temperature and stirred for 4 hours. After dilution with ethyl acetate, the mixture was filtered, and the filtrate was washed with a pH 1 salt solution (formed by mixing water, brine, and 1 M aqueous HCl solution). Further washing (twice) with 1 M $NaHCO_3$ was followed by drying over $Na_2SO_4$ and evaporation to give 1.1 g of crude title amide.

| TLC (10% [10% conc. aqueous $NH_3$ in $CH_3OH$] in $CH_2Cl_2$ - anisaldehyde): | |
|---|---|
| cyclohexylbutylamine HCl | 0.27 |
| title amide | 0.47 |

B. N-(4-Cyclohexylbutyl)-L-serinamide

To a solution of 1.1 g crude Part A amide in 4 mL $CH_2Cl_2$ at room temperature was added 4 mL

trifluoroacetic acid. The mixture was stirred for 4 hours. After solvent evaporation, residual trifluoroacetic acid was azeotropically removed by rotoevaporation with $CHCl_3$. Flash chromatography (150 g silica, 10% [10% concentrated aqueous $NH_3$ in $CH_3OH$] in $CH_2Cl_2$) gave, after azeotroping with toluene and exposure to high vacuum, 495 mg of pure title amine as a white solid. The yield of title amine was 68% overall from 4-cyclohexylbutyl amine hydrochloride.

| TLC (10% [10% conc. aqueous $NH_3$ in $CH_3OH$] in $CH_2Cl_2$ - anisaldehyde): | |
|---|---|
| Part A amide | 0.47 |
| title amine | 0.17 |

$^{13}C$ NMR (67.8 MHz in $CDCl_3$):
173.4, 64.6, 56.3, 39.1, 37.3, 36.9, 33.1, 29.6, 26.5, 26.2, 24.0

C. [1S-[1α,2α(Z),3α,4α]]-6-[3-(Hydroxymethyl)-7-oxabicyclo[2.2.1]hept-2-yl]-4-hexenoic acid, methyl ester

To a partial solution of 36.27 g of [4aR(4aα,5β,8β,8aβ)]-octahydro-5,8-epoxy-1H-2-benzopyran-3-ol (prepared as described in U.S. Patent No. 4,143,054) (0.23 mol) and 3-carboxypropyltriphenylphosphonium bromide (127.34 g, 0.37 mol) in 600 mL of dry THF under argon at 3 °C was added dropwise over 1 hour a solution of 370.6 mL of potassium t-amylate (0.68 mol of a 1.8M toluene solution) with mechanical stirring. Initially the reaction temperature reached a maximum of 8 °C and subsequently leveled off to 4 °C for the remainder of the base addition. The reaction was then run at room temperature for 90 minutes. A 0 °C ice bath was introduced and the reaction was quenched by the addition of 152 mL of glacial acetic acid, over 30 minutes. Solvents were removed in vacuo (azeotroped with toluene). Water (640 mL) and 50 mL of concentrated HCl were added (pH 2.6). Dilution with 640 mL of ethyl acetate, the addition of 149 g of NaCl and a few seed crystals of 3-carboxypropyltriphenylphosphonium bromide was followed by vigorous stirring for 15 minutes. The precipitate was collected by filtration and washed with 2 portions each of 320 mL of ethyl acetate. The ethyl acetate layer was separated, the aqueous layer was extracted with ethyl acetate (2 x 200 mL each), the combined ethyl acetate layers were dried over $MgSO_4$ and concentrated. Aqueous 5% $K_2CO_3$ was added (507 mL) followed by vigorous stirring for 1 hour. No precipitation occurred. The reaction mixture was concentrated to a paste and suspended in 508 mL of water. Several hours of vigorous stirring produced no precipitate. The water was decanted off and the residue was suspended in 200 mL of aqueous 5% $K_2CO_3$ solution. After vigorous stirring, a light tan solid was collected by filtration and rinsed several times with water. The combined aqueous layers were extracted 5 X with 1:1 toluene/ether (230mL each). After cooling the combined aqueous layers with a 0 °C ice bath, concentrated HCl was added to pH 2.5, followed by extraction 1 X with 460 mL then 2 X with 230 mL each of ethyl acetate. The combined ethyl acetate layers were dried over $MgSO_4$ and evaporated in vacuo to yield 49.74g of an oil. Trituration from 330 mL of ether (room temperature, overnight) oiled out phosphorous by-products. The ether solution was decanted away from the dark red oil into a separatory funnel, and the oil which was carried over by the decantation was drained off (1.56 g). Evaporation of the ether solution in vacuo yielded 43.08 g of [1S-[1α,2α(Z),3α,4α]]-6-[3-hydroxymethyl)-7-oxabicyclo[2.2.1]hept-2-yl]-4-hexenoic acid in the form of a viscous yellow oil.
$^1$HNMR indicated a product: triphenylphosphine oxide: ether molar ratio of 23:1:1.8 (mass % 93:4.7:2.2). Yield exclusive of triphenylphosphine oxide/ether, 40.06 g (72.5%).

Acetyl chloride (5.20 mL, 0.073 mol) was added dropwise to 80 mL of methanol at room temperature under argon. The acetyl chloride/methanol solution was then added to a solution of 42.98 g (0.18 mol) of the above acid in 700 mL of methanol in one portion. Stirring was continued for 3 hours. Triethylamine was added (0.09 mol, 12.21 mL), methanol was removed in vacuo, and the residue was partitioned between 300 mL of ethyl acetate and 150 mL of water. After separation of the layers, the aqueous layer was extracted with 150 mL of ethyl acetate, the combined ethyl acetate layers were washed with brine, dried over $Na_2SO_4$ and evaporated in vacuo to yield 43.06 g of a viscous tan oil. Flash chromatography on 1350 g of E. Merck Kieselgel 60 silica gel (240-400 mesh, 75/25 ether/hexanes, then ether after the desired product began eluting off the column) yielded 35.74 g title ester in the form of a viscous light yellow oil, free from triphenylphosphine oxide by NMR.
$^1$H NMR ($CDCl_3$, ref. TMS): δ 5.41-5.38, m (2H); 4.49, d, J=4.69Hz (1H); 4.22, d, J=4.69Hz (1H); 3.73-3.69,

m (1H); 3.67, s, (3H); 3.60, m (1H); 2.37, br s (4H); 2.12-1.99, m (3H); 1.97-1.85, m (1H); 1.72, m (2H); 1.46, m (2H).

$^{13}$C NMR ($CDCl_3$, ref. 77.00): $\delta$ 173.50, 130.42, 128.63, 80.23, 79.22, 61.74, 51.49, 48.95, 46.45, 33.86, 29.69, 29.31, 25.94, 22.92

D. [1S-[1α,2α(Z),3α,4α]]-6-[3-(Carboxy)-7-oxabicyclo [2.2.1]hept-2-yl]-4-hexenoic acid, methyl ester

To a solution of 2.43 g of impure Part C alcohol (80% pure = 1.94 g, 7.6 mmol, contaminated with triphenylphosphine oxide) in 40 mL acetone under argon at 0°, was added slowly 8 mL Jones' Reagent (2.6 M in $Cr^{VI}$). The red color of the reagent persisted toward the end of the addition. This resulting precipitated mixture was stirred for 20 minutes before 2-propanol was added to quench excess reagent. Still at 0°, 3 M aqueous $NaHSO_3$ solution was added with stirring until all salts dissolved. Brine was added, and extraction (3 times) with ethyl acetate followed. After drying the extracts over $Na_2SO_4$ and solvent evaporation, flash chromatography (150 g silica, 25% to 40% [5% acetic acid in ethyl acetate] in hexane gradient) afforded, after azeotropic removal of acetic acid with toluene, 1.91 g of an oil. This oil was impure title acid (80% pure = 1.53 g, contaminated with triphenylphosphine oxide), obtained in 75% yield.

| TLC (50% [5% acetic acid in ethyl acetate] in hexane - anisaldehyde): | |
|---|---|
| Part C alcohol | 0.33 |
| title acid | 0.35 |

$^{13}$C NMR (67.8MHz in $CDCl_3$):
175.3, 173.1, 129.1, 128.8, 78.0, 78.0, 51.6, 51.1, 47.4, 33.5, 28.8, 28.5, 26.9, 22.5

E. [1S-[1α,2α(Z),3α(R*),4]1]-6-[3-[[[2-[(4-Cyclohexylbutyl)amino]-1-(hydroxymethyl)-2-oxoethyl]amino]-carbonyl]-7-oxabicyclo[2.2.1]hept-2-yl]4-hexenoic acid, methyl ester

To a solution of 733 mg impure Part D acid (80% pure = 586 mg, 2.2 mmol, 1.1 equiv, contaminated with triphenylphosphine oxide) in 4 mL dry tetrahydrofuran (THF) under argon was added 356 mg 1,1'-carbonyldiimidazole (2.2 mmol, 1.1 equiv), and the mixture was left for 1 hour. Since a large volume of precipitate had formed, 5 mL dry THF was added, and the mixture was gently warmed to obtain a solution. (TLC showed a stable acylimidazole.) After stirring 30 minutes, a solution of 495 mg Part B amine (2.0 mmol) in 10mL dry THF was added using an additional 5 mL THF to quantitatively transfer the amine. TLC of the homogeneous mixture after 1 hour stirring at room temperature indicated very slow reaction. Therefore, THF was evaporated by passing argon over the mixture overnight until its volume was reduced to 2 mL and a precipitate had formed. Addition of 5 mL THF redissolved all precipitate. After 5 hours more stirring, the mixture was evaporated, and flash chromatography (150 g silica, 50% to 100% ethyl acetate in hexane gradient, then 0% to 10% $CH_3OH$ in ethyl acetate gradient) gave 230 mg of pure title hydroxybisamide as an oil. The yield of title hydroxybisamide was 23%.

Also isolated were the isomeric aminoesteramide (27%) and the 2:1 adduct (16%). These by-products could be converted in good yields to title hydroxybisamide by transesterification with KCN in $CO_3OH$ at room temperature, although the aminoesteramide may isomerize spontaneously.

| TLC (50% [5% acetic acid in ethyl acetate] in hexane - anisaldehyde): | |
|---|---|
| Part B amine | 0.00 |
| Part D acid | 0.38 |
| acylimidazole | 0.18 |
| title hydroxybisamide | 0.22 |
| aminoesteramide | 0.04 |
| 2:1 adduct | 0.33 |

$^{13}C$ NMR (67.8 MHz in $CDCl_3$):
173.3, 172.8, 170.4, 129.2, 129.0, 78.9, 78.8, 62.7, 54.0, 53.8, 51.3, 47.9, 39.4, 37.3, 36.9, 33.6, 33.1, 29.5, 29.4, 28.6, 27.2, 26.4, 26.1, 24.0, 22.6

F. [1S-[1α,2α(Z),3α(R*),4α]]-6-[3-[4-[[(4-Cyclohexylbutyl)amino]carbonyl]-4,5-dihydro-2-oxazolyl]-7-oxabicyclo[2.2.1]hept-2-yl]-4-hexenoic acid, methyl ester

This chemistry is described by M.J. Miller, P.G. Mattingly, M.A. Morrison, and J.F. Kerwin, Jr., J. Am. Chem. Soc., 1980, 102, 7026.

To a solution of 240 mg of pure Part E hydroxybisamide (0.48 mmol) in 3 mL dry THF under argon at room temperature, was added 189 mg triphenylphosphine (0.72 mmol, 1.5 equiv), 73 mg triethylamine (0.72 mmol, 1.5 equiv), and 89 mg $CCl_4$ (0.58 mmol, 1.2 equiv), and the mixture was heated to reflux. After 1 hour another aliquot each of $CCl_4$ and triethylamine were added, and after 2.5 hours more another aliquot of each were added again. 2 hours later another aliquot each of $CCl_4$ and triethylamine and half an aliquot (95 mg) of triphenylphosphine were added. After 2 hours more, TLC finally indicated complete consumption of Part E hydroxybisamide, and the initially colorless, homogeneous mixture had formed a white precipitate and had darkened. Solvent evaporation was followed by flash chromatography (silica, 15% acetone in toluene) which afforded 190 mg of pure title oxazoline, an oil. The oxazoline was obtained in 83% yield.

| TLC (20% acetone in toluene - anisaldehyde): | |
|---|---|
| Part E hydroxybisamide | 0.07 |
| title oxazoline | 0.29 |

$^{13}C$ NMA (67.8 MHz in $CDCl_3$):
173.1, 171.2, 169.1, 129.3, 128.9, 79.0, 78.9, 69.6, 68.3, 51.3, 48.2, 46.3, 39.0, 37.4, 36.9, 33.7, 33.1, 29.6, 29.5, 28.7, 27.1, 26.5, 26.2, 24.0, 22.7

G. [1S-[1α,2α(Z),3α,4α]]-6-[3-[4-[[(4-Cyclohexylbutyl)amino]carbonyl]-2-oxazolyl]-7-oxabicyclo[2.2.1]hept-2-yl]-4-hexenoic acid, methyl ester

This chemistry is described by D.L. Evans, D.K. Minster, U. Jordis, S.M. Hecht, A.L. Mazzu, Jr., and A.I. Meyers, J. Org. Chem., 1979, 44, 497.

To a solution of 190 mg of pure Part F oxazoline (0.40 mmol) in 10 mL $CHCl_3$, was added 200 mg untitrated $NiO_2$, and the heterogenous mixture was stirred at room temperature. TLC indicated some progress in the first 1 hour, but then reaction stopped. Over 1 day, five additional aliquots of the reagent were added until the reaction was complete. The mixture was diluted with ethyl acetate, and this was stirred with 3 M aqueous $NaHSO_3$ solution until the black color of the $NiO_2$ disappeared and most of the solids dissolved. Extraction (3 times) with ethyl acetate was followed by drying over $Na_2SO_4$ and evaporation. Flash chromatography (silica, 25% to 35% ethyl acetate in hexane gradient) afforded 90 mg of pure title oxazole, a solid. The oxazole was obtained in 48% yield.

| TLC (100% ethyl acetate - anisaldehyde): | |
|---|---|
| Part F oxazoline | 0.52 |
| title oxazole | 0.81 |

$^{13}C$ NMA (67.8 MHz in $CDCl_3$):
173.2, 163.8, 160.5, 140.4, 136.0, 129.4, 128.5, 79.5, 79.3, 51.4, 49.6, 46.6, 39.0, 37.4, 37.0, 33.7, 33.3, 29.8, 29.7, 28.9, 27.8, 26.6, 26.3, 24.1, 22.7

H. [1S-[1α,2α(Z),3α,4α]]-6-[3-[4-[[(4-Cyclohexylbutyl)amino]carbonyl]-2-oxazolyl]-7-oxabicyclo[2.2.1]hept-2-yl-4-hexenoic acid

To 90 mg of pure Part G oxazole (0.19 mmol) in 4 mL $CH_3OH$ at room temperature, was added 2 mL of 1.0 M aqueous NaOH solution. After stirring the mixture for 1.3 hours, 1 M aqueous HCl solution was added to lower the pH to 1. Extraction with ethyl acetate (3 times) followed. The extracts were dried over $Na_2SO_4$, and solvent evaporation gave crude title acid. Flash chromatography (25% to 50% [5% acetic acid in ethyl acetate] in hexane gradient) afforded, after azeotropic removal of acetic acid with toluene, 71 mg of pure title acid as a solid. The yield of title acid was 81%.

| TLC (50% [5% acetic acid in ethyl acetate] in hexane - anisaldehyde): | |
|---|---|
| Part G oxazole | 0.43 |
| title acid | 0.25 |

$^{13}C$ NMR (67.8 MHz in $CDCl_3$):
176.9, 163.9, 160.7, 140.9, 135.7, 129.5, 128.4, 79.5, 79.3, 49.6, 46.5, 39.1, 37.4, 36.9, 33.7, 33.2, 29.7, 29.7, 28.8, 27.8, 26.6, 26.2, 24.1, 22.5

## Example 2

[1S-[1α,2α(Z),3α,4α]]-6-[3-[4-[[(4-Cyclohexylbutyl)methylamino]carbonyl]-2-oxazolyl]-7-oxabicyclo[2.2.1]hept-2-yl]-4-hexenoic acid

A. N[(1,1-(Dimethylethoxy)carbonyl]-O-(phenylmethyl)-L-serine, 2-(trimethylsilyl)ethyl ester

This chemistry is described by P. Sieber, Helv. Chim. Acta, (1977), 60, 2711.

To a solution of 20.7 g of N-t-butyloxycarbonyl-O-benzyl-(L)-serine (70 mmol), 11.0 g pyridine (139 mmol, 2.0 equiv), and 9.9 g 2-trimethylsilylethanol (84 mmol, 1.2 equiv) in 50 mL dry $CH_3CN$ stirring under argon at 0°, was added 15.8 g 1,3-dicyclohexylcarbodiimide (76 mmol, 1.1 equiv) in a single portion. A precipitate formed. After 3 hours the mixture was warmed to room temperature and stirred for 12 hours. A solution of 1.4 g oxalic acid dihydrate (11 mmol, 0.15 equiv) in 3 mL dimethylformamide (DMF) was added, and the mixture was stirred for 1 hour before filtration. The filtercake was washed with ethyl acetate until the filtrate was free of title ester. The filtrate was washed twice with 1 M aqueous HCl solution plus brine and twice with 1 M aqueous $NaHCO_3$ solution. Drying over $Na_2SO_4$ and evaporation gave 34.8 g of crude title ester (79% pure = 27.6 g, contaminated with solvent and 2-trimethylsilylethanol) as an oil in 100% yield.

| TLC (50% [5% acetic acid in ethyl acetate] in hexane - anisaldehyde): | |
|---|---|
| N-BOC-O-benzyl-(L)-serine | 0.40 |
| Part A ester | 0.84 |

$^{13}C$ NMR (67.8 MHz in $CDCl_3$): 170.5, 155.3, 137.5, 128.2, 127.6, 127.4, 79.5, 73.0, 69.9, 63.6, 54.0, 28.1, 17.2, -1.7

B. N-[(1,1-Dimethylethoxy)carbonyl]-L-serine, 2-(trimethylsilyl)ethyl ester

To a solution of 34.8 g of crude Part A ester (79% pure = 27.6 g, contaminated with solvent and 2-trimethylsilylethanol, 70 mmol) in 200 mL ethyl acetate plus 300 mL acetic acid at room temperature under argon was added 10 g of 30% Pd on carbon catalyst. The mixture was then stirred under an atmosphere of $H_2$ for 4 days. TLC showed nearly complete clean conversion. The mixture was filtered through a polycarbonate membrane, and after evaporation of the solvent, residual acetic acid was removed by azeotroping with toluene and $CH_2Cl_2$. Flash chromatography (750 g silica, 10% to 30% ethyl acetate in hexane gradient) gave 3.58 g of pure starting material, Part A ester, (13% yield) and 16.49 g of pure title debenzylated ester as an oil. The yield of title ester was 78%.

| TLC (25% ethyl acetate in hexane - anisaldehyde): | |
|---|---|
| Part A ester | 0.61 |
| Part B ester | 0.28 |

$^{13}C$ NMR (67.8 MHz in $CDCl_3$): 170.9, 155.6, 79.9, 63.8, 63.0, 55.8, 28.1, 17.2, -1.7

C. L-serine, 2-(Trimethylsilyl)ethyl ester, monohydrochloride

This chemistry is described by P. Sieber, R.H. Andreatta, K. Eisler, B. Kamber, B. Riniker, and H. Rink, Peptides: Proceedings of the Fifth American Peptide Symposium, M. Goodman and J. Meienhofer, Eds., Halsted Press, New York. (1977), pp. 543-545.

To a solution of 10.4 g of Part B ester (34.1 mmol) in 200 mL diethyl ether was added 40 mL of an approximately 6.5 M HCl/methanol/methyl acetate solution (260 mmol). (This solution was prepared by adding 42 g acetyl chloride (0.54 mmol) dropwise to 35 g methanol (1.1 mmol) stirring at 0° followed by stirring at room temperature for 2 hours). The mixture was then stirred for 4 hours during which gas slowly evolved. While stirring in a room temperature bath, 32.8 g $NaHCO_3$ (390 mmol) was added cautiously in small portions to control gas evolution during neutralization. The mixture was then filtered through a glass frit, using 50% methanol in diethyl ether to wash the filtercake. Evaporation of the solvent gave 8.06 g of the semisolid, crude, nearly pure title amine hydrochloride. This material may have been only a partial salt, but assuming a pure 1:1 salt the yield was 98%.

| TLC (10% [10% concentrated aqueous $NH_3$ in $CH_3OH$] in $CH_2Cl_2$-anisaldehyde): | |
|---|---|
| Part B ester | 0.63 |
| Part C amine | 0.22 |

$^{13}C$ NMR (67.8 MHz in $CDCl_3$): 171.3, 63.9, 61.9, 55.5, 17.1, -1.8

D. [1S-[1α,2α(Z),3α(R*),4α]]-6-[3-[[[1-(Hydroxymethyl)-2-oxo-2-[(trimethylsilyl)ethoxy]ethyl]amino]carbonyl]-7-oxabicyclo[2.2.1]hept-2-yl]-4-hexenoic acid, methyl ester

To a solution of 9.40 g, Example 1, Part D acid (85% pure = 7.99 g, 29.8 mmol), 8.04 g of crude, nearly pure Part C amine hydrochloride (95% pure = 7.60 g, 31.6 mmol, 1.06 equiv), 4.43 g 1-hydroxybenzotriazole hydrate (32.8 mmol, 1.1 equiv), and 4.24 g diisopropylethylamine (32.8 mmol, 1.1 equiv) in 50 mL dry THF stirring under argon at room temperature, was added approximately 6.8 g 1,3-dicyclohexylcarbodiimide (33 mmol, 1.1 equiv) in a single portion. A precipitate slowly formed. After 16 hours, the solvent was evaporated, and flash chromatography (silica, 30% to 100% ethyl acetate in hexane gradient) gave an oil containing a solid. This material was taken up in diethyl ether in which the solid did not dissolve, and filtered. Evaporation gave 9.45 g of nearly pure title amide (90% pure = 8.51 g), a clear oil. The yield of title amide was 63%.

| TLC (10% [10% concentrated aqueous $NH_3$ in $CH_3OH$] in $CH_2Cl_2$ - anisaldehyde): | |
|---|---|
| Part C amine | 0.37 |
| Part D amide | 0.65 |
| TLC (50% [5% acetic acid in ethyl acetate] in hexane - anisaldehyde): | |
| Example 1, Part D acid | 0.42 |
| Part D amide | 0.36 |

$^{13}$CNMR (67.8 MHz in $CDCl_3$): 173.5, 172.4, 170.3, 129.4, 129.1, 79.2, 79.1, 63.9, 63.2, 54.6, 54.5, 51.4, 47.8 33.7, 29.7, 28.5, 27.3, 22.7, 17.2, -1.7

E. [1S-[1α,2α(Z),3α(R*),4α]]-6-[3-[4,5-Dihydro-4-[[2-(trimethylsilyl)ethoxy]carbonyl]-2-oxazolyl]-7-oxabicyclo-[2.2.1]hept-2-yl]-4-hexenoic acid, methyl ester

To a solution of 8.3 g of nearly pure Part D amide (90% pure = 7.5 g, 16.6 mmol) in 150 mL dry $CH_3CN$ under argon at room temperature (room temperature bath), was added 13.1 g triphenylphosphine (50 mmol, 3.0 equiv), 6.4 g diisopropylethylamine (50 mmol, 3.0 equiv), and finally 7.7 g $CCl_4$ (50 mmol, 3.0 equiv). After stirring for 2 hours, 1 M aqueous $NaHCO_3$ solution was added, and the mixture was extracted three times with $CH_2Cl_2$. Drying over $Na_2SO_4$ was followed by evaporation and flash chromatography (silica, 20% to 50% ethyl acetate in hexane gradient) which afforded 5.9 g of nearly pure title oxazoline (90% pure = 5.3 g), an oil. The oxazoline was obtained in 73% yield.

| TLC (50% ethyl acetate in hexane - anisaldehyde): | |
|---|---|
| Part D amide | 0.20 |
| Part E oxazoline | 0.44 |

$^{13}$C NMR (67.8 MHz in $CDCl_3$): 172.7, 170.7, 169.0, 129.0 128.8, 78.3, 78.3, 69.0, 67.5, 63.2, 50.9, 48.1, 46.0, 33.4, 29.3, 28.4, 26.7, 22.4, 16.8, -2.0

F. [1S-[1α,2α(Z),3α,4α]]-6-[3-[4-[[2-(Trimethylsilyl)ethoxy]carbonyl]-2-oxazolyl]-7-oxabicyclo[2.2.1]hept-2-yl]-4-hexenoic acid, methyl ester

This chemistry is described by D.L. Evans, D.K. Minster, U. Jordis, S.M. Hecht, A.L. Mazzu, Jr., and A.I. Meyers, J. Org. Chem., (1979), 44, 497.

To a solution of 5.1 g of nearly pure Part E oxazoline (90% pure = 4.6 g, 10.5 mmol) in 50 mL $CH_2Cl_2$,

was added 10.1 g untitrated $NiO_2$, and the heterogenous mixture was stirred at room temperature. (Exothermic reaction resulted in the mixture warming somewhat.) TLC indicated nearly complete reaction after 1 hour. An additional aliquot, 2.0 g, of $NiO_2$ was then added. After 30 minutes the reaction was complete, and 150 mL ethyl acetate was added. To reduce and dissolve the Ni salts, 100 mL 3 M aqueous $NaHSO_3$ solution and 200 mL 1 M aqueous trisodium citrate solution were added. Stirring caused all of the solids to dissolve, and the mixture warmed. Separation and extraction twice more with ethyl acetate (TLC indicated complete extraction of title oxazole) was followed by drying over $Na_2SO_4$ and evaporation. Flash chromatographic purification of the 3.8 g of crude product (150 g silica, 20% to 75% ethyl acetate in hexane gradient) afforded 2.60 g of pure title oxazole, an oil. The oxazole was obtained in 57% yield.

| TLC (50% ethyl acetate in hexane - anisaldehyde): | |
|---|---|
| Part E oxazoline | 0.34 |
| Part F oxazole | 0.58 |

$^{13}$C-NMR (67.8 MHz in $CDCl_3$): 172.9, 164.5, 161.0, 143.3, 133.0, 129.1, 128.4, 79.0, 78.9, 63.0 51.1, 49.5, 46.8, 33.5, 29.5, 28.7, 27.6, 22.5, 17.2, -1.8

G. [1S-[1α,2α(Z),3α,4α]]-6-[3-(4-Carboxy-2-oxazolyl)-7-oxabicyclo[2.2.1]hept-2-yl]-4-hexenoic acid, methyl ester

This chemistry is described by P. Sieber, R.H. Andreatta, K. Eisler, B. Kamber, B. Riniker, and H. Rink, Peptides: Proceedings of the Fifth American Peptide Symposium, M. Goodman and J. Meienhofer, Eds., Halsted Press, New York, (1977), pp.543-545.

To a solution of 3.1 g of pure Part F oxazole (7.1 mmol) in 20 mL dry DMF under argon, was added 12.0 g of tetrabutylammonium fluoride on silica (Fluka, 13.9 mmol, 1.96 equiv), and the heterogenous mixture was stirred at room temperature for 6 hours. The mixture was diluted with 20 mL of 1% $F_3CCO_2H$, 1% $CH_2OH$ 98% ethyl acetate and filtered using 40 mL of the same solvent to wash the filtercake. The filtrate was evaporated and azeotroped three times with toluene to remove the DMF. The crude product was purified on an ion exchange resin: After washing a 250 g column of AG 50W-X8 (hydrogen form) with water and then 50% $CH_3OH$ in water until the eluant was colorless, the crude product dissolved in 40 mL of 50% $CH_3OH$ in water was loaded, and elution was carried out with the same solvent. This afforded 2.65 g of nearly pure title oxazole acid (90% pure = 2.38 g), an oil. The oxazole acid was obtained in 100% yield.

| TLC (1% $F_3CCO_2H$, 1% $CH_3OH$, 98% ethyl acetate - anisaldehyde): | |
|---|---|
| Part F oxazole | 0.46 |
| Part G oxazole acid | 0.15 |

$^{13}$C NMR (67.8 MHz in $CD_3OD$): 175.0, 166.7, 163.8, 145.6, 134.2, 130.4, 129.7, 80.7, 80.7, 52.0, 50.7, 47.7, 34.6, 30.5, 29.7, 28.9, 23.8

H. N-Methyl-4-cyclohexylbutylamine, monohydrochloride

To a solution of 750 mg of 4-cyclohexylbutylamine hydrochloride (3.9 mmol) and 1.08 g triethylamine (10.8 mmol, 2.8 equiv) in 10 mL dry THF stirring under argon at 0°, was added 584 mg $ClCO_2C_2H_5$ (5.4 mmol, 1.4 equiv). After warming to room temperature, the heterogeneous mixture was stirred for 3 hours. Dilution with diethyl ether was followed by washing (twice) with 1 M aqueous HCl solution. The organic layer was dried over $Na_2SO_4$ and evaporated to obtain 950 mg of crude ethyl carbamate intermediate, an oil, contaminated with some of the imide.

To a solution of 950 mg of crude ethyl carbamate intermediate in 10 mL dry THF stirring under argon at

0°, was added 950 mg lithium aluminum hydride (25 mmol, 6.4 equiv). Gas was evolved. The mixture was then heated to reflux for 2 hours. After recooling to 0° and adding 20 mL diethyl ether, 1.0 mL water was cautiously added to quench excess hydride. The mixture was rewarmed to room temperature, and while stirring vigorously, 1.0 mL 15% aqueous NaOH solution, then 3.0 mL water were added. The mixture was filtered, washing the filter cake with 10% [10% concentrated aqueous $NH_3$ in $CH_3OH$] in diethyl ether, and the filtrate was evaporated. This material was evaporated several times with $CH_3OH$ to remove $NH_3$, and finally acidified with concentrated aqueous HCl solution. Azeotropic removal of water with toluene and $CH_3OH$ yielded, after exposure to high vacuum, 830 mg of impure secondary amine hydrochloride (80% pure = 660 mg, contaminated with tertiary amine hydrochloride) as a solid. The product was obtained in 83% yield and was taken on without purification.

| TLC (10% [10% concentrated aqueous $NH_3$ in $CH_3OH$] in $CH_2Cl_2$-anisaldehyde): | |
|---|---|
| 4-cyclohexylbutylamine | 0.07 |
| carbamate intermediate | 0.89 |
| Part H amine | 0.11 |

I. [1S-[1α,2α(Z),3α,4α]]-6-[3-[4-[[(4-Cyclohexylbutyl)methylamino]carbonyl]-2-oxazolyl]-7-oxabicyclo[2.2.1]-hept-2-yl]-4-hexenoic acid, methyl ester

A sample of impure Part G oxazole acid (contaminated with tetrabutylammonium, but as the free acid, 0.20 mmol) was dried by azeotroping with dry DMF and toluene twice (high vacuum). This material was placed in 2 mL toluene, and while stirring the heterogeneous mixture at room temperature under argon, 127 mg oxalyl chloride (1.0 mmol, 5 equiv) was added. Gas was evolved. After 1 hour an additional 127 mg oxalyl chloride was added. Again gas was evolved This mixture was stirred overnight. TLC indicated clean conversion to the acid chloride having the name [1S-[1α,2α(Z),3α,4α]]-6-[3-[4-(chlorocarbonyl)-2-oxazolyl]-7-oxabicyclo[2.2.1]hept-2-yl]-4-hexenoic acid, methyl ester. The solvent was evaporated, and toluene was added and evaporated again to purge any remaining oxalyl chloride.

To the crude acid chloride of the Part G acid was added 4 mL $CHCl_3$. The material did not all dissolve. While stirring under argon at room temperature, 100 mg of impure Part H secondary amine hydrochloride (80% pure = 80 mg, contaminated with tertiary amine hydrochloride, 0.39 mmol, 2 equiv) and 145 mg triethylamine (1.4 mmol, 7 equiv) were added. After stirring at room temperature for 1 hour, the mixture was diluted with ethyl acetate, water was added, and extraction (twice) with ethyl acetate was followed by drying over $Na_2SO_4$ and evaporation. This gave 120 mg of impure title amide as a gum. The crude product was taken on without purification.

| TLC (1% $F_3CCO_2H$, 1% $CH_3OH$, 98% ethyl acetate - anisaldehyde): | |
|---|---|
| Part G oxazole acid | 0.28 |
| acid chloride intermediate | 0.78 |
| Part I amide | 0.64 |

J. [1S-[1α,2α(Z),3α,4α]]-6-[3-[4-[[(4-Cyclohexylbutyl)methylamino]carbonyl]-2-oxazolyl]-7-oxabicyclo[2.2.1]-hept-2-yl]-4-hexenoic acid

To 120 mg of impure Part I amide in 6 mL $CH_3OH$ at room temperature, was added 2 mL of 1.0 M aqueous NaOH solution. After stirring the mixture for 3 hours, 1 M aqueous HCl solution was added to lower the pH to 1. Extraction with ethyl acetate (3 times) followed. The extracts were dried over $Na_2SO_4$, and solvent evaporation gave crude acid title product. Flash chromatography (50% to 100% [5% acetic acid in

ethyl acetate] in hexane gradient) afforded, after azeotropic removal of acetic acid with toluene, 70 mg of pure title product as an oil. The yield of title product was 72% overall from the impure Part G oxazole acid.

| TLC (50% [5% acetic acid in ethyl acetate] in hexane - anisaldehyde): | |
|---|---|
| Part I amide | 0.36 |
| Part J Title Product | 0.20 |

$^{13}C$ NMR (67.8 MHz in $CDCl_3$. Two conformations were seen. Lines clearly due to only one conformation are listed in parentheses. ):
177.0, 163.1, (162.3), (162.0), 142.5, 136.4, 129.6, 128.5, 79.5,79.4, (50.4), 49.7, (48.7), 46.7, 37.5, 37.0, (36.4), 34.1, 33.3, 29.7, 29.0, 27.9, (27.2), 26.6, 26.3, (24.2), (23.7), 22.9

## Example 3

[1S-[1α,2α(Z),3α,4α]]-6-[3-[4-[(1-Pyrrolidinyl)carbonyl]-2-oxazolyl]-7-oxabicyclo[2.2.1]hept-2-yl]-4-hexenoic acid

A. 1-[N-[(1,1-Dimethylethoxy)carbonyl]-L-seryl]pyrrolidine

To a stirred solution of pyrrolidine (1.11 g, 15.7 mmol), t-butyloxycarbonyl (BOC)-(L)-serine (3.22 g, 15.7 mmol), 1-hydroxybenzotriazole hydrate (2.12 g, 15.7 mmol) and diisopropylethyl amine (2.73 mL, 15.7 mmol) in 30 mL of THF under argon was added 1,3-dicyclohexylcarbodiimide (3.23 g, 15.7 mmol). This mixture was stirred at room temperature for 17 hours and concentrated in vacuo. The mixture was diluted with 200 mL ethyl acetate and the precipitate was filtered off. The precipitate was rinsed with ethyl acetate (3 x 40 mL). The combined filtrates were washed with 1N aqueous HCl solution (3 x 70 mL), and saturated $NaHCO_3$ solution (2 x 80 mL). The organic layer was dried ($MgSO_4$), filtered and concentrated in vacuo. This was chromatographed on 140 g of Merck silica gel 60 using 2% $CH_3OH$ in $CH_2Cl_2$ as eluant to give 1.64 g (41%) of title amide.
TLC: silica gel, 4% $CH_3OH$ in $CH_2CL_2$, $R_f$ 0.24, $Ce(SO_4)_2$
$^{13}C$ NMR (67.5 MHz, $CDCl_3$) δ: 169.4, 155.6, 79.7, 62.8, 53.5, 46.5, 45.9, 28.0, 28.0 28.0, 25.7, 23.8

B. [1S-[1α,2α(Z),3α(R*),4α]]-6-[3-[[[1-(Hydroxymethyl)-2-oxo-2-(1-pyrrolidinyl)ethyl]amino]carbonyl]-7-oxabicyclo[2.2.1]hept-2-y1]-4-hexenoic acid, methyl ester

To a stirred solution of Part A amide (0.96 g, 3.72 mmol) in 20 mL of dry $CH_2Cl_2$ under argon at 0° C was added 5 mL of trifluoroacetic acid (TFA). The mixture was stirred at 0° C for 2 hours and diluted with 50 mL of toluene. This mixture was concentrated in vacuo. To a stirred solution of this amine-TFA salt, 1-hydroxybenzotriazole hydrate (0.50 g, 3.73 mmol), and 5 mL of triethylamine in 20 mL of DMF was added a solution of Example 1, Part D acid (1.00 g, 3.73 mmol) in 10 mL of DMF. To this mixture was then added ethyl-3(3-dimethylamino)propyl carbodiimide hydrochloride salt. The reaction mixture was stirred at room temperature for 19 hours and concentrated in vacuo. The mixture was diluted with 400 mL of ethyl acetate and washed with 1N HCl solution (3 x 30 ml), 0.2 N NaOH solution (2 x 30 mL) and brine (1 x 60 mL). The organic layer was dried ($MgSO_4$), filtered, and concentrated in vacuo. Purification was effected by flash chromatography on 50 g of Merck silica gel 60 using 0.4 L each of 2% and 4% $CH_3OH$ in $CH_2Cl_2$ as eluants to give 320 mg (22%) of title alcohol.
TLC: silica gel, 4% $CH_3OH$ in $CH_2Cl_2$, $R_f$ 0.22, $Ce(SO_4)_2$.
$^{13}C$ NMR (67.5 MHz, $CDCl_3$) δ: 173.4, 172.2, 168.8, 129.3, 129.2, 78.9, 78.9, 63.5, 54.3, 52.2, 51.4, 48.0, 46.6, 45.9, 33.7, 29.5, 28.8, 27.2, 25.8, 24.0, 22.7

C. [1S-[1α,2α(Z),3α(R*),4α]]-6-[3-[4,5-Dihydro-4-[(1-pyrrolidinyl)carbonyl]-2-oxazolyl]-7-oxabicyclo[2.2.1]hept-2-yl]-4-hexenoic acid, methyl ester

To a stirred solution of Part B alcohol (305 mg, 0.75 mmol) and diisopropylethyl amine (0.39 mL, 2.24 mmol) in 10 mL of $CH_2Cl_2$ under argon at 0° C was added methanesulfonyl chloride (0.07 mL, 0.90 mmol). This mixture was stirred at room temperature for 4 hours and concentrated in vacuo. The crude mesylate was dissolved in 30 mL of acetone and combined with 0.60 g of $K_2CO_3$. This mixture was heated to reflux for 4 hours, cooled to room temperature, and diluted with 100 mL of acetone. The precipitate was filtered off and rinsed with acetone (3 x 40 mL). The filtrate was concentrated in vacuo and chromatographed on 50 g of Merck silica gel 60 using 2% $CH_3OH$ in $CH_2Cl_2$ as eluant to give 210 mg (72%) of title oxazoline.
TLC: silica gel, 4% $CH_3OH$ in $CH_2Cl_2$, $R_f$ 0.20, $Ce(SO_4)_2$.
$^{13}C$ NMR (67.5 MHz, $CDCl_3$) δ: 173.4, 168.2, 167.7, 129.5, 129.1, 78.9, 78.9, 68.7, 67.5, 51.4, 48.4, 46.5, 46.4, 46.1, 33.9, 29.7, 28.8, 27.1, 25.9, 24.1, 22.8

D. [1S-[1α,2α(Z),3α,4α]]-6-[3-[4-[(1-Pyrrolidinyl)carbonyl]-2-oxazolyl]-7-oxabicyclo[2.2.1]hept-2-yl]-4-hexenoic acid, methyl ester

To a stirred solution of Part C oxazoline (200 mg, 0.51 mmol) in 5 mL of $CH_2Cl_2$ was added 200 mg of $NiO_2$. The reaction mixture was stirred at room temperature for 3 hours at which time another 200 mg of $NiO_2$ was added. The mixture was stirred at room temperature for 1 hour and 200 mg of $NiO_2$ was added. This mixture was stirred for another 1 hour and one more portion of 200 mg of $NiO_2$ was added. The reaction mixture was stirred at room temperature for 17 hours and diluted with 80 mL of ethyl acetate. To the resulting mixture was added 5 mL of 3M $NaHSO_3$ solution and 40 mL of 1M sodium citrate solution. The organic layer was separated and the aqueous layer was extracted with ethyl acetate (3 x 70 mL). The combined organic extracts were dried ($MgSO_4$), filtered, and concentrated in vacuo. This was chromatographed on 18 g of Merck silica gel 60 using 2% $CH_3OH$ in $CH_2Cl_2$ as eluant to give 69.2 mg (35%) of title ester.
TLC: silica gel, 4% $CH_3OH$ in $CH_2Cl_2$, $R_f$ 0.24, $Ce(SO_4)_2$.
$^{13}C$ NMR (67.5 MHz, $CDCl_3$) δ: 173.2, 163.1, 160.3, 142.2, 137.1, 129.3, 128.7, 79.4, 79.1, 51.4, 49.6, 48.2, 46.7, 46.6, 33.7, 29.7, 28.9, 27.8, 26.4, 23.7, 22.7

E. [1S[1α,2α(Z),3α,4α]]-6-[3-[4-[(1-Pyrrolidinyl )carbonyl]-2-oxazolyl]-7-oxabicyclo[2.2.1]hept-2-yl]-4-hexenoic acid

To a stirred solution of Part D ester (69.0 mg, 0.18 mmol) and 2 mL of water in 12 mL of THF was added 2 mL of 1N LiOH solution. This mixture was sparged with argon for 10 minutes and stirred at room temperature for 7 hours. The mixture was acidified to pH 2 by the addition of 1N HCl solution and saturated with NaCl. The THF layer was separated and the aqueous layer was extracted with ethyl acetate (4 x 60 mL). The combined organic extracts were dried ($MgSO_4$), filtered and concentrated in vacuo. Purification was effected by flash chromatography on 10 g of Merck silica gel 60 using 10% $CH_3OH$ in $CH_2Cl_2$ as eluant to give 26 mg (39%) of title acid.
TLC: silica gel, 6% $CH_3OH$ in $CH_2Cl_2$, $R_f$ 0.22, $Ce(SO_4)_2$.
$^{13}C$ NMR (67.5 MHz, DMSO-$d_6$) δ: 175.1, 163.2, 159.6, 142.2, 136.3, 130.5, 127.6, 78.8, 78.4, 48.8, 47.7, 46.3, 45.8, 29.3, 28.4, 27.5, 25.9, 23.3, 23.2, 22.8

## Example 4

[1S-[1α,2α(Z),3α,4α]]-6-[3-[4-[(Cyclohexylamino)carbonyl]-2-oxazolyl]-7-oxabicyclo[2.2.1]hept-2-yl]-4-hexenoic acid

A. [(1,1-Dimethylethoxy)carbonyl]-N-cyclohexyl-L-serinamide

To a stirred solution of cyclohexylamine (1.11 g, 15.7 mmol), Boc-(L)-serine (3.22 g, 15.7 mmol), 1-hydroxybenzotriazole hydrate (2.12 g, 15.7 mmol) and diisopropylethyl amine (2.73 mL, 15.7 mmol) in 30 mL of THF under argon at 0° C was added 1,3-dicyclohexylcarbodiimide (3.23 g 15.7 mmol). This mixture was stirred at room temperature for 18 hours. The mixture was diluted with 200 mL ethyl acetate and the precipitate was filtered. The precipitate was rinsed with ethyl acetate (3 x 40 mL). The combined filtrates were washed with 1N aqueous HCl solution (3 x 70 mL), and saturated $NaHCO_3$ solution (2 x 80 mL). The organic layer was dried ($MgSO_4$), filtered and concentrated in vacuo. This was chromatographed on 140 g of Merck silica gel 60 using 2% $CH_3OH$ in $CH_2Cl_2$ as eluant to give 1.64 g (41%) of title amide.
TLC: silica gel, 4% $CH_3OH$ in $CH_2Cl_2$, $R_f$ 0.24, $Ce(SO_4)_2$.
$^{13}C$ NMR (67.5 MHz, $CDCl_3$) δ: 170.1, 156.1, 80.2, 62.7, 48.1, 32.6, 32.6, 28.1, 28.1, 28.1, 25.3, 24.5, 24.5, 24.5

B. [1S-[1α,2α(Z),3α(R*),4α]]-6-[3-[[[2-(Cyclohexylamino)-1-(hydroxymethyl)-2-oxoethyl]amino]carbonyl]-7-oxabicyclo[2.2.1]hept-2-yl]-4-hexenoic acid, methyl ester

To a stirred solution of Part A amide (1.06 g, 3.73 mmol) in 20 mL of dry $CH_2Cl_2$ under argon at 0° C was added 5 mL of TFA. The mixture was stirred at 0° C for 2 hours and diluted with 50 mL of toluene. This mixture was concentrated in vacuo. To a stirred solution of this amine-TFA salt, 1- hydroxybenzotriazole hydrate (0.50 g, 3.73 mmol), and 6 mL of triethyl amine in 20 mL of DMF was added a solution of Example 1, Part D acid (1.00 g, 3.73 mmol) in 10 mL of DMF. To this mixture was then added ethyl-3-(3-dimethylamino)propyl carbodiimide hydrochloride salt (0.55 g, 3.73 mmol). The reaction mixture was stirred at room temperature for 19.5 hours and concentrated in vacuo. The mixture was diluted with 400 mL of ethyl acetate and washed with 1N HCl solution (3 x 40 mL), 0.2 N NaOH solution (2 x 30 mL), saturated $NaHCO_3$ solution (1 x 30 mL) and brine (1 x 100 mL). The organic layer was dried ($MgSO_4$), filtered, and concentrated in vacuo. Purification was effected by flash chromatography on 60 g of Merck silica gel 60 using 2% $CH_3OH$ in $CH_2Cl_2$ as eluant to give 690 mg (42%) of title alcohol.
TLC: Silica gel, 4% $CH_3OH$ in $CH_2Cl_2$, $R_f$ 0.30, $Ce(SO_4)_2$.
$^{13}C$ NMR (67.5 MHz in, $CDCl_3$) δ: 173.3, 173.0, 169.6, 129.4, 129.0, 79.1, 78.9, 62.7, 54.4, 53.7, 51.4, 48.2, 48.0, 33.7, 32.6, 32.6, 32.6, 29.5, 28.6, 27.5, 25.3, 24.6, 24.6, 22.7

C. [1S-[1α,2α(Z),3α(R*),4α]]-6-[3-[4-[(Cyclohexylamino)carbonyl]-4,5-dihydro-2-oxazolyl]-7-oxabicyclo[2.2.1]-hept-2-yl]-4-hexenoic acid, methyl ester

To a stirred solution of Part B alcohol (680 mg, 1.56 mmol) and diisopropylethyl amine (0.82 mL, 4.68 mmol) in 20 mL of $CH_2Cl_2$ under argon at 0° C was added methanesulfonyl chloride (0.13 mL, 1.63 mmol). This mixture was stirred at room temperature for 1 hour and concentrated in vacuo. The crude mesylate was dissolved in 20 mL of acetone and combined with 647 mg of $K_2CO_3$. This mixture was heated at 54° C for 3.5 hours, cooled to room temperature and diluted with 100 mL of acetone. The precipitate was filtered off and rinsed with acetone (3 x 50 mL). The filtrate was concentrated in vacuo and chromatographed on 50 g of Merck silica gel 60 using 2% $CH_3OH$ in $CH_2Cl_2$ as eluant to give 540 mg (83%) of title oxazoline.
TLC: silica gel, 4% $CH_3OH$ in $CH_2Cl_2$, $R_f$ 0.42, $Ce(SO_4)_2$.
$^{13}C$ NMR (67.5 MHz, $CDCl_3$) δ: 173.2, 170.4, 169.2, 129.5, 128.9, 79.1, 79.0, 69.7, 68.4, 51.4, 48.4, 47.8, 46.4, 33.8, 33.0, 32.8, 29.6, 28.9, 27.3, 25.4, 24.7, 24.7, 22.8

D. [1S-[1α,2α(Z),3α,4α]]-6-[3-[4-[(Cyclo hexylamino)carbonyl]-2-oxazolyl]-7-oxabicyclo[2.2.1]hept-2-yl]-4-hexenoic acid, methyl ester

To a stirred solution of Part C oxazoline (530 mg, 1.26 mmol) in 5 mL of $CH_2Cl_2$ was added 1.06 g of $NiO_2$. The reaction mixture was stirred at room temperature for 3 hours at which time another 530 mg of $NiO_2$ was added. The mixture was stirred at room temperature for 1 hour and 530 mg of $NiO_2$ was added. This mixture was stirred for another 80 minutes and diluted with 120 mL of ethyl acetate. To the resulting mixture was added 10 mL of 3M $NaHSO_3$ solution and 70 mL of 1M sodium citrate solution. The organic layer was separated and the aqueous layer was extracted with ethyl acetate (2 x 100 mL). The combined organic extracts were washed with brine (1 x 100 mL), dried ($MgSO_4$), filtered, and concentrated in vacuo. This was chromatographed on 40 g of Merck silica gel 60 using 2% $CH_3OH$ in $CH_2Cl_2$ as eluant to give 370

mg (70%) of title ester.
TLC: silica gel, 4% $CH_3OH$ in $CH_2Cl_2$, $R_f$ 0.64, $Ce(SO_4)_2$.
$^{13}C$ NMR (67.5 MHz, $CDCl_3$) δ: 173.2, 163.7, 159.6, 140.6, 136.1, 129.4, 128.5, 79.5, 79.3, 51.4, 49.6, 47.8, 46.6, 33.7, 33.0, 33.0, 29.7, 28.8, 27.8, 25.4, 24.9, 24.9, 22.7

E. [1S-[1α,2α(Z),3α,4α]]-6-[3-[4-[(Cyclohexylamino)carbonyl]-2-oxazolyl]-7-oxabicyclo[2.2.1]hept-2-yl]-4-hexenoic acid

To a stirred solution of Part D ester (360 mg, 0.86 mmol) and 10 mL of water in 60 mL of THF was added 10 mL of 1N LiOH solution. This mixture was sparged with argon for 10 minutes and stirred at room temperature for 7 hours. The mixture was acidified to pH 2 by the addition of 1N HCl solution and saturated with NaCl. The THF layer was separated and the aqueous layer was extracted with ethyl acetate (4 x 80 mL). The combined organic extracts were dried ($MgSO_4$), filtered and concentrated in vacuo. Purification was effected by flash chromatography on 40 g of Merck silica gel 60 using 4% $CH_3OH$ in $CH_2Cl_2$ as eluant to give 300 mg (86%) of title acid.
TLC: silica gel, 6% $CH_3OH$ in $CH_2Cl_2$, $R_f$ 0.32, $Ce(SO_4)_2$.
$^{13}C$ NMR (67.5 MHz, $CDCl_3$) δ: 176.9, 163.9, 160.0, 141.0, 129.5, 128.5, 79.5, 79.5, 48.1, 46.6, 33.7, 32.9, 32.9, 29.7, 28.9, 27.9, 25.5, 24.9, 24.9

## Example 5

[1S-[1α,2α(Z),3α,4α]]-6-[3-[4-[[(2-Cyclohexylethyl)amino]carbonyl]-2-oxazolyl]-7-oxabicyclo[2.2.1]hept-2-yl]-4-hexenoic acid

A. [(1,1-Dimethylethoxy)carbonyl]-N-(2-cyclohexylethyl-L-serinamide

To a stirred solution of 2-cyclohexylethylamine (2.00 g, 15.7 mmol), Boc-(L)-serine (3.22 g, 15.7 mmol), 1-hydroxybenzotriazole hydrate (2.12 g, 15.7 mmol) and diisopropylethyl amine (2.73 mL, 15.7 mmol) in 40 mL of THF under argon at 0° C was added 1,3-dicyclohexylcarbodiimide (3.23 g, 15.7 mmol). This mixture was stirred at room temperature and 10 mL of DMF was added. The reaction mixture was then stirred at room temperature for 18 hours. The mixture was diluted with 300 mL ethyl acetate and the precipitate was filtered. The precipitate was rinsed with ethyl acetate (3 x 40 mL). The combined filtrate was washed with 1N aqueous HCl solution (3 x 70 mL), and saturated $NaHCO_3$ solution (2 x 80 mL). The organic layer was dried ($MgSO_4$), filtered and concentrated in vacuo. This was chromatographed on 160 g of Merck silica gel 60 using 1:4 hexane-diethyl ether as eluant to give 1.91 g (39%) of title amide.
TLC: silica gel, 4% $CH_3OH$ in $CH_2Cl_2$, $R_f$ 0.34, $Ce(SO_4)_2$.
$^{13}C$ NMR (67.5 MHz, $CDCl_3$) δ: 171.1, 80.3, 62.9, 37.3, 36.8, 35.2, 33.1, 33.1, 28.3, 28.3, 28.3, 26.4, 26.1, 26.1

B. [1S-[1α,2α(2),3α(R*),4α]]-6-[3-[[[2-[(2-Cyclohexylethyl)amino]-1-(hydroxymethyl)-2-oxoethyl]amino]-carbonyl]-7-oxabicyclo[2.2.1]hept-2-yl]-4-hexenoic acid, methyl ester

To a stirred solution of Part A amide (1.28 g, 3.73 mmol) in 20 mL of dry $CH_2Cl_2$ under argon at 0° C was added 5 mL of TFA. The mixture was stirred at 0° C for 2 hours and diluted with 50 mL of toluene. This mixture was concentrated in vacuo. To a stirred solution of this amine-TFA salt, 1-hydroxybenzotriazole hydrate (0.50 g, 3.73 mmol), and 5 mL of triethylamine in 20 mL of DMF was added a solution of Example 1, Part D acid (1.00 g, 3.73 mmol) in 10 mL of DMF. To this mixture was then added ethyl-3(3-dimethylamino)propyl carbodiimide hydrochloride salt (0.55 g, 3.73 mmol). The reaction mixture was stirred at room temperature for 17 hours and concentrated in vacuo. The mixture was diluted with 400 mL of ethyl acetate and washed with 1N HCl solution (3 x 40 mL), 0.2 N NaOH solution (2 x 30 mL), saturated $NaHCO_3$ solution (1 x 30 mL) and brine (1 x 100 mL). The organic layer was dried ($MgSO_4$), filtered, and concentrated in vacuo. Purification was effected by flash chromatography on 60 g of Merck silica gel 60

using 2% $CH_3OH$ in $CH_2Cl_2$ as eluant to give 1.00 g (54%) of title alcohol.
TLC: silica gel, 4% $CH_3OH$ in $CH_2Cl_2$, $R_f$ 0.30, $Ce(SO_4)_2$.
$^{13}C$ NMR (67.5 MHz, $CDCl_3$) δ: 173.4, 172.9, 170.4, 129.3, 129.0, 79.0, 78.9, 62.7, 54.2, 53.7, 51.4, 48.0, 37.3, 36.6, 35.3, 33.7, 32.9, 32.9, 29.5, 28.6, 27.3, 26.3, 26.0, 26.0, 22.7

C. [1S-[1α,2α(Z),3α(R*),4α]]-6-[3-[4-[[(2-Cyclohexylethyl)amino]carbonyl]-4,5-dihydro-2-oxazolyl]-7-oxabicyclo[2.2.1]hept-2-yl]-4-hexenoic acid, methyl ester

To a stirred solution of Part B alcohol (890 mg, 1.80 mmol) and diisopropylethyl amine (0.94 mL, 5.40 mmol) in 30 mL of $CH_2Cl_2$ under argon at 0° C was added methanesulfonyl chloride (0.14 mL, 1.80 mmol). This mixture was stirred at room temperature for 1 hour and concentrated in vacuo. The crude mesylate was dissolved in 30 mL of acetone and combined with 0.77 g of $K_2CO_3$. This mixture was heated to reflux for 4 hours, cooled to room temperature, and diluted with 100 mL of acetone. The precipitate was filtered off and rinsed with acetone (3 x 50 mL). The filtrate was concentrated in vacuo and chromatographed on 60 g of Merck silica gel 60 using 2% $CH_3OH$ in $CH_2Cl_2$ as eluant to give 480 mg (54%) of title oxazoline.
TLC: silica gel, 4% $CH_3OH$ in $CH_2Cl_2$, $R_f$ 0.42, $Ce(SO_4)_2$.
$^{13}C$ NMR (67.5 MHz, $CDCl_3$) δ: 173.1, 171.2, 169.2, 129.4, 128.9, 79.1, 79.0, 69.6, 68.3, 51.4, 48.3, 46.3, 36.9, 36.8, 35.2, 33.7, 33.0, 32.9, 29.6, 28.8, 27.2, 26.3, 26.1, 26.1, 22.8

D. [1S-[1α,2α(Z),3α,4α]]-6-[3-[4-[[(2-Cyclohexylethyl)amino]carbonyl]-2-oxazolyl]-7-oxabicyclo[2.2.1]hept-2-yl]-4-hexenoic acid, methyl ester

To a stirred solution of Part C oxazoline (480 mg, 1.01 mmol) in 20 mL of $CH_2Cl_2$ was added 480 mg of $NiO_2$. The reaction mixture was stirred at room temperature for 1 hour at which time another 480 mg of $NiO_2$ was added. The mixture was stirred at room temperature for 3 hours and 480 mg of $NiO_2$ was added. This mixture was stirred for another 15.5 hours and one more portion of 480 mg of $NiO_2$ was added. The reaction mixture was stirred for 1.5 hours and diluted with 100 mL of ethyl acetate. To the resulting mixture was added 20 mL of 3M $NaHSO_3$ solution and 30 mL of 1M sodium citrate solution. The organic layer was separated and the aqueous layer was extracted with ethyl acetate (2 x 100 mL). The combined organic extracts were washed with brine (1 x 50 mL), dried ($MgSO_4$), filtered, and concentrated in vacuo. This was chromatographed on 45 g of Merck silica gel 60 using 2% $CH_3OH$ in $CH_2Cl_2$ as eluant to give 200 mg (42%) of title ester.
TLC: silica gel, 4% $CH_2OH$ in $CH_3Cl_2$, $R_f$ 0.60, $Ce(SO_4)_2$.
$^{13}C$ NMR (67.5 MHz, $CDCl_3$) δ: 173.1, 163.7, 160.4, 140.3, 136.0, 129.3, 128.4, 79.4, 79.3, 51.3, 49.6, 46.6, 36.9, 36.7, 35.2, 33.6, 33.0, 33.0, 29.7, 28.8, 27.7, 26.4, 26.0, 26.0, 22.7

E. [1S-[1α,2α(Z),3α,4α]]-6-[3-[4-[[(2-Cyclohexylethyl)amino]carbonyl]-2-oxazolyl]-7-oxabicyclo[2.2.1]hept-2-yl]-4-hexenoic acid

To a stirred solution of Part D ester (190 mg, 0.40 mmol) and 4 mL of water in 30 mL of THF was added 4 mL of 1N LiOH solution. This mixture was sparged with argon for 10 minutes and stirred at room temperature for 5 hours. The mixture was acidified to pH 2 by the addition of 1N HCl solution and saturated with NaCl. The THF layer was separated and the aqueous layer was extracted with ethyl acetate (4 x 25 mL). The combined organic extracts were dried ($MgSO_4$), filtered and concentrated in vacuo. Purification was effected by flash chromatography on 20 g of Merck silica gel 60 using 4% $CH_3OH$ in $CH_2Cl_2$ as eluant to give 106.7 mg (59%) of title acid.
TLC: silica gel, 6% $CH_3OH$ in $CH_2Cl_2$, $R_f$ 0.32, $Ce(SO_4)_2$.
$^{13}C$ NMR (67.5 MHz, $CDCl_3$) δ: 176.9, 163.9, 160.7, 140.8, 135.7, 129.4, 128.4, 79.5, 79.4, 49.6, 46.5, 36.9, 36.8, 35.2, 33.7, 33.0, 33.0, 29.6, 28.8, 27.8, 26.4, 26.1, 26.1, 22.6

## Example 6

[1S-[1α,2α(Z),3α,4α]]-6-[3-[4-[[[2-(4-Chlorophenyl)ethyl]amino]carbonyl]-2-oxazolyl]-7-oxabicyclo[2.2.1]hept-2-yl]-4-hexenoic acid.

A. [(1,1-Dimethylethoxy)carbonyl]-N-[2-(4-chlorophenyl)ethyl]-L-serinamide

To a stirred solution of 2-(4-chlorophenyl)ethylamine (2.44 g, 15.7 mmol), Boc-(L)-serine (3.22 g, 15.7 mmol), 1-hydroxybenzotriazole hydrate (2.12 g, 15.7 mmol) and diisopropylethyl amine (2.73 mL, 15.7 mmol) in 30 mL of THF under argon at 0° C was added 1,3-dicyclohexylcarbodiimide (3.23 g, 15.7 mmol). This mixture was stirred at room temperature and 10 mL of DMF was added. The reaction mixture was then stirred at room temperature for 18 hours. The mixture was diluted with 200 mL ethyl acetate and the precipitate was filtered. The precipitate was rinsed with ethyl acetate (3 x 40 mL). The combined filtrates was washed with 1N aqueous HCl solution (3 x 70 mL), and saturated $NaHCO_3$ solution (2 x 80 mL). The organic layer was dried ($MgSO_4$), filtered and concentrated in vacuo. This was chromatographed on 160 g of Merck silica gel 60 using 1:4 hexane-diethyl ether as eluant to give 2.56 g (48%) of title amide.

TLC: silica gel, 4% $CH_3OH$ in $CH_2Cl_2$, $R_f$ 0.34, $Ce(SO_4)_2$.

$^{13}C$ NMR (67.5 MHz, $CDCl_3$) δ: 173.1, 157.6, 139.2, 133.1, 131.4, 131.4, 129.4, 129.4, 80.8, 63.3, 58.0, 41.7, 35.7, 34.7, 28.7, 28.7, 28.7

B. [1S-[1α,2α(Z),3α(R*),4α]]-6-[3-[[[2-[[2-(4-Chlorophenyl)ethyl]amino]-1-(hydroxymethyl)-2-oxoethyl]amino]-carbonyl]-7-oxabicyclo[2.2.1]hept-2-yl]-4-hexenoic acid, methyl ester

To a stirred solution of Part A amide (1.28 g, 3.73 mmol) in 20 mL of dry $CH_2Cl_2$ under argon at 0° C was added 5 mL of TFA. The mixture was stirred at 0° C for 2 hours and diluted with 50 mL of toluene. This mixture was concentrated in vacuo. To a stirred solution of this amine-TFA salt, 1-hydroxybenzotriazole hydrate (0.50 g, 3.73 mmol), and 5 mL of triethylamine in 20 mL of DMF was added a solution of Example 1, Part D acid (1.00 g, 3.73 mmol) in 10 mL of DMF. To this mixture was then added ethyl-3(3-dimethylamino)propyl carbodiimide hydrochloride salt (0.55 g, 3.73 mmol). The reaction mixture was stirred at room temperature for 17 hours and concentrated in vacuo. The mixture was diluted with 400 mL of ethyl acetate and washed with 1N HCl solution (3 x 40 mL), 0.2N NaOH solution (2 x 30 mL), saturated $NaHCO_3$ solution (1 x 30 mL) and brine (1 x 100 mL). The organic layer was dried ($MgSO_4$), filtered, and concentrated in vacuo. Purification was effected by flash chromatography on 60 g of Merck silica gel 60 using 2% $CH_3OH$ in $CH_2Cl_2$ as eluant to give 1.00 g (54%) of title alcohol.

TLC: silica gel, 4% $CH_3OH$ in $CH_2Cl_2$, $R_f$ 0.30, $Ce(SO_4)_2$.

$^{13}C$ NMR (67.5 MHz, $CDCl_3$) δ: 173.5, 172.9, 170.6, 137.2, 132.1, 130.0, 130.0, 129.3, 129.1, 128.5, 128.5, 79.0, 78.9, 62.7, 54.1, 53.9, 51.4, 48.0, 40.8, 34.8, 33.7, 29.5, 28.7, 27.3, 22.7

C. [1S-[1α,2α(2),3α(R*),4α]]-6-[3-[4-[[[2-(4-Chlorophenyl)ethyl]amino]carbonyl]-4,5-dihydro-2-oxazolyl]-7-oxabicyclo[2.2.1] hept-2-yl]-4-hexenoic acid, methyl ester

To a stirred solution of Part B alcohol (890 mg, 1.80 mmol) and diisopropylethyl amine (0.94 mL, 5.40 mmol) in 30 mL of $CH_2Cl_2$ under argon at 0° C was added methanesulfonyl chloride (0.14 mL, 1.80 mmol). This mixture was stirred at room temperature for 1 hour and concentrated in vacuo. The crude mesylate was dissolved in 30 mL of acetone and combined with 0.77 g of $K_2CO_3$. This mixture was heated to reflux for 4 hours, cooled to room temperature, and diluted with 100 mL of acetone. The precipitate was filtered off and rinsed with acetone (3 x 50 mL). The filtrate was concentrated in vacuo and chromatographed on 60 g of Merck silica gel 60 using 2% $CH_3OH$ in $CH_2Cl_2$ as eluant to give 480 mg (54%) of title oxazoline.

TLC: silica gel, 4% $CH_3OH$ in $CH_2Cl_2$, $R_f$ 0.42, $Ce(SO_4)_2$.

$^{13}C$ NMR (67.5 MHz, $CDCl_3$) δ: 171.6, 169.4, 169.3, 137.0, 132.3, 130.0, 130.0, 129.5, 129.0, 128.6, 128.6, 79.1, 79.0, 69.6, 68.3, 51.5, 48.2, 46.3, 40.0, 34.8, 33.8, 29.6, 28.9, 27.2, 22.8

D. [1S-[1α,2α(Z),3α,4α]]-6-[3-[4-[[[2-(4-Chlorophenyl)ethyl]amino]carbonyl]-2-oxazolyl]-7-oxabicyclo[2.2.1]-hept-2-yl]-4-hexenoic acid, methyl ester

To a stirred solution of Part C oxazoline (480 mg, 1.01 mmol) in 20 mL of $CH_2Cl_2$ was added 480 mg of

$NiO_2$. The reaction mixture was stirred at room temperature for 1 hour at which time another 480 mg of $NiO_2$ was added. The mixture was stirred at room temperature for 3 hours and 480 mg of $NiO_2$ was added. This mixture was stirred for another 15.5 hours and one more portion of 480 mg of $NiO_2$ was added. The reaction mixture was stirred for 1.5 hours and diluted with 100 mL of ethyl acetate. To the resulting mixture was added 20 mL of 3M $NaHSO_3$ solution and 30 mL of 1M sodium citrate solution. The organic layer was separated and the aqueous layer was extracted with ethyl acetate (2 x 100 mL). The combined organic extracts were washed with brine (1 x 50 mL), dried ($MgSO_4$), filtered, and concentrated in vacuo. This was chromatographed on 45 g of Merck silica gel 60 using 2% $CH_3OH$ in $CH_2Cl_2$ as eluant to give 200 mg (42%) of title ester.
TLC: silica gel, 4% $CH_3OH$ in $CH_2Cl_2$, $R_f$ 0.60, $Ce(SO_4)_2$.
$^{13}C$ NMR (67.5 MHz, $CDCl_3$) δ: 173.2, 163.9, 160.6, 140.5, 137.2, 135.8, 132.2, 130.0, 130.0, 129.4, 128.6, 128.6, 128.5, 79.5, 79.3, 51.4, 49.6, 46.6, 40.1 35.2, 33.7, 29.7, 28.9, 27.8, 22.7

E. [1S-[1α,2α(Z),3α,4α]]-6-[3-[4-[[[2-(4-Chlorophenyl)ethyl]amino]carbonyl]-2-oxazolyl]-7-oxabicyclo[2.2.1]-hept-2-yl]-4-hexenoic acid

To a stirred solution of Part D ester (190 mg, 0.40 mmol) and 4 mL of water in 30 mL of THF was added 4 mL of 1N LiOH solution. This mixture was sparged with argon for 10 minutes and stirred at room temperature for 5 hours. The mixture was acidified to pH 2 by the addition of 1N HCl solution and saturated with NaCl. The THF layer was separated and the aqueous layer was extracted with ethyl acetate (4 x 25 mL). The combined organic extracts was dried ($MgSO_4$), filtered and concentrated in vacuo. Purification was effected by flash chromatography on 20 g of Merck silica gel 60 using 4% $CH_3OH$ in $CH_2Cl_2$ as eluant to give 106.7 mg (59%) of title acid.
TLC: silica gel, 6% $CH_3OH$ in $CH_2Cl_2$, $R_f$ 0.32, $Ce(SO_4)_2$.
$^{13}C$ NMR (67.5 MHz, $CDCl_3$) δ: 176.9, 175.4, 164.0, 160.9, 140.9, 137.2, 130.1, 130.1, 129.4, 128.6, 128.6, 79.6, 79.5, 49.6, 46.6, 40.2, 35.1, 33.7, 29.7, 28.9, 27.9, 22.6

## Example 7

[1S-[1α,2α(Z),3α,4α]]-6-[3-[4-[[(4-Chlorophenyl)amino]carbonyl]-2-oxazolyl]-7-oxabicyclo[2.2.1]hept-2-yl]-4-hexenoic acid

A. [(1,1-Dimethylethoxy)carbonyl]-N-(4-chlorophenyl)-L-serinamide

To a stirred solution of 4-chloroaniline (2.00 g, 15.7 mmol), Boc-(L)-serine (3.22 g, 15.7 mmol), 1-hydroxybenzotriazole hydrate (2.12 g, 15.7 mmol) and diisopropylethyl amine (5.40 mL, 31.2 mmol) in 40 mL of DMF under argon was added ethyl-3(3-dimethylamino)propyl carbodiimide, hydrochloride salt (2.31 g, 15.7 mmol). This mixture was stirred at room temperature for 17 hours and concentrated in vacuo. The mixture was diluted with 400 mL ethyl acetate and washed with 1N aqueous HCl solution (3 x 70 mL), and saturated $NaHCO_3$ solution (2 x 70 mL). The organic layer was dried ($MgSO_4$), filtered and concentrated in vacuo. This was chromatographed on 80 g of Merck silica gel 60 using 1 L of each of 1:3 and 1:4 hexane-diethyl ether as eluants to give 1.02 g (21%) of title amide.
TLC: silica gel, 4% $CH_3OH$ in $CH_2Cl_2$, $R_f$ 0.34, $Ce(SO_4)_2$.

B. [1S-[1α,2α(Z),3α(R*),4α]]-6-[3-[[[2-[(4-Chlorophenyl)amino]-1-(hydroxymethyl)-2-oxoethyl]amino]carbonyl]-7-oxabicyclo[2.2.1]hept-2-yl]-4-hexenoic acid, methyl ester

To a stirred solution of Part A amide (1.02 g, 3.24 mmol) in 12 mL of dry $CH_2Cl_2$ under argon at 0° C was added 3 mL of TFA. The mixture was stirred at 0° C for 3 hours and diluted with 50 mL of toluene. This mixture was concentrated in vacuo. To a stirred solution of this amine-TFA salt, 1-hydroxybenzotriazole hydrate (0.50 g, 3.73 mmol), and 5 mL of triethylamine in 20 mL of DMF was added a solution of Example 1, Part D acid (1.00 g, 3.73 mmol) in 10 mL of DMF. To this mixture was then added 1-(3-dimethylaminopropyl)-3-ethylcarbodiimide. hydrochloride salt. The reaction mixture was stirred at room temperature for 17 hours and concentrated in vacuo. The mixture was diluted with 400 mL of ethyl acetate and washed with 1N HCl solution (3 x 30 mL), 0.2N NaOH solution (2 x 30 mL) and brine (1 x 100 mL). The organic layer was dried ($MgSO_4$), and concentrated in vacuo. Purification was effected by flash chromatography on 80 g of Merck silica gel 60 using 2% $CH_3OH$ in $CH_2Cl_2$ as eluant to give 380 mg (22%) of title alcohol.

TLC: silica gel, 4% $CH_3OH$ in $CH_2Cl_2$, $R_f$ 0.31, $Ce(SO_4)_2$.

C. [1S-[1α,2α(Z),3α(R*),4α]]-6-[3-[4-[[(4-Chlorophenyl)amino]carbonyl]-4,5-dihydro-2-oxazolyl]-7-oxabicyclo[2.2.1]hept-2-yl]-4-hexenoic acid, methyl ester

To a stirred solution of Part B alcohol (370 mg, 0.80 mmol) and diisopropylethyl amine (0.42 mL, 2.39 mmol) in 5 mL of $CHCl_3$ under argon at 0° C was added methanesulfonyl chloride (0.068 mL, 0.88 mmol). This mixture was stirred at room temperature for 30 minutes and concentrated in vacuo. The crude mesylate was dissolved in 10 mL of acetone and combined with 0.70 g of $K_2CO_3$. This mixture was heated almost to reflux for 3 hours, cooled to room temperature and diluted with 100 mL of acetone. The precipitate was filtered off and rinsed with acetone (3 x 30 mL). The filtrate was concentrated in vacuo and chromatographed on 35 g of Merck silica gel 60 using 2% $CH_3OH$ in $CH_2Cl_2$ as eluant to give 210 mg (59%) title oxazoline.

TLC: silica gel, 4% $CH_3OH$ in $CH_2Cl_2$, $R_f$ 0.68, $Ce(SO_4)_2$.

$^{13}C$ NMR (67.5 MHz, $CDCl_3$) δ: 173.2, 170.0, 169.6, 135.8, 129.5, 129.4, 128.9, 128.9, 128.5, 120.9, 120.9, 79.2, 79.2, 69.4, 68.7, 51.4, 48.3, 46.4, 33.7, 29.6, 28.8, 27.2, 22.8

D. [1S-[1α,2α(Z),3α,4α]]-6-[3-[4-[[(4-Chlorophenyl)amino]carbonyl]-2-oxazolyl]-7-oxabicyclo[2.2.1]hept-2-yl]-4-hexenoic acid, methyl ester

To a stirred solution of Part C oxazoline (200 mg, 0.45 mmol) in 3 mL of $CH_2Cl_2$ was added 200 mg of $NiO_2$. The reaction mixture was stirred at room temperature for 1.5 hours at which time another 200 mg of $NiO_2$ was added. The mixture was stirred at room temperature for 1.5 hours and 200 mg of $NiO_2$ was added. This mixture was stirred for another 1.5 hours and one more portion of 200 mg of $NiO_2$ was added. The reaction mixture was stirred for 2 hours and diluted with 100 mL of ethyl acetate. To the resulting mixture was added 5 mL of 3M $NaHSO_3$ solution and 30 mL of 1M sodium citrate solution. The organic layer was separated and the aqueous layer was extracted with ethyl acetate (2 x 100 mL). The combined organic extracts were dried ($MgSO_4$), filtered, and concentrated in vacuo. This was chromatographed on 15 g of Merck silica gel 60 using 2% $CH_3OH$ in $CH_2Cl_2$ as eluant to give 140 mg (70%) of title ester.

TLC: silica gel, 4% $CH_3OH$ in $CH_2Cl_2$, $R_f$ 0.76, $Ce(SO_4)_2$.

$^{13}C$ NMR (67.5 MHz, $CDCl_3$) δ: 173.2, 164.2, 158.4, 141.4, 136.0, 135.9, 129.5, 129.3, 128.9, 128.9, 128.4, 121.1, 121.1, 79.6, 79.4, 51.4, 49.6, 46.6, 33.7, 29.7, 28.9, 27.9, 22.8

E. [1S-[1α,2α(Z),3α,4α]]-6-[3-[4-[[(4-Chlorophenyl)amino]carbonyl]-2-oxazolyl]-7-oxabicyclo[2.2.1]hept-2-yl]-4-hexenoic acid

To a stirred solution of Part D ester (135 mg, 0.03 mmol) and 3 mL of water in 15 mL of THF was added 3 mL of 1N LiOH solution. This mixture was sparged with argon for 10 minutes and stirred at room temperature for 5 hours. The mixture was acidified to pH 2 by the addition of 1N HCl solution and saturated with NaCl. The THF layer was separated and the aqueous layer was extracted with ethyl acetate (4 x 15 mL). The combined organic extracts were dried ($MgSO_4$), filtered and concentrated in vacuo. Purification was effected by flash chromatography on 15 g of Merck silica gel 60 using 4% $CH_3OH$ in $CH_2Cl_2$ as eluant to give 79.2 mg (61%) of title acid.

TLC: silica gel, 6% $CH_3OH$ in $CH_2Cl_2$, $R_f$ 0.36, $Ce(SO_4)_2$.

$^{13}C$ NMR (67.5 MHz in, $CDCl_3$) δ: 179.0, 177.6, 164.2, 141.7, 136.0, 135.9, 129.4, 128.9, 128.5, 121.2, 121.2, 79.7, 79.5, 49.6, 46.6, 33.6, 32.3, 29.7, 28.9, 27.9, 22.6

Example 8

[1S-[1α,2α(Z),3α,4α]]-6-[3-[4-[[[4-(4-Chlorophenyl)butyl]amino]carbonyl]-2-oxazolyl]-7-oxabicyclo[2.2.1]hept-2-yl]-4-hexenoic acid

A. [1S-[1α,2α(Z),3α,4α]]-6-[3-[4-[[[4-(4-Chlorophenyl)butyl]amino]carbonyl]-2-oxazolyl]-7-oxabicyclo[2.2.1]-hept-2-yl]-4-hexenoic acid, methyl ester

0.21 g of impure Example 2, Part G compound (50% pure = 0.11 g, 0.33 mmol) was stirred in about 4 mL toluene, and 0.1 mL (1.14 mmol) of oxalyl chloride was added. One drop of dimethylformamide was added, and the mixture was stirred for 2 hours at room temperature after which the reaction was complete by TLC. The reaction mixture was concentrated in vacuo; the residue was reconcentrated twice from 2 mL of toluene to afford an orange oil namely [1S-[1α,2α(Z),3α,4α]]-6-(4(chlorocarbonyl)-2-oxazolyl)-7-oxabicyclo[2.2.1]hept-2-yl]-4-hexenoic acid, methyl ester. To this oil was added about 3 mL of $CHCl_3$ followed by 0.11 mL (0.08 g, 0.78 mmol) of triethylamine and 0.13 g (0.71 mmol) of 4-(4-chlorophenyl) butyl amine, and the mixture was stirred overnight at room temperature. The reaction mixture was diluted with ethyl acetate and water, the organic layer was separated, and the aqueous layer was extracted twice with 20 mL of ethyl acetate. The organic layers were combined, washed with brine, dried over $MgSO_4$, and concentrated in vacuo to afford 0.43 g of an orange oil. This oil was flash chromatographed (silica; 0% to 100% ethyl acetate in hexane gradient) to obtain 0.26 g of impure title methyl ester (50% pure = 0.13 g) as an oil. The product was obtained in 78% yield.

$^{13}C$ NMR (67.8 MHz, $CDCl_3$) δ: 172.8, 163.6, 160.3, 140.2, 140.2, 135.7, 131.0, 129.4, 129.1, 128.2, 128.0, 79.2, 79.1, 51.1, 49.4, 46.3, 38.4, 34.4, 33.5, 29.4, 28.6, 28.1, 27.6, 22.5

B. [1S-[1α,2α(Z),3α,4α]]-6-[3-[4-[[[4-(4-Chlorophenyl)butyl]amino]carbonyl]-2-oxazolyl]-7-oxabicyclo[2.2.1]-hept-2-yl]-4-hexenoic acid

0.26g of impure Part A ester (50% pure = 0.13 g, 0.26 mmol) was stirred in about 30 mL 1N NaOH and 2 mL of THF for 8 hours at room temperature. The reaction mixture was concentrated to remove THF and acidified to pH about 2 with concentrated HCl. Ethyl acetate was added and the organic layer was separated. The aqueous layer was extracted twice with about 20 mL ethyl acetate. The organic layers were combined, washed with saturated NaCl, dried over $MgSO_4$, and concentrated in vacuo to give 0.2 g of a clear oil. This was flash chromatographed (silica, 0.50% $CH_3OH$; 0.25% $CH_3CO_2H$; 99.25% ethyl acetate) to provide 0.10 g of pure product. The product was obtained in 79% yield.

$^{13}C$ NMR (67.8 MHz, $CDCl_3$) δ: 178.4, 164.4, 161.4, 141.0, 140.4, 135.6, 131.3, 129.6, 129.3, 128.5, 128.3, 79.5, 79.4, 49.6, 46.5, 38.8, 34.6, 33.6, 29.6, 28.9, 28.4, 27.8, 22.5

Example 9

[1S-[1α,2α(Z),3α,4α]]-6-[3[4-[[(4-Cyclohexylbutyl)amino]carbonyl]-2-thiazolyl]-7-oxabicyclo [2.2.1]hept-2-yl]-4-hexenoic acid

A. [1S-[1α,2α(Z),3α,4α]]-6-[3-(Aminocarbonyl)-7-oxabicyclo[2.2.1]hept-2-yl]-4-hexenoic acid, methyl ester

To a solution of Example 1, Part D acid (2.45 g, 9.13 mmol) in dry benzene (100 mL) was added, dropwise over a 10 minute period, oxalyl chloride (0.96 mL, 11 mmol). After stirring for 5 hours, the reaction was concentrated in vacuo, dissolved in dry THF (10 mL) and added dropwise over a 5 minute period to a 0 °C solution of concentrated ammonium hydroxide (3 mL) in THF (100 mL). The reaction was then concentrated in vacuo. The residual solid was partitioned between ethyl acetate (150 mL) and 0.25 M $K_2CO_3$ (25 mL). The aqueous layer was extracted with ethyl acetate (25 mL). The combined organic layers

were dried ($Na_2SO_4$) and concentrated in vacuo. The residue (2.2 g) was suspended in boiling ether (100 mL). Ethyl acetate (ca. 10 mL) was added to effect solution. The mixture was concentrated to ca. 50 mL on a steam bath, cooled to room temperature, seeded and chilled overnight. Pure title amide (1.19 g, 49%) was obtained by filtration. Additional title amide (145 mg) was obtained by concentration of the mothor liquors to 15 mL and allowing crystallization to occur.

B. [1S-[1α.2α(Z),3α,4α]]-6-[3-(Aminothiocarbonyl)-7-oxabicyclo[2.2.1]hept-2-yl]-4-hexenoic acid, methyl ester

To a 60° C solution of Part A amide (267 mg, 0.999 mmol) in dry toluene (10 mL) was added Lawesson's Aeagent (222 mg, 0.55 mmol). The reaction was stirred at 60° C for 30 minutes, diluted with diethyl ether (50 mL), and washed with half-saturated $NaHCO_3$ (2 x 5 mL). The organic layer was dried ($Na_2SO_4$) and concentrated in vacuo. The residue was passed through a short silica plug using 50% ethyl acetate/hexanes to yield a yellow solid (249 mg, 88%).

C. [1S-[1α,2α(Z),3α,4α]]-6-[3-(4-carboxy-2-thiazolyl)-7-oxabicyclo[2.2.1]hept-2-yl]-4-hexenoic acid, methyl ester

To a solution of Part B thioamide (400 mg, 1.41 mmol) and powdered anhydrous $K_2CO_3$ (390 mg, 2.82 mmol) in dry DMF (10 mL) was added, in several portions, bromopyruvic acid (contains 0.4 mol water/mol bromoacid, 295 mg, 1.69 mmol). The reaction was allowed to stir at room temperature for 30 minutes. After this time, an additional 29.5 mg portion of bromopyruvic acid was added. After an additional 1 hour, the solvent was removed in vacuo below 30° C. The residue was suspended/dissolved in methylene chloride (10 mL). Triethylamine (0.59 mL, 4.2 mmol) was added followed by the dropwise addition of methanesulfonyl chloride (0.33 mL, 4.2 mmol). After stirring for 5 minutes, the reaction was diluted with diethyl ether (40 mL). The organic layer was extracted with 0.5M $K_2CO_3$ (9 x 10 mL). The combined aqueous layers were brought to pH 1.5 with 6 N HCl and extracted with diethyl ether (6 x 25 mL). These combined organic layers were dried ($Na_2SO_4$) and concentrated in vacuo. The yield of the product acid was 212 mg (43%).

D. [1S-[1α,2α(Z),3α,4α]]-6-[3-[4-[[(4-Cyclohexylbutyl)amino]carbonyl]-2-thiazolyl]-7-oxabicyclo [2.2.1]hept-2yl]-4-hexenoic acid, methyl ester

To a solution of Part C acid (42.0 mg. 0.120 mmol) in dry DMF (1 mL) was added 1,1′-carbonyldiimidazole (20.3 mg, 0.125 mmol). The reaction was allowed to stir for 1 hour. A solution of (cyclohexylbutyl)amine hydrochloride (23.4 mg, 0.131 mmol) and triethylamine (0.020 mL, 0.14 mmol) in dry DMF (0.5 mL) was then added. The reaction was stirred for 1 hour, and concentrated to remove DMF. The residue was taken up in diethyl ether (20 mL) and 0.5 N HCl (5 mL). The organic layer was dried ($Na_2SO_4$) and concentrated in vacuo. The resultant amide (46.8 mg, 81%) was chromatographed (silica, 50% ethyl acetate/hexanes) to yield 37.1 mg (64%) of title ester as a colorless oil.

E. [1S-[1α,2α(Z),3α,4α]]-6-[3-[4-[[(4-Cyclohexylbutyl)amino]carbonyl]-2-thiazolyl]-7-oxabicyclo[2.2.1]hept-2-yl]-4-hexenoic acid

To a solution of Part D amide in methanol (1 mL) was added 2 N KOH (0.3 mL). The reaction was stirred for 2 hours. An additional 0.3 mL portion of KOH was added. After an additional 1 hour, the reaction was concentrated to remove methanol. The residue was dissolved in water (1 mL) and 1 N HCl was added to bring the pH to 2. The mixture was extracted with methylene chloride (3 x 5 mL). The combined organic layers were dried ($Na_2SO_4$) and concentrated in vacuo to yield an oil (34.7 mg, 96%).

## Example 10

[1S-[1α,2α(Z),3α,4α]]-6-[3-[5-[[(4-Cyclohexylbutyl)amino]carbonyl]-1H-imidazol-2-yl]-7-oxabicyclo[2.2.1]hept-2-yl]-4-hexenoic acid, methyl ester

A. 2-(((1,1-Dimethylethoxy)carbonyl)amino)-3-(((phenylmethoxy)carbonyl)amino)propanoic acid

N-α-Boc-asparagine (18.0 g, 77.5 mmol) was added to a solution of bis(trifluoroacetoxy)iodo sylbenzene (50.0 g, 116.3 mmol) in 1/1 DMF/$H_2O$ (620 mL). After 15 minutes, pyridine (12.5 mL, 155.1 mmol) was added. The dark yellow solution was stirred overnight (16 hours). The now pale yellow solution was concentrated in vacuo below 40° C. The residue was diluted with water (600 mL), washed with diethyl ether (6 X 400 mL), and then concentrated in vacuo. This crude product was dissolved in water (100 mL). To the solution was added 0.5M $Na_2CO_3$ to bring the pH to ca. 8. Then, THF (100 mL) was added. To this vigorously stirred mixture was added, dropwise benzylchloroformate (CBZ-Cl) (80% pure determined by NMR integration; 19.8 g, 93.0 mmol) dissolved in THF (50 mL). Additional $Na_2CO_3$ solution was added as needed to maintain a basic pH (as judged by pH 5-10 range pH strips). One hour after the addition was completed, the reaction was concentrated to remove THF. The residue was extracted with diethyl ether (3 X 200 mL). The aqueous layer was brought to pH 2 with concentrated $H_2SO_4$. The mixture was extracted with diethyl ether (4 X 200 mL). These latter extracts were dried ($Na_2SO_4$) and concentrated in vacuo. Chromatography (80% ethyl acetate/hexanes containing 1% acetic acid) yielded 4.23 g (16%) of a slightly impure title product.
$R_f$ (silica, ethyl acetate + 0.5% acetic acid) 0.27.

B. 2-(Trimethylsilyl)ethyl 2-(((1,1-dimethylethoxy)carbonyl)amino)-3-(((phenylmethoxy)carbonyl)amino)-propanoate

To a 0° C solution of the Part A acid (3.96 g, 11.7 mmol) in dry THF (50 mL) was added 1,1′-carbonyldiimidazole (2.08 g, 12.9 mmol). After 1 hour, 2-(trimethylsilyl)ethanol (3.4 mL) was added. The reaction was then brought to 70° C for 1 hour. After cooling, the reaction was concentrated and chromatographed (flash, silica, 50 mm dia, 25% ethyl acetate/hexanes) to yield 3.08 g (60%) of title ester in the form a transparent oil: $R_f$ (silica, 25% ethyl acetate/hexanes) 0.32.

C. 3-Amino-2-[[(1,1-dimethylethoxy)carbonyl]amino]propanoic acid, 2-(trimethylsilyl)ethyl ester

To a solution of the Part B Z-amine (2.89 g, 6.60 mmol) in 2-propanol/water (65 mL/6 mL) was added ammonium formate (2.08 g, 33.0 mmol) and then a slurry of Pd/C (10%, 0.5 g) in 2-propanol (4 ml). The mixture was stirred at room temperature for 1 hour. The reaction was filtered through Celite® and concentrated in vacuo. The residue was taken up in 70 mL of ethyl acetate and 30 mL of saturated NaCl containing 8 mL of 5N NaOH. The aqueous layer was further extracted with ethyl acetate (2 X 70 mL). The combined organic layers were dried ($Na_2SO_4$) and concentrated in vacuo to give title compound in the form of an oil (1.85 g, 92%): $R_f$ (silica, 10% methanol/chloroform) 0.75.

D. [1S-[1α,2α(Z),3α,4α]]-6-[3-[[[2-[[(1,1-Dimethylethoxy)carbonyl]amino]-3-oxo-3-[2-(trimethylsilyl)ethoxy]-propyl]amino]thioxomethyl]-7-oxabicyclo[2.2.1]hept-2-yl] -4-hexenoic acid, methyl ester

WSC (1.18 g, 6.15 mmol) was added to a solution of [1S-[1α,2α(Z),3α,4α]]-6-[3-(carboxy)-7-oxabicyclo-[2.2.1]hept-2-yl]-4-hexenoic acid, methyl ester (1.65 g, 6.15 mmol), Part C compound (1.85 g, 6.08 mmol), and HOBT (831 mg, 6.15 mmol) in methylene chloride (60 mL). The reaction was stirred for 16 hours, diluted with methylene chloride (300 mL), extracted (1 X 50 mL of 1N HCl; 1 X 50 mL of saturated $NaHCO_3$; 1 X 50 mL of water), dried ($Na_2SO_4$) and concentrated in vacuo. Chromatography (flash, silica, 50 mm dia, 40% ethyl acetate/hexanes, 2L; 60% ethyl acetate, 1L) yielded an oil: 2.11 g (63%): $R_f$ (silica, 10% methanol/chloroform) 0.67.

The preceding amide (1.94 g, 3.50 mmol) was stirred at 65° C in benzene (40 mL) with Lawesson's Reagent (850 mg, 2.10 mmol) for 1.5 hours. The reaction was cooled and diluted with diethyl ether (250 mL). The mixture was washed (2 X 50 mL of 0.5N $Na_2CO_3$), dried ($Na_2SO_4$), concentrated in vacuo, and chromatographed (flash, silica, 50 mm dia, 25% ethyl acetate/hexanes) to yield 1.61 g (80%) of title compound in the form of an oil: $R_f$ (silica, 25% ethyl acetate/hexanes) 0.14.

E. [1S-[1α,2α(Z),3α,4α]]-6-[3-[1-[(1,1-Dimethylethoxy)carbonyl]-4,5-dihydro-5-[[2-(trimethylsilyl)ethoxy]-carbonyl-1H-imidazole-2-yl]-7-oxabicyclo[2.2.1]hept-2-yl]-4-hexenoic acid, methyl ester

Carbon tetrachloride (3.00 mL, 31.2 mmol) was added to a solution of Part D compound (1.61 g, 2.82 mmol), triphenylphosphine (2.22 g, 8.46 mmol), triethylamine (1.18 mL, 8.46 mmol) in dry acetonitrile (28 mL). The reaction was stirred for 4 hours. The reaction was diluted with diethyl ether (125 mL), saturated NaCl (125 mL) and water (5 mL). The aqueous phase was further extracted with ether (125 mL). The combined organic layers were dried ($Na_2SO_4$) and concentrated in vacuo. The residue was triturated sequentially with ether (20 mL, 10 mL and 7 mL), each time taking and concentrating the filtrate for the next cycle. Chromatography (flash, silica, 50 mm dia, 50% ethyl acetate/hexanes) yielded 1.09 g (72%) of title compound in the form of an oil: $R_f$ (silica, 50% ethyl acetate/hexanes) 0.29.

F. [1S-[1α,2(Z),3α,4α]]-6-[3-(5-Carboxy-4,5-dihydro-1H-imidazole-2-yl)-7-oxabicyclo[2.2.1]hept-2yl]-4-hexenoic acid, methyl ester, monohydrochloride

To a solution of Part E compound (1.09 g, 2.03 mmol) in methylene chloride (2 mL) was added trifluoroacetic acid (4 mL). The reaction was stirred at room temperature for 1 hour, diluted with toluene (40 mL) and was then concentrated in vacuo. The residue was taken up in ethyl acetate (60 mL) and was washed (2 x 5 mL) with saturated $NaHCO_3$. The organic layer was dried ($Na_2SO_4$) and concentrated in vacuo to yield [1S-(1α,2α(Z),3α,4α]]-6-[3-(5-carboxy-4,5-dihydro-1H-imidazol-2-yl)-7-oxabicyclo[2.2.1]hept-2-yl]-4-hexenoic acid, methyl ester (261 mg, 27%). The aqueous phase was brought to pH 2 and concentrated in vacuo. Extraction of the solid with chloroform (2 X 15 mL) followed by concentration in vacuo yielded title compound (553 mg, 73%).

G. [1S-[1α,2α(Z),3α,4α]]-6-[3-[5-[[(4-Cyclohexylbutyl)amino] carbonyl]-1H-imidazol-2-yl]-7-oxabicyclo[2.2.1]-hept-2-yl]-4-hexenoic acid, methyl ester

A mixture of Part F acid (516 mg, 1.38 mmol), (4-cyclohexylbutyl)amine hydrochloride (345 mg, 1.80 mmol), WSC (345 mg, 1.80 mmol), HOBT (243 mg, 1.80 mmol) and triethylamine (0.50 mL, 3.6 mmol) in methylene chloride (15 mL) was stirred for 24 hours. The reaction was diluted with methylene chloride (50 mL) and was washed (2 X 10 mL of saturated $NaHCO_3$), dried ($Na_2SO_4$) and concentrated in vacuo to yield a yellowish oil, namely [1S-[1α,2α(Z),3α,4α]]-6-[3-[5-[[(4-cyclohexylbutyl)amino]carbonyl]-4,5-dihydro-1H-imidazol-2-yl]-7-oxabicyclo[2.2.1]hept-2-yl]-4-hexenoic acid, methyl ester. This oil was dissolved in chloroform (20 mL). Active $MnO_2$ (Aldrich, 1.0 g) was added. The reaction was stirred for 24 hours. An additional 0.5 g portion of $MnO_2$ was added and stirring was continued for 3 days. Further $MnO_2$(0.5 g) was added. After 5 hours, the reaction was filtered through Celite®. The pad was rinsed with portions of chloroform. The combined filtrates were concentrated in vacuo. Chromatography (flash, silica, 25 mm dia, 2% methanol/chloroform) yielded 235 mg (36%) of title ester compound in the form of an oil.

## Example 11

[1S-[1α,2α(Z),3α,4α]]-6-[3-[4-[[(4-Cyclohexylbutyl)amino]carbonyl]-1H-imidazol-2-yl]-7-oxabicyclo[2.2.1]hept-2-yl]-4-hexenoic acid

To a solution of Example 10 ester in methanol (8 mL) was added 2N KOH (4 mL). The reaction was stirred at room temperature for 4 hours. The methanol was removed in vacuo. The residue was taken up in methylene chloride (25 mL) and brought to pH 2 with 1N HCl. After shaking, the aqueous layer was further extracted with methylene chloride (25 mL). The combined organic layers were dried ($Na_2SO_4$) and concentrated in vacuo. The residue was taken up in methylene chloride (10 mL) and swirled with ethereal HCl (4 mL) for 30 seconds. The mixture was concentrated in vacuo. Trituration with 10 mL of ethyl acetate (boiling to room temperature) yielded a white solid which was collected by filtration, washed with ethyl acetate (5 mL) and dried to yield title acid as a white solid: 126.4 mg (52%); mp 168-173° C.

$^1$H NMR (tetradeuteriomethanol/deuteriochloroform, 270 MHz): δ 8.05 (s, 1H), 5.23-5.36 (m, 2H), 4.73 (s,

1H), 4.41 (s, 1H), 3.74 (d, J = 8 Hz, 1H), 3.34-3.37 (m, 2H), 0.84-2.44 (m, 28H);
$^{13}C$ NMR (complete decoupling, 67.8 MHz, tetradeuteriomethanol/deuteriochloroform): δ 175.4, 157.0, 147.8, 130.1, 127.3, 120.7, 80.0, 79.9, 44.9, 39.8, 37.4, 36.9, 33.5, 33.1, 29.1, 28.7, 28.1, 26.3, 26.2, 24.1, 22.6.
IR (KBr): 3427 (m), 3232 (m), 3009 (m), 2923 (s), 2851 (m), 1730 (m), 1718 (m), 1651 (m), 1566 (m), 1448 (w), 1334 (w), 1189 (w) $cm^{-1}$.
LRMS (Cl, $NH_3$ DEP 50, pos. ion spectrum) m/z (rel. int.) 458 (2), 184 (7), 173 (14), 172 (6), 169 (9), 168 (13), 167 (5), 157 (10), 156 (100), 155 (25), 154 (35), 152 (6).

| Anal. Calc'd for $C_{26}H_{40}ClN_3O_4 \cdot 0.25\ H_2O$: | |
|---|---|
| | C, 63.63; H, 8.19; Cl, 7.11; N, 8.43 |
| Found: | C, 62.76; H, 8.30; Cl, 6.82; N, 8.37. |

## Example 12

[1S-[1α,2α(Z),3α,4α]]-N-(4-Cyclohexylbutyl)2-[2-[5-(1H-tetrazol-5-yl)-2-pentenyl]-7-oxabicyclo[2.2.1]hept-3-yl]-4-oxazolecarboxamide

A. [1S-[1α,2α(Z),3α,4α]]-2-[5-(1H-Tetrazol-5-yl)-2-pentenyl]-7-oxabicyclo[2.2.1]heptane-3-methanol

To a 0°C slurry of 1.0 mmol [4aR-(4aα,5β,8β,8aα)]-octahydro-5,8-epoxy-1H-2-benzopyran-3-ol, prepared as described in U.S. Patent No. 4,143,054, and 1.4 mmol 3-(tetrazol-5-yl)propyltriphenylphosphonium bromide in tetrahydrofuran is added dropwise a 1.8M solution of KOt-amylate in toluene (2.8 mmol). The mixture is allowed to warm to room temperature overnight. The reaction mixture is quenched by the addition of acetic acid. The reaction mixture is concentrated in vacuo and purified by silica gel chromatography using $CH_3OH/CH_2Cl_2$ mixture as eluants.

B. [1S-[1α,2α(Z),3α,4α]]-2-[5-(1-Methoxymethyl-1H-tetrazol-5-yl)-2-pentenyl]-7-oxabicyclo[2.2.1]heptane-3-methanol

A solution of 1.0 mmol of title A tetrazole in THF is cooled to 0°C and treated with 1.0 mmol of triethylamine and 1.0 mmol of bromomethylmethyl ether. The mixture is stirred for 4 hours at room temperature and then partitioned between saturated aqueous $NaHCO_3$ and ethyl acetate. The ethyl acetate layer is dried, filtered and concentrated in vacuo to afford title B tetrazole.

C. [1S-[1α,2α(5),3α,4α]]-N-(4-Cyclohexylbutyl)-2-[2-[5-(1-methoxymethyl-1H-tetrazol-5-yl)-2-pentenyl]-7-oxabicyclo[2.2.1]hept-3-yl]-4-oxazolecarboxamide

Following Example 1 except substituting Example 12, Part B tetrazole for Example 1, Part C ester, the title compound is obtained.

D. [1S-[1α,2α(Z),3α,4α]]-N-(4-Cyclohexylbutyl)-2-[2-[5-(1H-tetrazol-5-yl)-2-pentenyl]-7-oxabicyclo[2.2.1]hept-3-yl]-4-oxazolecarboxamide

To a solution 1.0 mmol of title C tetrazole in $CH_3OH$ (25 mL) is added 1 drop of concentrated HCl solution. The solution is heated to reflux for 2 hours. On cooling, the reaction mixture is concentrated in vacuo. The crude product is purified by reverse-phase HPLC using $CH_3CN$/0.02% aqueous $H_3PO_4$ mixtures as the mobile phase to afford title tetrazole.

Examples of additional compounds in accordance with the present invention which may be prepared

following the procedures outlined in the specification and working Examples include, but are not limited to the following:

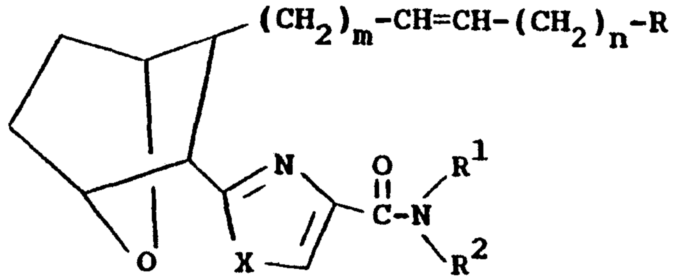

| Example No. | $(CH_2)_m$ m | $(CH_2)_n$ n | X | $R^1$ | $R^2$ | R |
|---|---|---|---|---|---|---|
| 13 | 1 | 2 | O | $C_6H_{13}$ | $CH_3$ | $CO_2H$ |
| 14 | 2 | 2 | O | $-(CH_2)_2-$ 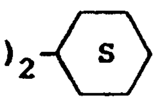 | $C_2H_5$ | $CO_2H$ |
| 15 | 3 | 1 | NH | 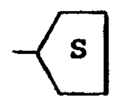 | $i-C_3H_7$ | $CONHSO_2CH_3$ |
| 16 | 1 | 2 | S | $-(CH_2)_2-$ 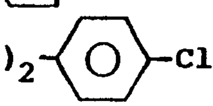 $-Cl$ | H | $CH_2$-5-tetrazolyl |
| 17 | 2 | 3 | O | $C_6H_5$ | $C_6H_5$ | $CO_2H$ |
| 18 | 1 | 2 | NH | $-CH_2C_6H_5$ | $CH_2C_6H_5$ | $CONHC_6H_5$ |
| 19 | 1 | 2 | O | $i-C_3H_7$ | H | $CONHSO_2C_6H_5$ |
| 20 | 1 | 3 | O | $-CH_2-$ 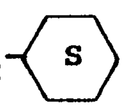 | $n-C_4H_9$ | $CONHSO_2CH_2C_6H_5$ |

| Example No. | $(CH_2)_m$ m | $(CH_2)_n$ n | X | $R^1$ | $R^2$ | R |
|---|---|---|---|---|---|---|
| 21 | 1 | 2 | NH | $-(CH_2)_3-$ 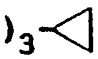 | H | $CONHCH_2C_6H_5$ |
| 22 | 2 | 2 | O | 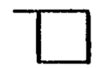 | $CH_2C_6H_5$ | $CO_2CH_3$ |
| 23 | 1 | 2 | S | $C_2H_5$ | H | $CO_2Li$ |
| 24 | 1 | 3 | NH | 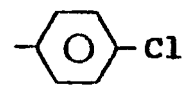 | $C_2H_5$ | $CO_2C_2H_5$ |
| 25 | 1 | 2 | O | $-(CH_2)_2C_6H_5$ | $CH_3$ | $CO_2H$ |
| 26 | 1 | 3 | O | $n-C_3H_7$ | $CH_2C_6H_5$ | $CH_2$-5-tetrazolyl |
| 27 | 1 | 2 | NH | $n-C_5H_{11}$ | H | $CO_2H$ |
| 28 | 2 | 3 | O | 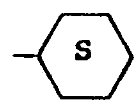 | $CH_3$ | $CONHCH_3$ |
| 29 | 1 | 2 | O | $-(CH_2)_6-$ | | $CONH_2$ |

Example 30

[1S-[1α,2α(Z),3α,4α]]-6-[3-[4-[[(6-Cyclohexylhexyl)amino]carbonyl]-2-oxazolyl]-7-oxabicyclo[2.2.1]-hept-2-yl]-4-hexenoic acid, methyl ester

A. Benzenepentanol, methanesulfonate ester

To a stirred solution of 5-phenyl-1-pentanol (5.0 g, 30.4 mmol) in 20 mL of methylene chloride at -70° C under argon, was added first $(C_2H_5)_3N$ (4.0 g, 39.5 mmol), then methanesulfonyl chloride (2.83 mL, 4.18 g, 36.5 mmol) dropwise. The reaction mixture was slowly warmed to room temperature. After stirring for 2 hours at room temperature, water was added, and the reaction mixture was extracted with methylene chloride (50 mL). The aqueous layer was extracted twice more with $CH_2Cl_2$ (25 mL). The organic layers were combined and washed with brine, dried over $MgSO_4$, and concentrated to obtain 7.30 g (100%) of title compound in the form of a yellow oil. $R_f$ 0.9 in 50% hexane-ethyl acetate (UV, $Ce(SO_4)_2$).
$^{13}C$ NMR ($CDCl_3$): δ 141.9, 128.2, 125.6, 69.9, 37.1, 35.5, 30.6, 28.8, 24.8.

B. Benzenehexanenitrile

To a stirred solution of Part A compound (7.30 g, 30.1 mmol) in 70 mL of $C_2H_5OH$ at room temperature was added a solution of KCN (9.81 g, 150.6 mmol) in 29 mL of water. This reaction mixture was stirred for 20 hours, then extracted with $CHCl_3$ three times (50 mL). The organic layers were combined and washed with water, then brine, and dried over $MgSO_4$ and concentrated in vacuo to obtain a yellow oil containing title compound and remaining Part A compound. This oil was purified by flash chromatography (95:5 hexane-ethyl acetate) to obtain 2.16 g (41%) of the desired title product as an oil.
$^{13}C$ NMR ($CDCl_3$): δ 141.8, 128.0, 125.5, 119.5, 35.4, 30.5, 27.9, 24.9, 16.7.

C. Cyclohexanehexanamine

To a stirred solution of Part B compound (2.16 g, 12.4 mmol) in 100 mL of $CH_3COOH$ was added $PtO_2$ (0.60 g). The mixture was stirred at room temperature under $H_2$ balloon at 1 atmosphere. After 20 hours the reaction mixture was filtered through a celite pad, and the pad was washed with ethanol twice. The filtrate was concentrated in vacuo to obtain a semi-solid. This was stirred in hexane, and title product in the form of a white fluffy solid (2.00 g, 95%) was obtained by filtration.

D. [1S-[1α,2α(Z),3α,4α]]-6-[3-[4-[[(6-Cyclohexylhexyl)amino]carbonyl]-2-oxazolyl]-7-oxabicyclo [2.2.1]hept-2-yl]-4-hexenoic acid, methyl ester

A sample of 1.61 g of Example 2, Part G oxazole acid (4.8 mmol) was dried by azeotroping with dry DMF and toluene twice (high vacuum). This material (which contained residual DMF) was dissolved in 30 mL toluene, and while stirring at room temperature under argon, 2.9 g oxalyl chloride (23 mmol) was added. Gas was evolved. A dark oil formed at the bottom of the reaction mixture. (The oil was the product of the reaction of DMF with oxalyl chloride.) After 5 minutes an additional 2.9 g oxalyl chloride was added. This mixture was stirred overnight. TLC indicated clean conversion to the acid chloride. The supernatant was pipetted off and transferred to an argon filled flask. Twice the oil was stirred with additional toluene and the supernatant transferred. The combined supernatants were evaporated with $CHCl_3$ to provide 1.54 g (91% yield) of the acid chloride, namely [1S-[1α,2α(Z),3α,4α]]-6-[3-[4-(chlorocarbonyl)-2-oxazolyl]-7-oxabicyclo [2.2.1]hept-2-yl]-4-hexenoic acid, methyl ester.

To a solution of (0.55 g, 3.0 mmol) Part C amine in 5 mL of chloroform at 0° C, was added (0.3 g, 3.0 mmol) triethylamine and (0.53 g, 1.5 mmol) the above acid chloride. The mixture was stirred at room temperature for 14 hours, then diluted with chloroform and water. The organic layer was separated, and the aqueous layer was extracted with 20 mL of chloroform twice. The organic layers were combined, washed with brine, dried over $MgSO_4$, and concentrated. Flash chromatography (0% to 75% EtOAc in hexane gradient) gave 0.49 g of product as a clear oil in 65% yield. $R_f$ 0.5 in 1:1 hexane-ethyl acetate (UV, $Ce(SO_4)_2$).
$^{13}C$ NMR (67.8 MHz, $CDCl_3$): δ 172.8, 163.5, 160.1, 140.1, 135.8, 129.1, 128.2, 79.2, 79.0, 51.0, 49.4, 46.3, 38.7, 37.2, 37.0, 33.4, 33.0, 29.4, 29.2, 28.6, 27.5, 26.6, 26.3, 26.1, 22.5.

Example 31

[1S-[1α,2α(Z),3α,4α]]-6-[3-[4-[[(6-Cyclohexylhexyl)amino]carbonyl]-2-oxazolyl]-7-oxabicyclo[2.2.1]hept-2-yl]-4-hexenoic acid

A solution of (0.49 g, 0.97 mmol) Example 30 ester in 8 of 1N NaOH and 8 mL of THF was stirred for 18 hours, then concentrated in vacuo to remove THF and acidified to pH 1.5 with 1N HCl. Ethyl acetate was added and the organic layer was separated. The aqueous layer was extracted twice with 20 mL ethyl acetate. The organic layers were combined, washed with brine, dried over $MgSO_4$, and concentrated in vacuo. A white solid was obtained. This was crystallized from hexane and chloroform to give 0.40 g (84%) of pure product as a white solid.

$R_f$ 0.36 in 0.1% $CH_3COOH$ in (4% methanol in ethyl acetate). Visualized by UV: $Ce(SO_4)_2$. $[\alpha]_D^{\circ}$ = +30.2, c 0.58 g/100 mL of $CH_3OH$, mp 82-83°C.

Example 32

[1S-[1α,2α(Z),3β,4α]]-6-[3-[4-[[(6-Cyclohexylhexyl)amino]carbonyl]-2-oxazolyl]-7-oxabicyclo[2.2.1]hept-2-yl]-4-hexenoic acid, methyl ester

To a stirred solution of 0.49 g (1.40 mmol) acid chloride prepared as described in Example 2, Part I plus an unknown quantity of Vilsmeier salt in 10 mL of chloroform, was added 0.28 g (2.80 mmol) of triethylamine and 0.38 g (2.1 mmol) of 6-cyclohexylhexylamine prepared in Example 30, Part C. The mixture was stirred at room tempera ture for 10 hours, then diluted with ethyl acetate and water. The organic layer was separated, and the aqueous layer was extracted with 20 mL of ethyl acetate twice. The organic layers were combined, washed with brine, dried over $MgSO_4$, and concentrated. Flash chromatography (0% to 100% ethyl acetate-hexane gradient) gave 0.17 g of title product as a clear oil in 25% yield. $R_f$=0.46 in 1:1 ethyl acetate:hexane.

Example 33

[1S-[1α,2α(Z),3β,4α]]-6-[3-[4-[[(6-Cyclohexylhexyl)amino]carbonyl]-2-oxazolyl]-7-oxabicyclo[2.2.1]hept-2-yl]-4-hexenoic acid

A solution of 0.17 g (0.33 mmol) of Example 32 ester in 5 mL of 1N NaOH and 2 mL of tetrahydrofuran was stirred at room temperature for 10 hours, then concentrated in vacuo to remove THF and acidified to pH 1.5 with concentrated HCl. Ethyl acetate was added and the organic layer was separated. The aqueous layer was extracted twice with 20 mL ethyl acetate. The organic layers were combined, washed with brine, dried over $MgSO_4$ and concentrated in vacuo. A clear oil was obtained. This was chromatographed in 1:1 EtOAc-hexane with 0.25% $CH_3COOH$ to give 0.13 g (78%) of title product as an oil (containing 2.5% of the cis isomer of Example 31A compound). $R_f$=0.36 in 4% $CH_3OH$, 0.1% $CH_3COOH$, 95.5% EtOAc (UV, Ce($SO_4$)$_2$). $[\alpha]_D^{\circ}$ = +55° in $CH_3OH$ at c=0.50 g/100 mL.

Example 34

[1α,2α(Z),3α,4α]-6-[3-[4-(1-Pyrrolidinylcarbonyl)-2-oxazolyl]-7-oxabicyclo[2.2.1]hept-2-yl]-4-hexenoic acid, methyl ester

To a stirred solution of 0.18 g (0.50 mmol) of the acid chloride prepared in Example 2, Part 1 in 3 mL of chloroform at 0° C, under argon was added 0.10 g (1.0 mmol) of triethylamine and 0.07 g (1.0 mmol) of pyrrolidine. The mixture was warmed to room temperature and stirred for 10 hours, then diluted with ethyl acetate and water. The organic layer was separated, and the aqueous layer was extracted with 20 mL of ethyl acetate twice. The organic layers were combined, washed with brine, dried over $MgSO_4$, and concentrated. Flash chromatography (50% to 100% ethyl acetate in hexane gradient then 2% methanol in ethyl acetate) gave 0.13 g of title product as a clear oil in 68% yield. $R_f = 0.6$ in 9:1 ethyl acetate-methanol (UV, $Ce(SO_4)_2$).
$^{13}C$ NMR (67.8 MHZ, $CDCl_3$): δ 172.9, 162.9, 160.0, 141.9, 136.9, 129.0, 128.5, 79.2, 78.9, 51.1, 49.4, 47.9, 46.5, 46.4, 33.5, 29.5, 28.7, 27.6, 26.1, 23.4, 22.5.

## Example 35

[1α,2α(Z),3α,4α]-6-[3-[4-(1-Pyrrolidinylcarbonyl)-2-oxazolyl]-7-oxabicyclo [2.2.1]hept-2-yl]-4-hexenoic acid

A solution of 0.13 g (0.33 mmol) of Example 34 compound in 10 mL of 1N NaOH and 2 mL of tetra hydrofuran was stirred for 10 hours at room temperature, then concentrated *in vacuo* to remove THF and acidified to pH 1.5 with 1N HCl. Ethyl acetate was added and the organic layer was separated. The aqueous layer was extracted twice with 20 mL ethyl acetate. The organic layers were combined, washed with brine, dried over $MgSO_4$ and concentrated *in vacuo*. A clear oil was obtained. This was chromatographed (EtOAc then 1% $CH_3OH$, 0.25% $CH_3COOH$, 98.75% EtOAc) to give 0.12 g (92%) of pure product as an oil. $R_f = 0.2$ in 1% $CH_3OH$, 0.5% $CH_3COOH$, 98.5% EtOAc. $[\alpha]^{\circ}_D = +35.7^{\circ}$ in $CH_3OH$ at c = 2.40 g/100 mL.

## Example 36

[1S-[1α,2α(Z),3α,4α]]-6-[3-[4-[(Propylamino)carbonyl]-2-oxazolyl]-7-oxabicyclo[2.2.1]hept-2-yl]-4-hexenoic acid, methyl ester

To a solution of (0.22 g, 3.7 mmol) propylamine in 5 mL of chloroform at 0° under argon, was added 0.162 g, (0.46 mmol) acid chloride prepared as described in Example 2, Part I. The mixture was warmed to room temperature and stirred for 14 hours, then diluted with chloroform and water. The organic layer was separated and the aqueous layer was extracted with 20 mL of chloroform twice. The organic layers were combined, washed with brine, dried over $MgSO_4$, and concentrated. Flash chromatography (0% to 75% EtOAc in hexane gradient) gave 0.15 g of title product as a clear oil in 85% yield. $R_f$ 0.29 in 4% methanol in ethyl acetate (UV, $Ce(SO_4)_2$).
$^{13}C$ NMR ($CDCl_3$): δ 173.0, 163.7, 160.4, 140.2, 136.0, 129.2, 128.4, 79.3, 79.2, 51.2, 49.5, 46.5, 40.5, 33.6, 29.6, 28.7, 27.7, 22.7, 22.6, 11.2.

## Example 37

[1S-[1α,2α(Z),3α,4α]]-6-[3-[4-[(Propylamino)carbonyl]-2-oxazolyl]-7-oxabicyclo[2.2.1]hept-2-yl]-4-hexenoic acid

A solution of 0.15 g (0.39 mmol) Example 36 ester in 8 mL of 1N NaOH and 8 mL of THF was stirred for 18 hours, then concentrated *in vacuo* to remove THF and acidified to pH 1.5 with 1N HCl. Ethyl acetate was added and the organic layer was separated. The aqueous layer was extracted twice with 20 mL ethyl acetate. The organic layers were combined, washed with brine, dried over $MgSO_4$ and concentrated *in vacuo*. A clear oil was obtained. This was crystallized from hexane and chloroform to give 0.14 g (100%) of pure product as a white solid. $R_f$ 0.41 in 0.1% $CH_3COOH$, 4% methanol, 95.9% ethyl acetate (UV, $Ce(SO_4)$-

2). mp 117-118° C, $[\alpha]^{\circ}_{D}$ = +42.12 at c 4.7 g/100 mL $CH_3OH$.

Example 38

[1S-[1α,2α(Z),3α,4α]]-6-[3-[4-[[(4-Butylphenyl)amino]carbonyl]-2-oxazolyl]-7-oxabicyclo[2.2.1]hept-2-yl]-4-hexenoic acid, methyl ester

To a solution of 0.189 g (1.27 mmol) 4-butylaniline in 5 mL of chloroform under argon at 0° C, was added 0.17 g (0.48 mmol) acid chloride prepared as described in Example 2, Part I and 0.145 g (1.44 mmol) triethylamine. The mixture was warmed to room temperature and stirred for 14 hours, then diluted with chloroform and water. The organic layer was separated, and the aqueous layer was extracted with 20 mL of chloroform twice. The organic layers were combined, washed with brine, dried over $MgSO_4$, and concentrated. Flash chromatography (0% to 75% EtOAc in hexane gradient) gave 0.16 g of title product as a clear oil in 69% yield. $R_f$ 0.87 in 4% methanol in ethyl acetate (UV, $Ce(SO_4)_2$).
$^{13}C$ NMR ($CDCl_3$): δ 173.1, 163.9, 158.2, 141.1, 139.0, 136.2, 129.4, 128.7, 128.4, 119.7, 79.4, 79.3, 51.3, 49.6, 46.6, 34.9, 33.6, 33.5, 29.7, 28.8, 27.8, 22.7, 22.1, 13.8.

Example 39

[1S-[1α,2α(Z),3α,4α]]-6-[3-[4-[[(4-Butylphenyl)amino]carbonyl]-2-oxazolyl]-7-oxabicyclo[2.2.1]hept-2-yl]-4-hexenoic acid

A solution of 0.16 g (0.34 mmol) Example 38 ester in 8 mL of 1N NaOH and 8 mL of THF was stirred for 18 hours, then concentrated in vacuo to remove THF and acidified to pH 1.5 with 1N HCl. Ethyl acetate was added and the organic layer was separated. The aqueous layer was extracted twice with 20 mL ethyl acetate. The organic layers were combined, washed with brine, dried over $MgSO_4$ and concentrated in vacuo. A clear oil was obtained. This was crystallized from hexane and chloroform to give 0.13 g (87%) of pure product as a yellow solid. $R_f$ 0.47 in 0.1% $CH_3COOH$, 4% methanol, 95.9% ethyl acetate (UV, $Ce(SO_4)_2$). mp 113-114° C. $[\alpha]^{\circ}_{D}$ = +6.44, 0.59 g/100 mL of $CH_3OH$.

Example 40

[1S-[1α,2α(Z),3α,4α]]-6-[3-[4-[(2,3-Dihydro-1H-indol-1-yl)carbonyl]-2-oxazolyl]-7-oxabicyclo[2.2.1]hept-2-yl]-4-hexenoic acid, methyl ester

To a solution of 0.18 g (1.50 mmol) indoline in 5 mL of chloroform, was added 0.18 g (1.8 mmol) triethylamine and 0.46 g (1.30 mmol) acid chloride prepared as described in Example 2, Part I. The mixture was stirred at room temperature for 14 hours, then diluted with chloroform and water. The organic layer was separated, and the aqueous layer was extracted with 20 mL of chloroform twice. The organic layers were combined, washed with brine, dried over $MgSO_4$, and concentrated. Flash chromatography (0% to 75% EtOAc in hexane gradient) gave 0.37 g of title product as a yellow oil in 65% yield. $R_f$ 0.8 in 98.9% ethyl acetate, 1% methanol, 0.1% TFA (UV, $Ce(SO_4)_2$).

Example 41

[1S-[1α,2α(Z),3α,4α]]-6-[3-[4-[(2,3-Dihydro-1H-indol-1-yl)carbonyl]-2-oxazolyl]-7-oxabicyclo[2.2.1]hept-2-yl]-4-hexenoic acid

A solution of 0.37 g (0.80 mmol) of Example 40 ester in 15 mL of 1N NaOH and 15 mL of methanol was stirred for 18 hours, and acidified to pH 2 with concentrated HCl. Ethyl acetate was added and the organic layer was separated. The aqueous layer was extracted twice with 20 mL ethyl acetate. The organic layers were combined, washed with brine, dried over $MgSO_4$, and concentrated in vacuo. A red oil was obtained. This was chromatographed (0.1% $CH_3COOH$ in (0% to 100% EtOAc in hexane gradient)) to give 0.25 g (75%) of pure title product as a yellow oil. $R_f$ 0.29 in 0.1% $CH_3COOH$ in 50% ethyl acetate in hexane (UV, $Ce(SO_4)_2$). $[\alpha]^{\circ}_{D}$ = +31.2 in $CH_3OH$ at c 0.48 g/100 mL.

## Example 42

[1S-[1α,2α(Z),3α,4α]]-6-[3-[4-[[(4-Cyclohexylbutyl)amino]carbonyl]-2-oxazolyl]-7-oxabicyclo]2.2.1]hept-2-yl]-N-(phenylsulfonyl)-4-hexenamide

To a stirred solution of 1-(3-dimethylaminopropyl)-3-ethylcarbodiimide, hydrochloride salt (102 mg, 0.50 mmol) and 4-dimethylaminopyridine (66.7 mg, 0.50 mmol) in 50 mL of DMF was added benzenesulfonamide (80 mg, 0.51 mmol) and $(C_2H_5)_3N$ (0.14 mL, 1.00 mmol) followed by Example 1 acid (229 mg, 0.50 mmol). The mixture was stirred at room temperature for 18 hours and concentrated in vacuo. The residue was partitioned between 25 mL of 1N HCl solution and EtOAc (4 x 40 mL). The combined EtOAc extracts were washed with 30 mL of brine, dried ($MgSO_4$), filtered and concentrated in vacuo. This was chromatographed on 30 g of Merck silica gel 60 using 2% $CH_3OH$ in $CH_2Cl_2$ as eluant to give 250 mg of title sulfonamide. This product was partitioned between 60 mL of EtOAc and $H_2O$ (1 x 20 mL). The organic layer was dried ($MgSO_4$), filtered and concentrated in vacuo to give 210 mg (70%) of title compound as a solid. m.p. 154-156° C, TLC: silica gel, 4% $CH_3OH/CH_2Cl_2$, $R_f$ 0.46, $Ce(SO_4)_2$.

$^{13}C$ NMR title compound ($CDCl_3$, 67.5 MHz): δ 171.1, 170.5, 163.9, 161.1, 140.7, 139.0, 135.9, 133.5, 128.7, 128.2, 79.7, 79.7, 49.5, 46.4, 39.2, 37.4, 37.0, 35.9, 33.3, 33.3, 29.7, 28.8, 27.9, 26.6, 26.3, 26.3, 24.1, 22.3.

## Example 43

[1S-[1α,2α(Z),3α,4α]]-6-[3-[4-[[(4-Cyclohexylbutyl)amino]carbonyl]-2-oxazolyl]-N-(methylsulfonyl)-7-oxabicyclo[2.2.1]hept-2-yl-4-hexenamide

To a stirred solution of 1-(3-dimethylaminopropyl)-3-ethylcarbodiimide, hydrochloride salt (102 mg, 0.50 mmol) and 4-dimethylaminopyridine (66.7 mg, 0.50 mmol) in 50 mL of DMF was added methanesulfonamide (47.6 mg, 0.50 mmol) and $(C_2H_5)_3N$ (0.14 mL, 1.00 mmol) followed by Example 1 acid (229 mg, 0.50 mmol). The mixture was stirred at room temperature for 18 hours and concentrated in vacuo. The residue was partitioned between 25 mL of 1N HCl solution and EtOAc (4 times with 40 mL). The combined EtOAc extracts were washed with 30 mL of brine, dried ($MgSO_4$), filtered and concentrated in vacuo. This was chromatographed on 30 g of Merck silica gel 60 using 2% $CH_3OH$ in $CH_2Cl_2$ as eluant to give 200 mg of title sulfonamide. This product was partitioned between 60 mL of EtOAc and $H_2O$ (1 x 20 mL). The organic layer was dried ($MgSO_4$), filtered and concentrated in vacuo to give 150 mg (56%) of title compound as a solid. m.p. 140-142° C, TLC: silica gel, 4% $CH_3OH/CH_2Cl_2$, $R_f$ 0.40, $Ce(SO_4)_2$.

$^{13}C$ NMR title compound ($CDCl_3$, 67.5 MHz): δ 171.8, 163.9, 161.0, 140.8, 135.9, 128.8, 128.7, 79.7, 79.7, 49.5, 46.4, 41.3, 39.2, 37.4, 37.0, 36.0, 33.3, 33.3, 29.7, 28.8, 28.0, 26.6, 26.3, 26.3, 24.1, 22.3.

## Example 44

[1S-[1α,2α(Z),3α,4α]]-7-[3-[4-[[(4-Cyclohexylbutyl)amino]carbonyl]-2-oxazolyl]-7-oxabicyclo-[2.2.1]hept-2-yl]-5-heptenoic acid, methyl ester

A. [1S-[1α,2α(Z),3α,4α]]-7-(3-Carboxy)-7-oxabicyclo[2.2.1]hept-2-yl]-5-heptenoic acid

To a stirred solution of [1S-[1α,2α(Z),3α,4α]]-7-(3-hydroxymethyl)-7-oxabicyclo [2.2.1]hept-2-yl]-5-heptenoic acid, methyl ester (1.60 g, 5.97 mmol) in 100 mL of acetone at 0 °C was added $MnSO_4$ treated Jones reagent (about 100 mg $MnSO_4$ dissolved in 100 mL of Jones reagent) until an orange-red color persisted. The mixture was stirred at 0 °C for 30 minutes and at room temperature for 1.5 hours. The mixture was quenched with isopropyl alcohol (IPA) and concentrated in vacuo. The residue was partitioned between 70 mL of 3M $NaHSO_3$ solution and EtOAc (4 x 100 mL). The combined EtOAc extracts were washed with $H_2O$ (1 x 70 mL) and brine (1 x 70 mL). The organic layer was dried ($MgSO_4$), filtered and concentrated in vacuo to give 1.57 g (93%) of title acid which was used for the next transformation without further purification. TLC: silica gel, 4% $CH_3OH/CH_2Cl_2$, $R_f$ 0.24, $Ce(SO_4)_2$.

$^{13}C$ NMR title acid ($CDCl_3$, 67.5 MHz): δ 176.7, 174.0, 130.5, 128.3, 78.4, 78.2, 51.8, 51.3, 47.8, 33.3, 29.0, 28.7, 27.2, 26.6, 24.6.

B. [2-[(4-Cyclohexylbutyl)amino)-1-(hydroxymethyl)-2-oxoethyl]carbamic acid, 1,1-dimethylethyl ester

To a stirred mixture of 4-cyclohexylbutylamine • HCl salt (19.5 g, 102 mmol), 1-hydroxybenzotriazole • $H_2O$ (16.5 g, 122 mmol) and Boc-L-serine (25.0 g, 122 mmol) in 400 mL of DMF under argon at 0 °C was added sequentially $(C_2H_5)_3N$ (42.5 mL, 305 mmol) and 1-(3-dimethylaminopropyl)-3-ethyl carbodiimide • HCl salt (23.4 g, 122 mmol). The mixture was stirred at 0 °C for 1 hour and at room temperature for 18 hours. The mixture was concentrated in vacuo and diluted with 800 mL of EtOAc. The resulting solution was washed with 1N HCl solution (3 times with 120 mL), 0.2N NaOH solution (2 times with 100 mL), saturated $NaHCO_3$ solution (1 time with 100 mL) and brine (1 time with 150 mL). The organic layer was dried ($MgSO_4$), filtered and concentrated in vacuo to give 39.9 g of title amide in a quantitative yield. TLC: silica gel, 4% $CH_3OH/CH_2Cl_2$, $R_f$ 0.38, $Ce(SO_4)_2$.

$^{13}C$ NMR title amide ($CDCL_3$, 67.5 MHz): δ 171.2, 156.2, 80.3, 62.8, 39.6, 37.4, 37.0, 33.3, 29.6, 28.2, 26.6, 26.3, 24.0.

C. [1S-[1α,2α(Z),3α,4α]]-7-[3-[[[2-[(4-Cyclohexylbutyl)amino]-1-(hydroxymethyl)-2-oxoethyl]amino]carbonyl]-7-oxabicyclo[2.2.1]hept-2-yl]-5-heptenoic acid, methyl ester

To a stirred solution of Part B amide (1.90 g, 5.56 mmol) in 20 mL of $CH_2Cl_2$ at 0 °C was added 8 mL of trifluoroacetic acid (TFA). This mixture was stirred at 0 °C for 3 hours. The mixture was diluted with 50 mL of toluene and concentrated in vacuo to give amine • TFA salt. To a stirred solution of this amine • TFA salt, Part A acid (1.57 g, 5.57 mmol) and 1-hydroxybenzotriazole • $H_2O$ (0.75 g, 5.57 mmol) in 60 mL of DMF at 0 °C under argon was added sequentially $(C_2H_5)_3N$ (3.88 mL, 27.8 mmol) and 1-(3-dimethylaminopropyl)-3-ethylcarbodiimide HCl • salt (1.07 g, 5.57 mmol). This mixture was then stirred at room temperature for 16 hours and concentrated in vacuo. The crude product was diluted with 400 mL of EtOAc and washed with 1N HCl solution (3 times with 70 mL), 0.2N NaOH solution (2 times with 50 mL) and saturated $NaHCO_3$ solution (1 time with 50 mL). The organic layer was dried ($MgSO_4$), filtered and concentrated in vacuo. This was chromatographed on 70 g of Merck silica gel 60 using 2% $CH_3OH$ in $CH_2Cl_2$ as eluant to give 1.74 g (42%) of title alcohol. TLC: silica gel, 2% $CH_3OH/CH_2Cl_2$, $R_f$ 0.18, $Ce(SO_4)_2$.

$^{13}C$ NMR title alcohol ($CDCl_3$, 67.5 MHz): δ 174.0, 173.2, 170.6, 130.6, 128.5, 79.2, 79.0, 62.7, 54.4, 53.5, 51.4, 48.0, 39.5, 37.4, 37.0, 33.3, 33.3, 33.3, 29.6, 28.6, 27.5, 26.6, 26.3, 26.3, 524.7, 24.1.

D. [1S-[1α,2α(Z),3α,4α]]-7-[3-[5-[[(4-Cyclohexylbutyl)amino]carbonyl]-4,5-dihydro-2-oxazol-2-yl]-7-oxabicyclo[2.2.1]hept-2-yl]-5-heptenoic acid, methyl ester

To a stirred solution of Part C alcohol (1.10 g, 2.17 mmol) and $(C_2H_5)_3N$ (0.61 mL, 4.35 mmol) in 10 mL of dry $CH_2Cl_2$ at 0 °C was added methanesulfonyl chloride (0.20 mL, 2.61 mmol). This mixture was stirred at 0 °C under argon atmosphere for 1.5 hours and diluted with 200 mL of $CH_2Cl_2$. This mixture was washed

with saturated $NaHCO_3$ solution (1 time with 20 mL) and brine (1 time with 20 mL). The organic layer was dried ($MgSO_4$), filtered and concentrated in vacuo. The crude product was dissolved in 20 mL of acetone and combined with 1.40 g of $K_2CO_3$. The mixture was refluxed for 5 hours and cooled to room temperature. The precipitate was filtered through a 2 inch pad of Celite® and rinsed with acetone (4 times with 50 mL). The filtrate was concentrated in vacuo. Purification was effected by flash chromatography on 36 g of Merck silica gel 60 using 2% $CH_3OH$ in $CH_2Cl_2$ as eluant to give 740 mg (70%) of title oxazoline. TLC: silica gel, 4% $CH_3OH/CH_2Cl_2$, $R_f$ 0.48, $Ce(SO_4)_2$. $^{13}C$ NMR title compound ($CDCl_3$, 67.5 MHz): δ 173.7, 171.2, 169.2, 130.5, 128.4, 79.0, 79.0, 69.6, 68.3, 51.3, 48.3, 46.3, 39.0, 37.4, 36.9, 33.3, 33.2, 33.2, 29.7, 29.6, 28.8, 27.2, 26.6, 26.5, 526.2, 26.2, 24.6, 24.0.

E. [1S-[1α,2α(Z),3α,4α]]-7-[3-[4-[[(4-Cyclohexylbutyl) amino]carbonyl]-2-oxazolyl]-7-oxabicyclo[2.2.1]hept-2-yl]-5-heptenoic acid, methyl ester

To a stirred solution of Part D oxazoline (730 mg, 1.50 mmol) in 15 mL of dry $CH_2Cl_2$ was added 1.10 g of $NiO_2$. This mixture was stirred at room temperature for one hour, then 0.74 g of $NiO_2$ was added. This mixture was stirred at room temperature for an additional hour and a second portion of 0.74 g of $NiO_2$ was added. This mixture was stirred at room temperature for one more hour and then diluted with 80 mL of EtOAc, 50 mL of 3M $NaHSO_3$ solution and 50 mL of 1M sodium citrate solution. The aqueous layer was separated and extracted with EtOAc (3 x 120 mL). The combined organic extracts were dried ($MgSO_4$), filtered and concentrated in vacuo. This was chromatographed on 30 g of Merck silica gel 60 using 2% $CH_3OH$ in $CH_2Cl_2$ as eluant to give 410 mg (56%) of title oxazole. TLC: silica gel, 2% $CH_3OH/CH_2Cl_2$, $R_f$ 0.31, $Ce(SO_4)_2$.

$^{13}C$ NMR title compound ($CDCl_3$, 67.5 MHz): δ 173.7, 163.8, 160.5, 140.4, 136.1, 130.5, 128.0, 79.5, 79.3, 51.3, 49.7, 46.6, 39.0, 37.4, 37.0, 33.3, 33.3, 29.8, 29.7, 28.9, 27.8, 26.6, 26.3, 24.6, 24.1.

## Example 45

[1S-[1α,2α(Z),3α,4α]]-7-[3-[4-[[(4-Cyclohexylbutyl)amino]carbonyl]-2-oxazolyl]-7-oxabicyclo[2.2.1]hept-2-yl]-5-heptenoic acid

To a stirred solution of Example 44 oxazole (410 mg, 0.84 mmol) in 20 mL of $CH_3OH$ was added 5 mL of 1N NaOH solution. This mixture was stirred at room temperature for 2.5 hours and concentrated in vacuo. This mixture was partitioned between 10 mL of 1N HCl solution saturated with NaCl and EtOAc (4 times with 20 mL). The EtOAc extracts were dried ($MgSO_4$), filtered and concentrated in vacuo. Purification was effected by flash chromatography on 20 g of Merck silica gel 60 using 150 mL of 2% $CH_3OH$ in $CH_2Cl_2$ as eluant, followed with the elution of 4% $CH_3OH$ in $CH_2Cl_2$ with 1% acetic acid to give 370 mg (93%) of pure title acid. m.p. 123-125 °C. TLC: silica gel, 6% $CH_3OH$ in $CH_2Cl_2$, $R_f$ 0.22, $Ce(SO_4)_2$.

$^{13}C$ NMR title compound ($CDCl_3$, 67.5 MHz): δ 177.8, 164.0, 160.9, 140.9, 135.8, 130.5, 128.1, 79.4, 79.4, 49.8, 46.7, 39.2, 37.5, 37.0, 33.3, 33.3, 29.8, 28.9, 27.8, 26.6, 26.5, 26.3, 26.3, 24.4, 24.2.

## Example 46

[1S-[1α,2α(Z),3α,4α]]-6-[3-[4-[[(7,7-Dimethyloctyl)amino]carbonyl]-2-oxazolyl]-7-oxabicyclo[2.2.1]hept-2-yl]-4-hexenoic acid, methyl ester

A. 3,3-Dimethylbutanal

A solution of oxalyl chloride (9.4 mL, 13.6 g, 107 mmol) under argon in $CH_2Cl_2$ (500 mL) was prepared in a 1000 mL flask and cooled to -60 °C. A solution of DMSO (15.4 mL, 18.5 g, 235 mmol) in 25 mL of $CH_2Cl_2$ was added dropwise over 10 minutes. The reaction mixture was stirred for 10 minutes, and 3,3-dimethyl-1-butanol (10 g, 98 mmol) was added slowly. Stirring was continued for an additional 20 minutes before $(C_2H_5)_3N$ (68.1 mL, 49.5 g, 489 mmol) was added, and then the reaction mixture was allowed to warm to room temperature. Water (50 mL) was then added. The mixture was separated, and the aqueous layer was extracted once with $CH_2Cl_2$ (30 mL). The organic layers were combined, washed sequentially with 1% aqueous HCl, water, saturated $NaHCO_3$, $H_2O$, and saturated NaCl, and dried over $MgSO_4$. The filtered solution was concentrated using a rotary evaporator to obtain 3.3 g (33%) of title compound in the form of a yellow oil. The low yield was probably due to product volatility.
$^{13}C$ NMR (67.8 MHz, $CDCl_3$): δ 203.3, 56.4, 31.0, 29.7

B. (Z)-7,7-Dimethyl-4-octenoic acid

To a stirred solution of 3-carboxypropyltriphenyl phosphonium bromide (13.72 g, 31.9 mmol) in 60 mL of dry THF under argon at -15° C was added dropwise 1.72 N K t-amylate/toluene (32 mL, 57.9 mmol) over 10 minutes. The orange colored mixture was stirred for 0.5 hours. To this, Part A aldehyde (2.00 g, 19.9 mmol) was added slowly as a solution in 5 mL of THF. The reaction mixture was stirred at -15° C for 1 hour and at room temperature for 20 hours. The reaction mixture was quenched with 12 mL of $CH_3COOH$ added dropwise and then concentrated in vacuo. The residue was partitioned between EtOAc (100 mL) and water (100 mL). The aqueous layer was extracted twice more with EtOAc (100 mL). The combined organic layers were washed sequentially with 1% aqueous HCl, $H_2O$, saturated $NaHCO_3$, $H_2O$, and saturated NaCl, dried over $MgSO_4$, and concentrated in vacuo. The residue was chromatographed eluting with 0.3% $CH_3COOH$ in (1% to 100% EtOAc in hexane gradient) to obtain 1.75 g (51%) of the desired title product.
$^{13}C$ NMR ($CDCl_3$): δ 179.8, 128.6, 128.4, 41.0, 34.1, 31.1, 29.2, 22.6.

C. 7,7-Dimethyloctanoic acid

To a stirred solution of Part B acid (1.2 g, 7.04 mmol) in 8 mL of $CH_3COOH$, was added 0.2 g of platinum oxide. This mixture was stirred for 14 hours under 1 atmosphere of $H_2$ (balloon). The reaction mixture was filtered through a Celite® pad. The filtrate was concentrated in vacuo. The residue was diluted with 50 mL toluene and concentrated again. This process was repeated once more, to obtain 1.2 g (100%) of the desired title product as oil.

D. 7,7-Dimethyloctanamide

To a stirred solution of Part C acid (1.21 g, 7.02 mmol) in 50mL of toluene was added 3 mL of oxalyl chloride. This reaction mixture was stirred for 1 hour at room temperature under argon and concentrated in vacuo. The residue was diluted with 20 mL of toluene and concentrated again. This was repeated to remove traces of oxalyl chloride. The residue was stirred at room temperature under argon in 5 mL of $CH_3OH$ and $(C_2H_5)_3N$ (1.17 mL, 8.43 mmol), and an excess of 9 M methanolic ammonia (2 mL) was added. After stirring for 16 hours, the reaction mixture was partitioned between 3 mL water and 20 mL EtOAc. The aqueous layer was extracted twice more with EtOAc (20 mL). The combined organic layers were washed with NaCl, dried over $MgSO_4$, filtered, and concentrated in vacuo to obtain a semi-solid. The semi-solid was crystallized by trituration with hexane to obtain 0.5 g (42%) of the desired title product as a solid.
$^{13}C$ NMR ($CDCl_3$): δ 176.3, 43.9, 35.9, 30.2, 30.1, 29.3, 25.5, 24.2.

E. 7,7-Dimethyloctanamine

To a solution of Part D amide (0.45 g, 2.62 mmol) in 50 mL of dry ether stirred under argon at 0° C was added lithium aluminum hydride (0.11 g, 2.88 mmol). Gas was evolved. The reaction mixture was stirred at room temperature for 4 days. While stirring vigorously, the reaction was cautiously quenched by addition of 0.02 mL of $H_2O$, 0.02 mL of 15% aqueous NaOH, and 0.072 mL of $H_2O$. A white precipitate formed. After stirring for 0.5 hours, the white precipitate was filtered, and the filtrate was concentrated in vacuo to obtain 0.4 g (89%) of title compound in the form of a yellow oil. This oil was crystallized by trituration with hexane and $CHCl_3$.

F. [1S-[1α,2α(Z),3α,4α]]-6-[3-[4-[[(7,7-Dimethyloctyl)amino]carbonyl]-2-oxazolyl]-7-oxabicyclo[2.2.1]hept-2-yl]-4-hexenoic acid, methyl ester

To a solution of acid chloride prepared in Example 2, Part I (0.46 g, 1.3 mmol) plus an unknown quantity of Vilsmeier salt in 5 mL of $CHCl_3$ under argon at room temperature, was added $(C_2H_5)_3N$ (0.25 mL, 0.18 g, 1.8 mmol) and Part E amine (0.24 g, 1.5 mmol). The reaction mixture was stirred at room temperature for 16 hours, then diluted with EtOAc and water. The organic layer was separated, and the aqueous layer was extracted with 20 mL of EtOAc twice. The organic layers were combined, washed with brine, dried over $MgSO_4$ and concentrated. Flash chromatography (0% to 100% EtOAc in hexane gradient) gave 0.20 g (32%) of the desired title product as an oil. $R_f$ is 0.8 in 1% TFA, 1% $CH_3OH$, 98% EtOAc.
$^{13}C$ NMR ($CDCl_3$): δ 172.8, 163.6, 160.2, 140.1, 135.9, 129.1, 128.3, 79.3, 79.1, 51.1, 49.4, 46.4, 43.9, 38.8, 33.5, 29.9, 29.5, 29.4, 29.1, 28.7, 27.6, 26.7, 24.1, 22.5.

## Example 47

[1S-[1α,2α(Z),3α,4α]]-6-[3-[4[[(7,7-Dimethyloctyl)amino]carbonyl]-2-oxazolyl]-7-oxabicyclo[2.2.1]hept-2-yl]-4-hexenoic acid

A solution of Example 46 ester (0.20 g, 0.38 mmol) in 10 mL of 1N NaOH and 10 mL of THF was prepared. The reaction mixture was stirred for 13 hours, then concentrated *in vacuo* to remove THF and acidified to pH 2 with 1N HCl. EtOAc was added and the organic layer was separated. The aqueous layer was extracted twice with 20 mL of EtOAc. The organic layers were combined, washed with brine, and concentrated *in vacuo* to obtain a clear oil. This oil was chromatographed (0.1% $CH_3COOH$ in (0% to 50% EtOAc in hexane gradient)) to obtain 0.12 g (66%) of title acid in the form of a white solid. mp 68-69 °C. $R_f$ is 0.18 in 1:1 hexane-EtOAc with 0.05% $CH_3COOH$.
$[\alpha]_D^{\circ}$ = +31.1 in $CH_3OH$ at c = 0.46 g/100 mL.
$^{13}C$ NMR ($CDCl_3$): δ 176.8, 163.9, 160.7, 140.8, 135.7, 129.4, 128.4, 79.5, 79.3, 49.6, 46.5, 44.0, 39.1, 33.6, 30.1, 29.6, 29.4, 29.3, 28.8, 27.8, 26.8, 24.3, 22.5.

## Example 48

[1S-[1α,2α(E),3α,4α]]-6-[3-[4-[[(4-Cyclohexylbutyl)amino]carbonyl]-2-oxazolyl]-7-oxabicyclo[2.2.1]hept-2-yl]-4-hexenoic acid

A. [1S-[1α,2α(Z),3α,4α]]-7-[3-[[Dimethyl(1,1-dimethylethyl)silyl]oxy]-7-oxabicyclo[2.2.1]hept-2-yl]-5-heptenoic acid, methyl ester

To a stirring solution of 20.57 g of [1S-[1α,2α(Z),3α,4α]]-7-[3-(hydroxymethyl)-7-oxabicyclo[2.2.1]hept-2-yl]-5-heptenoic acid, methyl ester (prepared as described in U.S. Patent No. 4,143,054) (76.8 mmol) and 5.74 g imidazole (84.4 mmol, 1.1 equiv) in 100 mL $CH_2Cl_2$ under argon at 0°, was added 12.05 g t-butyldimethylsilyl chloride (79.8 mmol, 1.04 equiv). A precipitate formed. The mixture was warmed to room temperature, and 10 minutes later it was diluted with diethyl ether ($Et_2O$), washed with water three times and brine once, dried over $Na_2SO_4$, and concentrated by rotoevaporation and then high vacuum. The residue, 30.31 g of nearly pure title silyl ether, was used without further purification. The yield of title ester was roughly quantitative.

| TLC (50% EtOAc in hexanes - anisaldehyde): | |
|---|---|
| starting material | 0.21 |
| title ester | 0.79 |

B. [1S-(1α,2α,3α,4α)]-3-[[[(1,1-Dimethylethyl)dimethylsilyl]oxy]methyl]-7-oxabicyclo[2.2.1]heptane-2-acetaldehyde

A solution of 30.31 g nearly pure Part A silyl ether (76.8 mmol) in 200 mL $CH_2Cl_2$ at -78° was treated with ozone until a blue color had persisted for 25 minutes. After purging excess $O_3$ with $O_2$, 48.0 g $(CH_3)_2S$ (770 mmol, 10 equiv) was added, and the mixture was warmed to room temperature. After 1 hour stirring at room temperature, solvent and excess $(CH_3)_2S$ were removed by rotoevaporation. $^1H$ NMR of the crude material revealed incomplete reduction of the ozonide. Therefore, after redissolving in $CH_2Cl_2$ at room temperature, 20.2 g triphenylphosphine (77 mmol, 1.0 equiv) was added. This mixture warmed due to exothermic reaction. After stirring overnight, most of the solvent was evaporated, and hexane was added to precipitate triphenylphosphine oxide and triphenylphosphine. The precipitate was filtered off, and the filtrate was concentrated before flash chromatography (5% to 15% EtOAc in hexanes gradient) allowed isolation of 16.31 g pure title aldehyde as an oil.

| TLC: 50% EtOAc in hexanes - anisaldehyde : | |
|---|---|
| Part A silyl ether | 0.89 |
| title aldehyde | 0.76 |
| methyl 5-oxopentanoate | 0.51 |

C. [1S-(1α,2α(E),3α,4α)]-4-[3-[[[(1,1-Dimethylethyl)dimethylsilyl]oxy]methyl]-7-oxabicyclo[2.2.1]hept-2-yl]-2-butenoic acid, methyl ester

A flask containing 3.3 g (37.9 mmol) of LiBr was placed under vacuum and heated with a heat gun to drive off any moisture. On cooling, the flask was flushed with Ar, and 20 mL of $CH_2Cl_2$ was added. To this stirred mixture was added a solution of 5.44 g (29.9 mmol) of trimethylphossphonoacetate in 30 mL of $CH_2Cl_2$ followed by 4.0 mL of $(C_2H_5)_3N$ (28.7 mmol). This mixture was stirred for 15 minutes after which a solution of 5.4 g (19.0 mmol max.) of crude Part B aldehyde in 35 mL of $CH_2Cl_2$ was added over 1 minute. This was accompanied by a slight exotherm and the formation of a sticky precipitate. This mixture was stirred vigorously at room temperature overnight. The reaction mixture was partitioned between 200 mL of hexane and 100 mL of 0.3 M HCl. The aqueous layer was extracted once with 100 mL of diethyl ether. The combined organic extracts were dried over $MgSO_4$, filtered, and concentrated in vacuo. TLC analysis showed that the reaction had not gone to completion (40-50%). The residue was then dissolved in 50 mL of $CH_2Cl_2$, and to it was added 6.3 g (18.8 mmol) of carbomethoxymethylenetriphenylphosphorane. This solution was stirred at room temperature for 22 hours and then concentrated in vacuo. The residue was triturated with diethyl ether and diluted with an equal volume of hexane. The mixture was refrigerated for 2 hours, and then the solid was removed by filtration. The filtrate was concentrated in vacuo. The crude product was purified by chromatography on 167 g silica gel using a 4:1 hexane-diethyl ether as eluent to provide 5.9 g (91%) of title ester.

TLC: silica gel, 2:1 hexane-ether, $R_f$ 0.6, vanillin.

$^{13}C$ NMR (67.8 MHz in $CDCl_3$): δ 148.8, 121.8, 79.9, 78.8, 61.9, 51.4, 44.8, 30.7, 29.5, 29.4, 25.9, 18.2, -5.4.

D. [1S-(1α,2α(E),3α,4α)]-4-[3-[[[(1,1-Dimethylethyl)dimethylsilyl]oxy]methyl]-7-oxabicyclo[2.2.1]hept-2-yl]-2-buten-1-ol

A solution of 5.2 g (15.3 mmol) of Part C ester in 80 mL of THF was cooled to -78° C. To this stirred solution was added dropwise 50.0 mL of a 1.5 M DIBAL-H solution in toluene over a period of 20 minutes. The reaction was stirred at -78° C for 5 hours, and then the reaction was quenched by the addition of a solution of 10 mL of acetone in 10 mL of toluene. This was followed by the slow addition of 60 g of 10:9 (w/w) silica gel water. After several grams of the moist silica gel had been added, the cold bath was removed. The reaction mixture was diluted with 200 mL of ether. The pot temperature was monitored, and when it reached 10° C, the flask was immersed in an ice bath. This was stirred for 1 hour, and then the silica gel was removed by filtration. The filter cake was rinsed with two 100 mL portions of ether. The combined filtrates were dried over $MgSO_4$, filtered, and concentrated in vacuo to afford 5.05 g (>100%) of crude title alcohol.

TLC: silica gel, 2:1 hexane-ether, $R_f$ 0.1, vanillin.

$^{13}C$ NMR (67.8 MHz in $CDCl_3$): $\delta$ 131.6, 130.3, 79.8, 78.8, 63.3, 61.8, 49.3, 45.4, 30.5, 29.5, 29.3, 25.9, 18.2, -5.4.

E. [1S-(1α,2α(E),3α,4α)]-4-[3-[[[(1,1-Dimethylethyl)dimethylsilyl]oxy]methyl]-7-oxabicyclo[2.2.1]hept-2-yl]-2-butene-1-bromide

To a flask containing 0.83 g of triphenylphosphine (3.17 mmol, 1.0 equiv) dissolved in 10 mL toluene under argon at 0°, was added 0.51 g of $Br_2$ (3.17 mmol, 1.0 equiv) in a single portion. A yellowish precipitate and an orange gum formed. Scraping with a spatula converted the gum to more free flowing precipitate. The mixture was briefly warmed to room temperature, then recooled to 0°. To this was added dropwise a solution of 1.04 g of Part D alcohol (95% pure = 0.99 g, 3.17 mmol) and 0.28 g of pyridine (3.49 mmol, 1.1 equiv) in 5 mL toluene plus a 5 mL wash-in. The mixture was stirred at 0° for 30 minutes, then warmed to room temperature. Reaction was incomplete according to TLC. After 4 hours (no further progress by TLC), the mixture was filtered, and the filtrate was evaporated. Flash chromatography (3% to 50% EtOAc in hexanes gradient) allowed isolation of 880 mg of title bromide, an oil, and 190 mg of unreacted Part D alcohol. The yield of title bromide was 74%.

| TLC (25% EtOAc in hexanes - anisaldehyde): | |
|---|---|
| Part D alcohol | 0.21 |
| title bromide | 0.75 |

$^{13}C$ NMR (67.8 MHz in $CDCl_3$): $\delta$ 135.6, 127.5, 79.8, 78.8, 61.8, 49.4, 45.3, 33.1, 30.5, 29.5, 29.4, 25.9, 18.2, -5.3.

F. [1S-(1α,2α(E),3α,4α)]-6-[3-[[[(1,1-Dimethylethyl)dimethylsilyl]oxy]methyl]-7-oxabicyclo[2.2.1]hept-2-yl]-4-hexenoic acid, 1,1-dimethylethyl ester

To a solution of LDA in THF (2.70 mmol, 1.15 equiv, prepared by slowly adding 1.08 mL of 2.5 M $C_4H_9Li$ in hexanes solution (2.70 mmol, 1.15 equiv) to a solution of 303 mg diisopropyl amine (3.0 mmol, 1.28 equiv) in 4 mL dry THF stirring at 0° under argon and then stirring for 15 minutes) stirring under argon at -78° was added dropwise a solution of 348 mg of t-butyl acetate (t-BuOAc) (3.0 mmol, 1.28 equiv) in 3 mL dry THF over 15 minutes. After stirring 1 hour, a solution of 880 mg Part E bromide (2.35 mmol) in 3 mL dry THF was added dropwise with two 2 mL wash-ins. After 8 hours of stirring at -78°, TLC showing only partial reaction, the mixture was allowed to very slowly warm (approximately 8° per hour) to room temperature. TLC indicated further progress, but still incomplete consumption of bromide. Addition of 1 mL of saturated aqueous $NH_4Cl$ solution was followed by drying over $Na_2SO_4$ and evaporation. The residue was flash chromatographed (5% EtOAc in hexane) to obtain 550 mg of nearly pure title ester product (97% pure = 534 mg). The yield of title ester was 55%.

| TLC (5% EtOAc in hexanes - anisaldehyde): | |
|---|---|
| Part E bromide | 0.20 |
| title ester | 0.13 |

$^{13}C$ NMR (67.8 MHz in $CDCl_3$): δ 172.4, 130.6, 129.6, 80.0, 79.8, 78.8, 61.9, 49.4, 45.8, 35.4, 30.8, 29.6, 29.4, 28.1, 25.9, 18.2, -5.3.

G. [1S-(1α,2α(E),3α,4α)]-6-[3-(Hydroxymethyl)-7-oxabicyclo[2.2.1]hept-2-yl]-4-hexenoic acid, methyl ester

To a solution of 550 mg of nearly pure Part F ester (97% pure = 534 mg, 1.30 mmol) in 20 mL $CH_3OH$ stirring at room temperature under argon, was added 2 mL of a solution of dry HCl in $CH_3OH$ (prepared by adding 2 drops of acetyl chloride to 2 mL $CH_3OH$ at room temperature and then allowing to stand 1 minute). TLC indicated complete conversion to an intermediate after 1 hour. This intermediate was then very slowly converted to product. Addition of 10 mL more HCl in $CH_3OH$ solution hastened the reaction. After 14 days, 2 mL $(C_2H_5)_3N$ was added, and the mixture was evaporated. This gave 630 mg of crude title alcohol.

| TLC (50% EtOAc in hexanes - anisaldehyde): | |
|---|---|
| Part F ester | 0.94 |
| intermediate | 0.33 |
| title alcohol | 0.23 |

H. [1S-(1α,2α(E),3α,4α)]-6-[3-Carboxy-7-oxabicyclo[2.2.1]hept-2-yl]-4-hexenoic acid, methyl ester

To a solution of 630 mg of crude Part G alcohol in 20 mL acetone under argon at 0°, was added slowly 4 mL Jones' Reagent (2.6M in $Cr^{VI}$). The red color of the reagent persisted toward the end of the addition. The resulting precipitated mixture was allowed to warm to room temperature for 20 minutes before recooling and 2-propanol addition to quench excess reagent. Still at 0°, 3M aqueous $NaHSO_3$ solution was added with stirring until all salts dissolved. Brine was added, and extraction (3 times) with EtOAc followed. After drying the extracts over $Na_2SO_4$ and solvent evaporation, flash chromatography (silica, 25% to 50% (5% acetic acid in EtOAc) in hexane gradient) afforded, after azeotropic removal of acetic acid with toluene, 260 mg of title acid, obtained in 75% overall yield from Part F ester.

| TLC (50% (5% $CH_3COOH$ in EtOAc) in hexane - anisaldehyde): | |
|---|---|
| Part G alcohol | 0.32 |
| title acid | 0.36. |

$^{13}C$ NMR (67.8 MHz in $CDCl_3$): δ 177.1, 173.4, 130.4, 129.2, 78.2, 78.1, 51.9, 51.3, 47.4, 33.8, 32.4, 28.9, 28.8, 27.7.

I. [1S-[1α,2α(E),3α,4α]]-6-[3-[4-[[(4-Cyclohexylbutyl)amino]carbonyl]-2-oxazolyl]-7-oxabicyclo[2.2.1]hept-2-yl]-4-hexenoic acid

Following the procedure of Example 1, starting at Part E except substituting the Part H acid for the

Example 1, Part D acid, the title compound is obtained. mp 122-125°; TLC (50% [5% AcOH in EtOAc] in hexane; title acid $R_f$ 0.33;
$^{13}C$ NMR ($CDCl_3$, 67.8 MHz): δ 176.9, 163.9, 160.7, 140.8, 135.6, 130.0, 128.8, 79.4, 79.1, 49.1, 46.5, 39.1, 37.4, 36.9, 33.7, 33.2, 33.0, 29.7, 29.5, 28.8, 27.5, 26.5, 26.2, 24.0.

## Example 49

[1S-(1α,2α,3α,4α)]-3-[4-[[(4-(Cyclohexylbutyl)amino]carbonyl]-2-oxazolyl]-7-oxabicyclo[2.2.1]heptane-2-hexanoic acid

A solution of 130 mg of Example 1 acid in 10 mL of ethyl acetate and 1.0 mL of acetic acid was degassed via a vacuum-fill cycle with argon. To this solution was added 34 mg of 10% Pd/C and the atmosphere was exchanged for hydrogen by two vacuum-fill cycles. A slight positive pressure was maintained through use of a hydrogen balloon. The mixture was stirred at room temperature for 22.5 hours, diluted with $CH_2Cl_2$ and filtered through a polycarbonate filter to remove the catalyst. The filtrate was concentrated in vacuo. The residue was diluted with toluene and reconcentrated. Upon addition of ethyl acetate to the residue, a small amount of gel-like material did not dissolve. The solution was decanted off and concentrated in vacuo. The crude product was dissolved in minimal hot ethyl acetate and diluted with ca. three volumes of hexane. On cooling no solid appeared, however, after standing at 5°C overnight a white gel-like solid formed. This was removed by filtration and dried in vacuo. The resulting white powder was triturated in hexane, filtered and dried in vacuo to afford 61 mg of pure title acid; m.p. 97(softens), 122-3°C.

| Analysis Calc'd for $C_{26}H_{40}N_2O_5$: | |
|---|---|
| | C, 67.79; H, 8.75; N, 6.08 |
| Found: | C, 67.58; H, 8.79; N, 5.97 |

$^{13}C$ NMR ($CDCl_3$, 67.5 MHz): δ 164.2, 160.8, 140.7, 135.9, 79.5, 79.4, 49.8, 47.2, 39.2, 37.5, 37.1, 33.7, 33.4, 29.9, 29.7, 29.2, 29.0, 28.1, 26.7, 26.4, 24.5, 24.2.

## Example 50

[1S-[1α,2α(Z),3α,4α]]-6-[3-[4-[[(4-Cyclohexylbutyl)amino] carbonyl]-2-oxazolyl]-7-oxabicyclo[2.2.1]hept-2-yl]-4-hexenoic acid, methyl ester

A. [1S-[1α,2α(Z),3α(R*),4α]]-6-[3-[[[2-[(4-Cyclohexylbutyl)amino]-1-(hydroxymethyl)-2-oxoethyl]amino]-carbonyl]-7-oxabicyclo[2.2.1]hept-2-yl]-4-hexenoic acid, methyl ester

To a stirred mixture of Example 1, Part D acid (6.70 g, 25.2 mmol), 1-hydroxybenzotriazole monohydrate (3.40 g, 25.2 mmol) and TFA salt of Example 1, Part B amine (8.97 g, 25.2 mmol) in 100 mL of DMF under argon at 0°C was added sequentially $(C_2H_5)_3N$ (17.6 mL, 126 mmol) and 1-(3-dimethylaminopropyl)-3-ethylcarbodiimide hydrochloride salt (4.82 g, 25.2 mmol). The mixture was stirred at 0°C for 2 hours and at room temperature for 16 hours. The mixture was concentrated in vacuo and partitioned between 800 mL of EtOAc and 1N HCl solution (2 X 100 mL), 0.2N NaOH solution (2 X 100 mL), saturated $NaHCO_3$ solution (1 X 100 mL). The organic layer was dried ($MgSO_4$), filtered and concentrated in vacuo. This was chromatographed on 180 g of Merck silica gel 60 using 2% $CH_3OH/CH_2Cl_2$ as eluant to give 4.75 g (39%) of title amide.
TLC: silica gel, 50% (5% AcOH in EtOAc) in hexane, $R_f$ 0.22, anisaldehyde.

B. [1S-[1α,2α(Z),3α(R*),4α]]-6-[3-[4-[[(4-Cyclohexylbutyl)amino]carbonyl]-4,5-dihydro-2-oxazolyl]-7-oxabicyclo [2.2.1]hept-2-yl]-4-hexenoic acid, methyl ester

To a stirred mixture of Part A amide (4.69 g, 9.53 mmol) in 60 mL of dry $CH_2Cl_2$ under argon at 0° C was added sequentially $(C_2H_5)_3N$ (2.66 mL, 19.1 mmol) and mesyl chloride (0.82 mL, 10.5 mmol). The mixture was stirred at 0° C for 1 hour and diluted with 100 mL of $CH_2Cl_2$ and washed with saturated $NaHCO_3$ solution (1 X 30 mL) and brine (1 X 30 mL). The organic layer was dried ($MgSO_4$), filtered and concentrated in vacuo. The crude mesylate was dissolved in 60 mL of acetone and combined with $K_2CO_3$ - (5.0 g, 36.2 mmol). The mixture was refluxed for 4.5 hours and cooled to room temperature. The solid was filtered off and rinsed with acetone (4 X 40 mL). The filtrate was concentrated in vacuo and chromatographed on 120 g of Merck silica gel 60 using 2% $CH_3OH/CH_2Cl_2$ as eluant to give 3.93 g (86%) of title oxazoline.
TLC: silica gel, 20% acetone in toluene, $R_f$ 0.29, anisaldehyde.

C. [1S-[1α,2α(Z),3α,4α]]-6-[3-[4-[[(4-Cyclohexylbutyl)amino]carbonyl]-2-oxazolyl]-7-oxabicyclo [2.2.1]hept-2-yl]-4-hexenoic acid, methyl ester

To a stirred mixture of Part 9 oxazoline (3.90 g, 8.23 mmol) in 80 mL of dry $CH_2Cl_2$ under argon was added 6 g of $NiO_2$. The mixture was stirred at room temperature for 40 minutes at which time 4 g of $NiO_2$ was added. The mixture was stirred for another 70 minutes and again 2 g of $NiO_2$ was added. The mixture was stirred at room temperature for 2 hours and diluted with 120 mL of EtOAc, 60 mL of 3M $NaHSO_3$ solution and 60 mL of 1M sodium citrate solution. The aqueous layer was separated and extracted with EtOAc (4 X 150 mL). The combined EtOAc extracts were dried ($MgSO_4$), filtered and concentrated in vacuo. Purification was effected by flash chromatography on 120 g of Merck silica gel 60 using 2% $CH_3OH/CH_2Cl_2$ as eluant to give 1.85 g (48%) of title oxazole.
TLC: silica gel, EtOAc, $R_f$ 0.81, anisaldehyde.

## Claims

1. A compound having the formula I

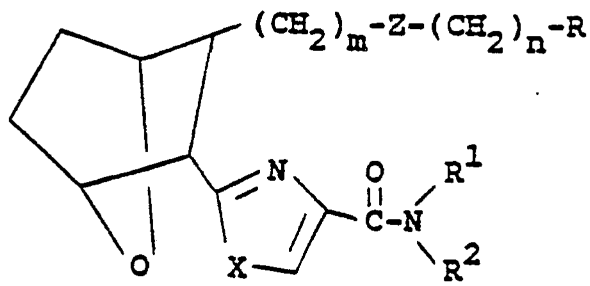

(I)

including all stereoisomers thereof, wherein
m is 1, 2 or 3;
n is 0, 1, 2, 3 or 4;
Z is $-(CH_2)_2-$ or -CH=CH-
with the proviso when Z is -CH=CH-, n is 1, 2, 3 or 4;
R is $CO_2H$, $CO_2$alkali metal, $CO_2$lower alkyl, $CH_2OH$, $CONHSO_2R^3$, $CONHR^{3a}$ or $-CH_2$-5-tetrazolyl;
X is O, S or NH;
$R^1$ is lower alkyl, lower alkenyl, lower alkynyl, aryl, aralkyl, cycloalkyl or cycloalkylalkyl;
$R^2$ is hydrogen, lower alkyl, aryl, or aralkyl;
or $R^1$ and $R^2$ together with the N to which they are linked form a 5- to 8- membered ring;
$R^3$ is lower alkyl, aryl or aralkyl; and
$R^{3a}$ is hydrogen, lower alkyl, aryl or aralkyl.

2. The compound according to Claim 1 having the formula

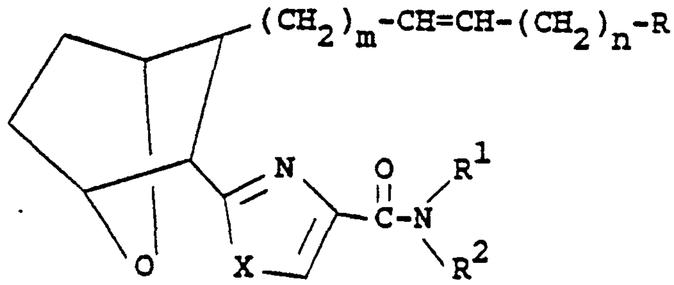

3. The compound according to Claim 2 having the formula

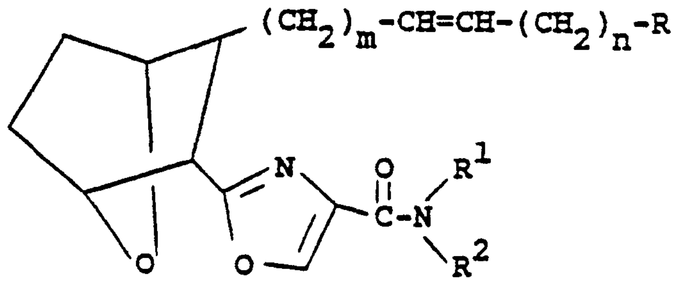

4. The compound according to Claim 2 where m = 1 and n = 2.

5. The compound according to Claim 1 having the formula

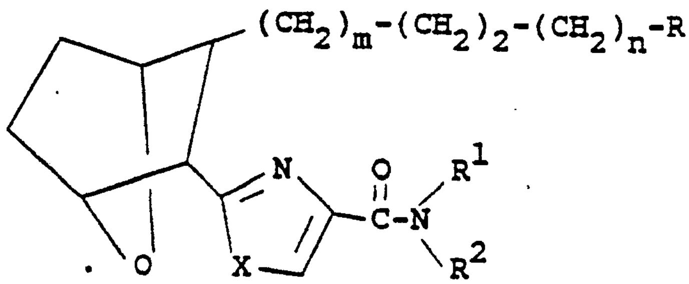

6. The compound according to Claim 5 where m = 1 and n = 2.

7. The compound according to Claim 5 having the formula

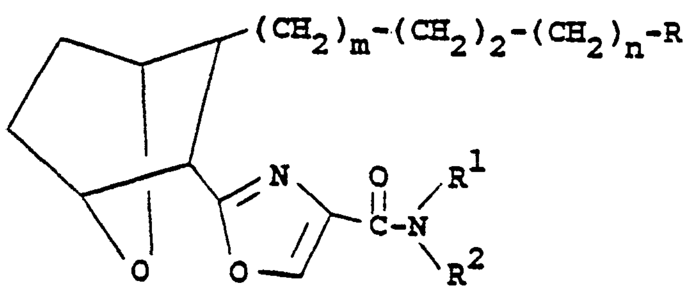

8. The compound according to Claim 1 having the formula

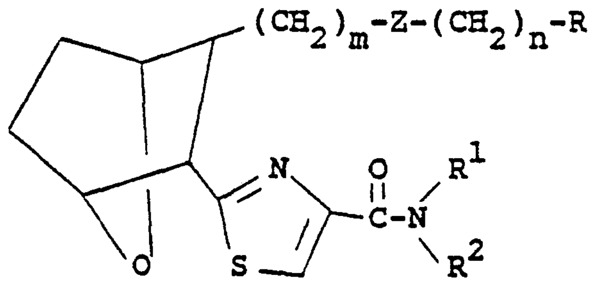

9. The compound according to Claim 1 having the formula

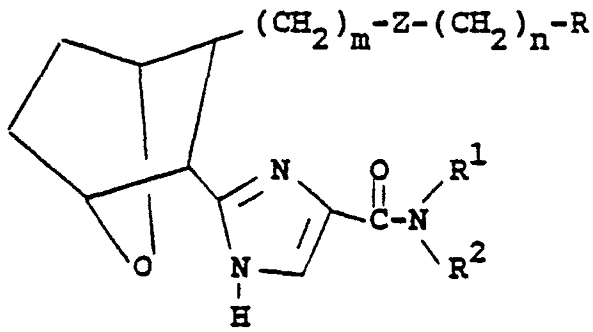

10. The compound according to Claim 1 wherein R is $CO_2H$, $CONHSO_2R^3$ or $-CH_2$-5-tetrazolyl.

11. The compound according to Claim 1 having the formula

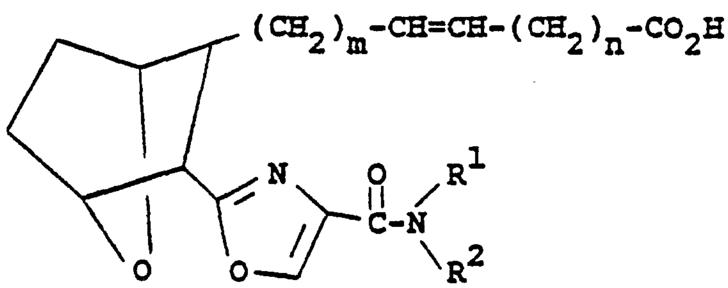

12. The compound according to Claim 1 having the name [1S-[1α,2α(Z),3α,4α]]-6-[3-[4-[[(4-cyclohexylbutyl)amino]carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[[(4-cyclohexylbutyl)amino]carbonyl]-2-thiazolyl]-7-oxabicyclo[2.2.1]hept-2-yl]-4-hexenoic acid, or esters or salts thereof; [1S[1α,2α(Z),3α,4α]]-6-[3-[4-[[(4-cyclohexylbutyl)methylamino]-carbonyl]-2-oxazolyl]-7-oxabicyclo[2.2.1]hept-2-yl]-4-hexenoic acid, or esters or salts thereof; [1S-[1α,2α(Z)-,3α,4α]]-6-[3-[4-[(1-pyrrolidinyl)carbonyl]-2-oxazolyl]-7-oxabicyclo[2.2.1] hept-2-yl]-4-hexenoic acid, or esters or salts thereof; [1S-[1α,2α(Z),3α,4α]]-6-[3-[4-[(cyclohexylamino)carbonyl]-2-oxazolyl]-7-oxabicyclo[2.2.1]-hept-2-yl]-4-hexenoic acid or esters or salts thereof; [1S-[1α,2α(Z),3α,4α]]-6-[3-[4-[[(2-cyclohexylethyl)-amino]carbonyl]-2-oxazolyl]-7-oxabicyclo[2.2.1]hept-2-yl]-4-hexenoic aicd, or esters or salts thereof; [1S-[1α,2α(Z),3α,4α]]-6-[3-[4-[[[2-(4-chlorophenyl)ethyl]amino]carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[[(4-chlorophenyl)amino]carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[[[4-(4-chlorophenyl)butyl]amino]-carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4a-[[(6-cyclohexylhexyl)amino]carbonyl]-2-oxazolyl]-7-oxabicyclo[2.2.1]hept-2-yl]-4-hexenoic acid, or esters, or salts thereof; [1S-[1α,2α(Z),3β,4α]]-6-[3- [4-[[(6-cyclohexylhexyl)amino]carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[(propylamino)carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[[(4-butylphenyl)amino]carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[(2,3-dihydro-1H-indol-1-yl )carbonyl]-2-oxazolyl]-7-oxabicyclo[2.2.1]hept-2-yl]-4-hexenoic acid, or esters

or salts thereof; [1S-[1α,2α(Z),3α,4α]]-6-[3-[4-[[(4-cyclohexylbutyl)amino]carbonyl]-2-oxazolyl]-7-oxabicyclo-[2.2.1]hept-2-yl]-N-(phenylsulfonyl)-4-hexenamide; [1S-[1α,2α(Z),3α,4α]]-6-[3-[4-[[(4-cyclohexylbutyl)amino]-carbonyl]-2-oxazolyl]-N-(methylsulfonyl)-7-oxabicyclo[2.2.1]hept-2-yl]-4-hexenamide; [1S-[1α,2α(Z),3α,4α]]-7-[3-[4-[[(4-cyclohexylbutyl)amino]carbonyl]-2-oxazolyl]-7-oxabicyclo[2.2.1]hept-2-yl]-5-heptenoic acid, or esters or salts thereof; [1S-[1α,2α(Z),3α,4α]]-6-[3-[4-[[(4-cyclohexylbutyl)amino]carbonyl]-1H-imidazol-2-yl]-7-oxabicyclo[2.2.1]hept-2-yl]-4-hexenoic acid or esters or salts thereof; [1S-[1α,2α,3α,4α]-6-[3-[4-[[(7,7-dimethyloctyl)amino]carbonyl]-2-oxazolyl]-7-oxabicyclo[2.2.1]hept-2-yl]-4-hexenoic acid, or esters or salts thereof; [1S-[1α,2α(E),3α,4α]]-6-[3-[4-[[(4-cyclohexylbutyl)amino]carbonyl]-2-oxazolyl]-7-oxabicyclo[2.2.1]-hept-2-yl]-4-hexenoic acid; [1S-1α,2α,3α,4α]]-3-[4-[[(4- cyclohexylbutyl)amino] carbonyl]-2-oxazolyl]-7-oxabicyclo[2.2.1]heptane-2-hexanoic acid or esters or salts thereof.

13. The compound according to Claim 1 having the name [1S-[1α,2α(Z),3α,4α]]-6-[3-[4-[[(4-cyclohexylbutyl)amino]carbonyl]-2-oxazolyl]-7-oxabicyclo[2.2.1]hept-2-yl]-4-hexenoic acid, or esters or salts thereof.

14. A compound of the general formula I according to any one of claims 1 to 13 for use in a method of inhibiting platelet aggregation and bronchoconstriction.

15. A compound of the general formula I according to any one of claims 1 to 13 for use in a method for improving post-ischemic myocardial function.

16. A compound of the general formula I according to any one of claims 1 to 13 for use in a method for treating toxemia during pregnancy.

17. A compound of the general formula I according to any one of claims 1 to 13 for use in a method for preventing or reducing venous thrombosis.

18. A compound of the general formula I according to any one of claims 1 to 13 for use in a method for preventing or reducing platelet loss during extracorporeal circulation.

19. A compound of the general formula I according to any one of claims 1 to 13 for use in a method for treating burn injuries and/or promoting wound healing.

20. A compound of the general formula I according to any one of claims 1 to 13 for use in a method for reducing post-ischemic myocardial injury together with an effective amount of a thrombolytic agent within 6 hours of a myocardial infarction.

21. A compound of the general formula I according to any one of claims 1 to 13 for use in a method according to claim 20 wherein said thrombolytic agent is t-PA, streptokinase, urokinase, prourokinase or anisoylated plasminogen-streptokinase activator complex.

22. A pharmaceutical composition comprising a compound of the general formula I according to any one of claims 1 to 13.

Claims for the following Contracting State: ES

1. A method for the production of a compound having the formula I

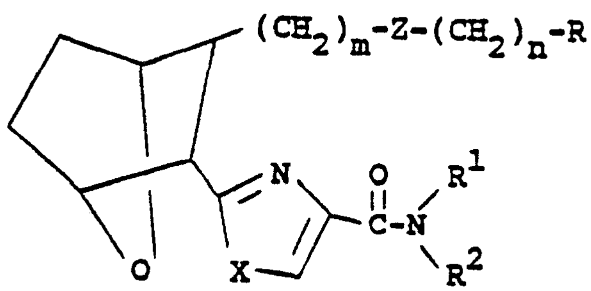

(I)

including all stereoisomers thereof, wherein
m is 1, 2 or 3;
n is 0, 1, 2, 3 or 4;
Z is $-(CH_2)_2-$ or -CH=CH-
with the proviso when Z is -CH=CH-, n is 1, 2, 3 or 4;
R is $CO_2H$, $CO_2$alkali metal, $CO_2$lower alkyl, $CH_2OH$, $CONHSO_2R^3$, $CONHR^{3a}$ or $-CH_2$-5-tetrazolyl;
X is O, S or NH;
$R^1$ is lower alkyl, lower alkenyl, lower alkynyl, aryl, aralkyl, cycloalkyl or cycloalkylalkyl;
$R^2$ is hydrogen, lower alkyl, aryl, or aralkyl;
or $R^1$ and $R^2$ together with the N to which they are linked form a 5- to 8- membered ring;
$R^3$ is lower alkyl, aryl or aralkyl; and

$R^{3a}$ is hydrogen, lower alkyl, aryl or aralkyl
characterized in that

a) for the production of compounds wherein Z is -CH=CH-, preferably in the cis-form and X is O, a hydroxymethyl compound of the general formula II

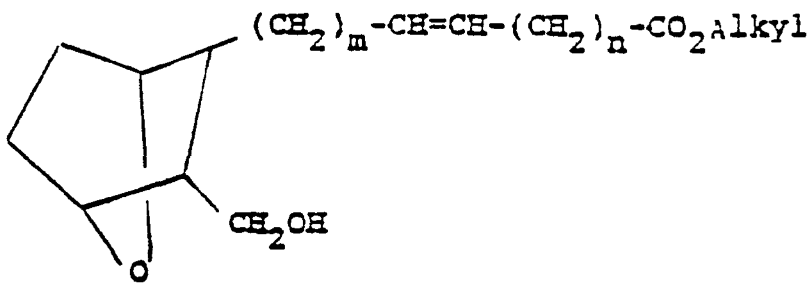

(II)

is reacted to the corresponding carboxylic acid of the general formula III

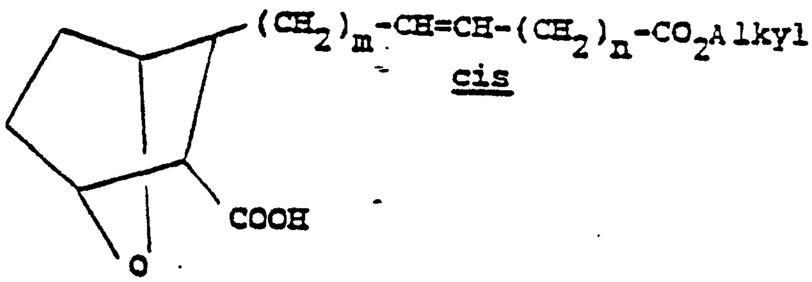

(III)

the acid of the general formula III is subjected to a carbodiimide coupling reaction with an amide of the general formula A

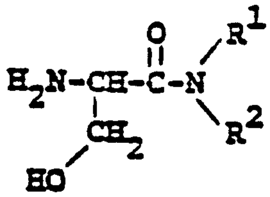

(A)

the obtained hydroxybisamide of the general formula IV

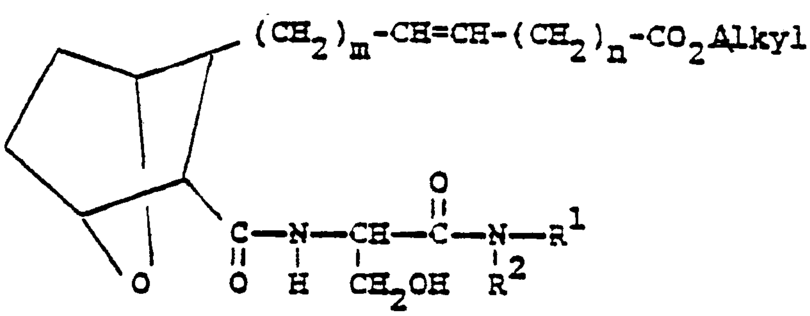

(IV)

is then subjected to a cyclodehydration reaction and the obtained oxazoline of the general formula V

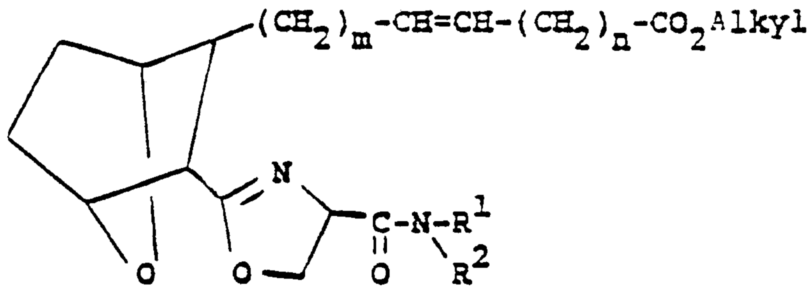

(V)

is oxidized to form an oxazol of the general formula IA′

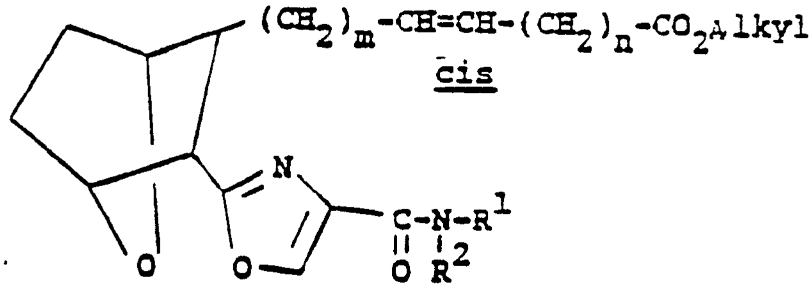

(IA')

b) alternatively for the production of an oxazol of the general formula IA′ an acid of the general formula III

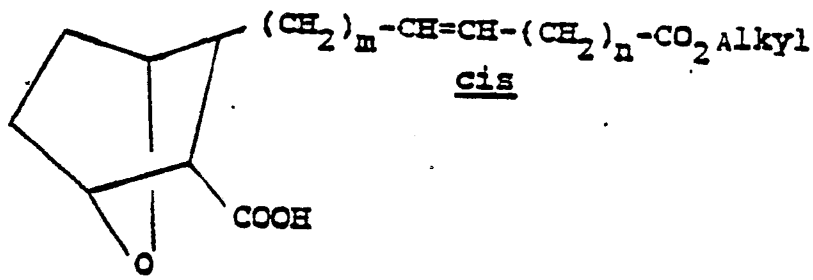

(III)

is subjected to a carbodiimide coupling reaction with a compound of the general formula A′

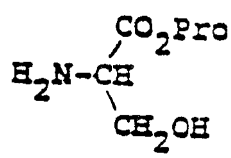

(A')

wherein Pro is a conventional protecting group, the obtained hydroxyamide of the general formula VI

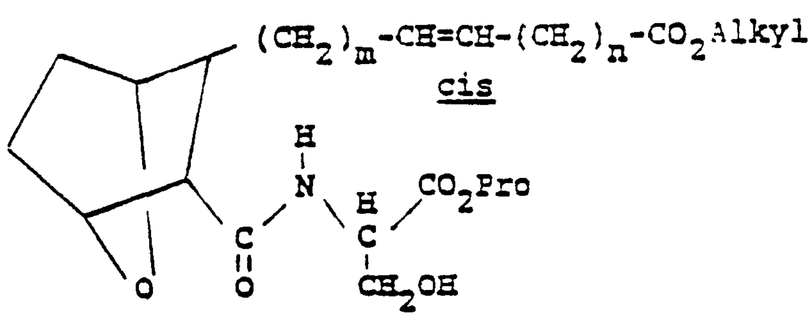

(VI)

is then subjected to a cyclodehydration and oxidation reaction as described above for the compounds of the formulae IV and V,

the obtained compound of the general formula VII

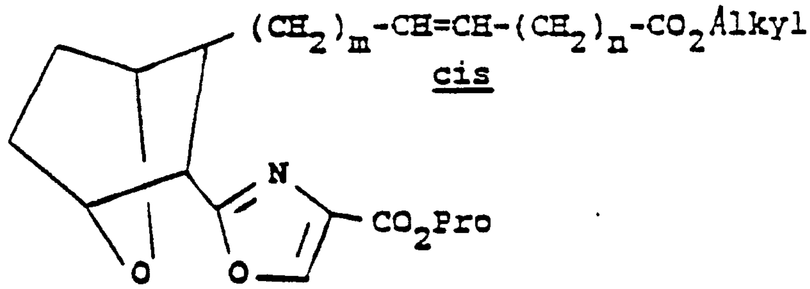

(VII)

is then subjected to a treatment to remove the protecting group,

the obtained compound of the general formula VIII

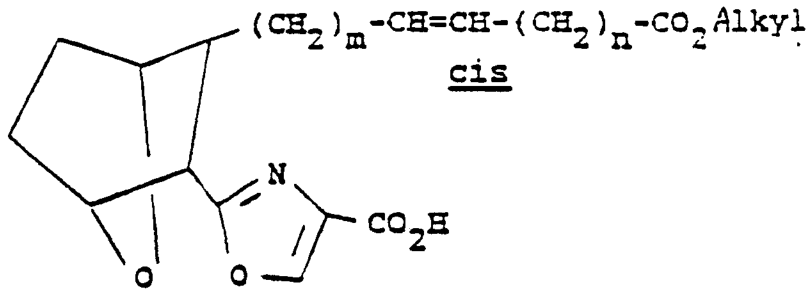

(VIII)

is reacted with an excess oxalylchloride and the obtained crude acid chloride of the general formula IX

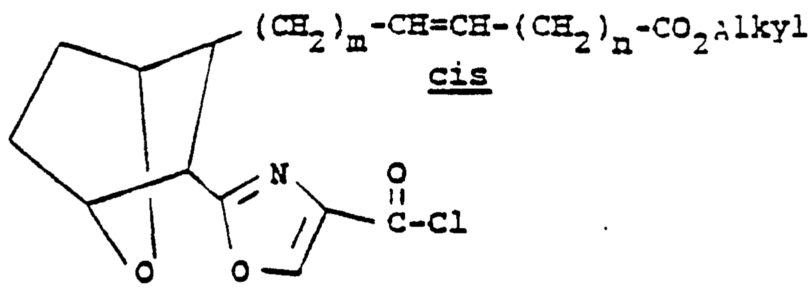

(IX)

is reacted with an amine hydrochloride of the general formula A″

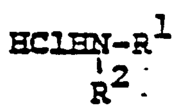

(A")

in the presence of an organic base to form a compound of the general formula IA′

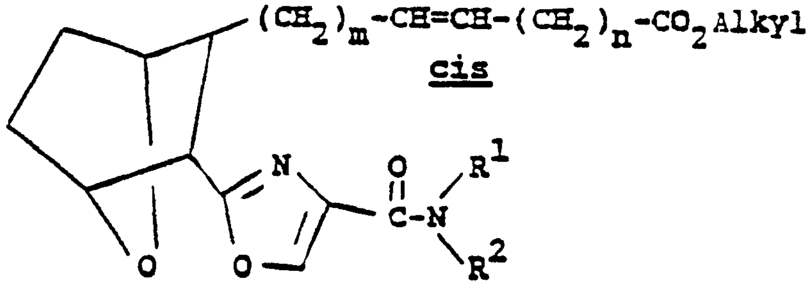

(IA')

c) for the preparation of compounds of the general formula I wherein Z represents -CH = CH- which is the trans double bond isomer, starting with a hydroxymethyl compound of the general formula II with a cis double bond which is protected with a protecting group, the protected compound with the general formula X is prepared

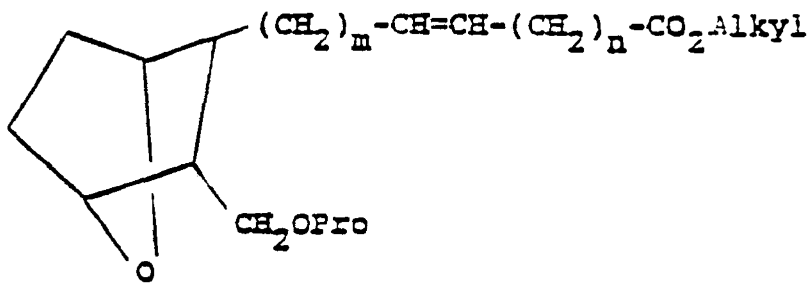

(X)

is treated with excess ozone,
the obtained aldehyde of the general formula XI

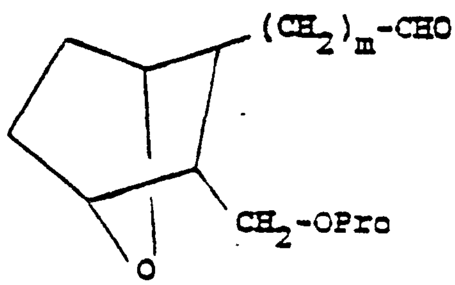

(XI)

is reacted to an ester of the general formula XII for the production of compounds wherein Z is -CH = CH- in the trans form and n is 2, the obtained ester of the general formula XII

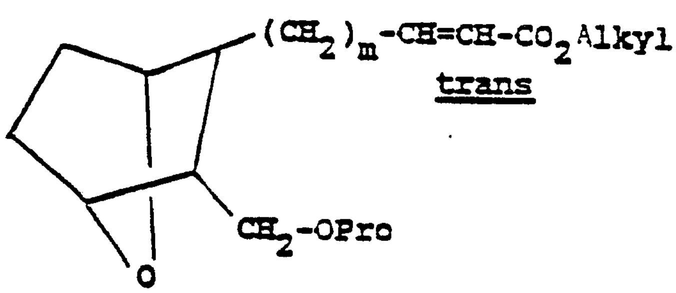

(XII)

is reacted to an alcohol,
the obtained alcohol of the general formula XIII

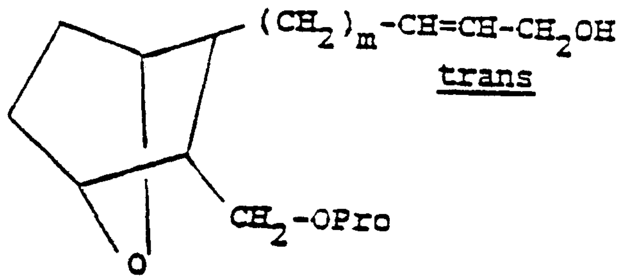

(XIII)

is reacted to form a bromide,
the obtained bromide of the general formula XIV

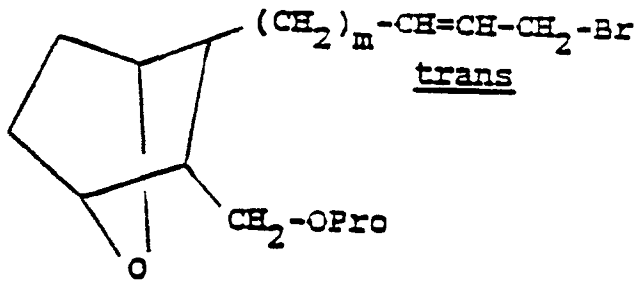

(XIV)

is reacted to an ester of the general formula XV wherein n is 2

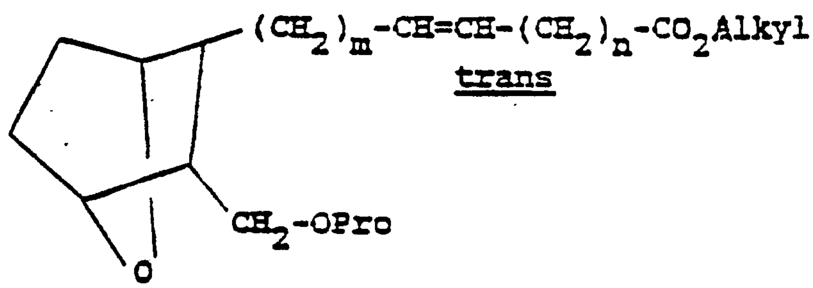

(XV)

for the production of compounds wherein 2 represents -CH=CH- in the trans form and n is 1, 3 or 4, an aldehyde of the general formula XI is reacted with a phosphonium salt of the general formula P

$$Br^{\ominus} \quad (C_6H_5)_3\overset{\oplus}{P}-(CH_2)_{n+1}-CH_2OH \qquad (P)$$

the obtained compound of the general formula XIII′

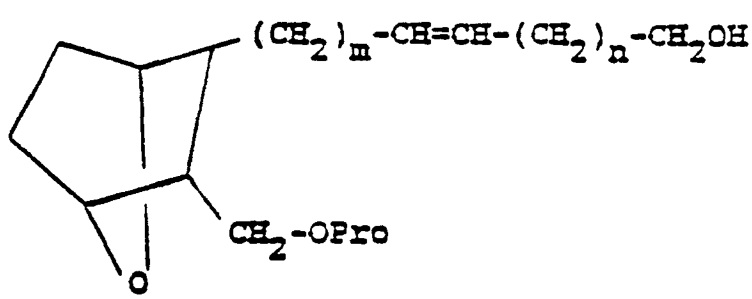

(XIII')

is oxidized and esterified using procedures known to those skilled in the art to form an ester of the general formula XV wherein n is 1, 3 or 4 and then the ester of the general formula XV is subjected to a treatment to remove the protecting group,
the obtained alcohol of the general formula XVI

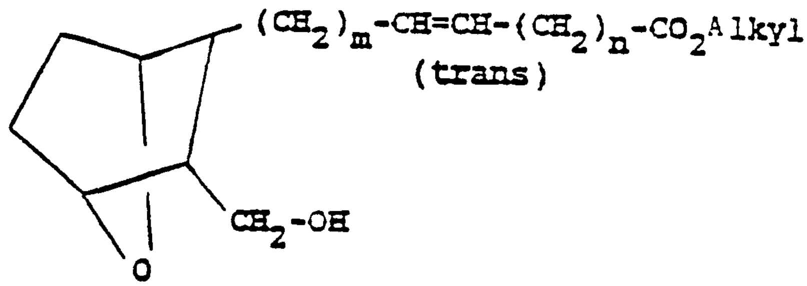

(XVI)

is used in place of a compound of the general formula II as a starting material following the procedure described hereinbefore and
the obtained compound of the general formula IIIA

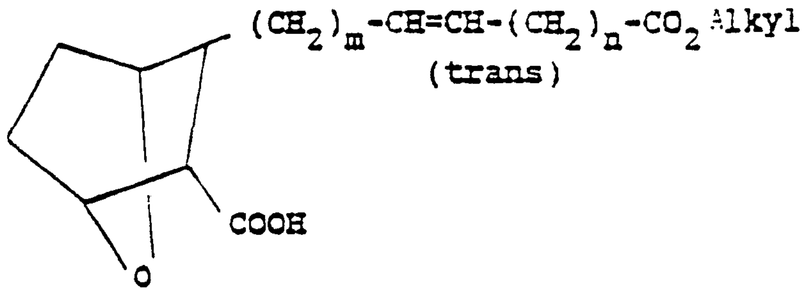

(IIIA)

is reacted to the trans compound of the general formula IA"

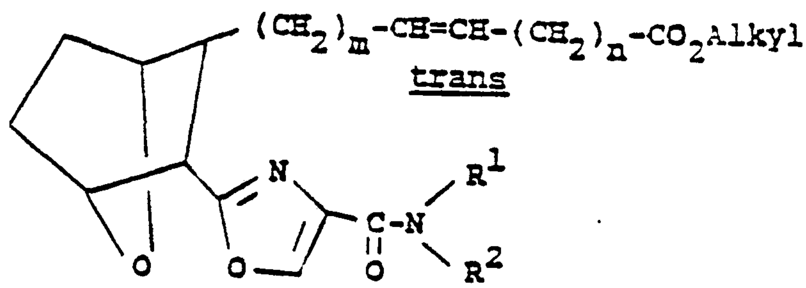

(IA")

d) for the production of compounds of the general formula IB

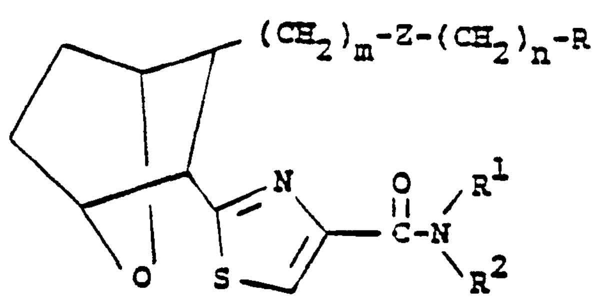

(IB)

wherein Z is -CH=CH- and X is S, an acid of the general formulae III or IIIA is reacted to form a chloride, the obtained acid chloride is amidated by reacting with ammonia or the acids of the formulae III or IIIA are reacted with an alkyl chloroformiate in the presence of an amine to form the mixed anhydrid and the

anhydrid is reacted with ammonia to the amide of the general formula XVII

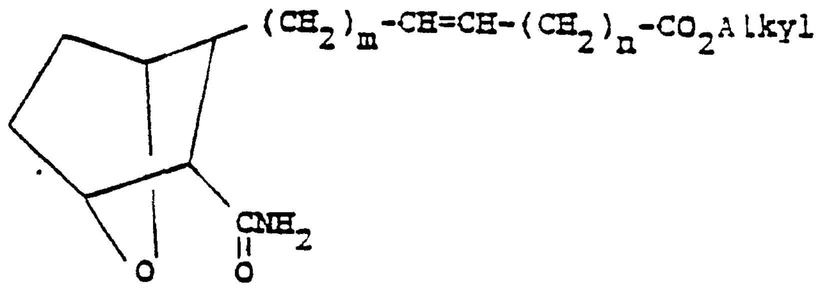

(XVII)

the amide of the general formula XVII is reacted with phosphorpentasulfid or Lawesson's reagent (2,4-bis-(4-methoxyphenyl)-1,3-dithia-2,4-diphosphetane-2,4-disulfide),
the obtained thioamide of the general formula XVIII

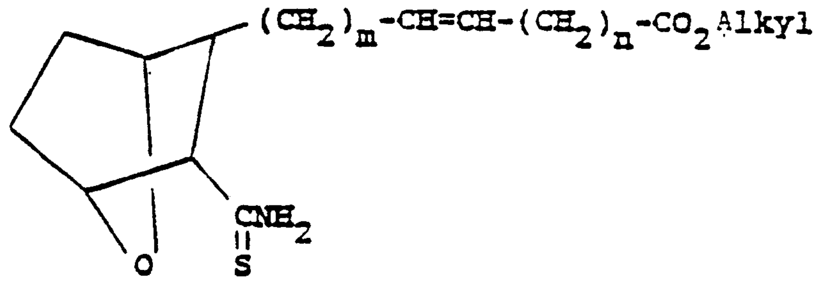

(XVIII)

is reacted with bromopyrovic acid of the formula
$(Br-CH_2-\overset{\overset{O}{\|}}{C}-CO_2H)$
the obtained thiazoine of the general formula XIX

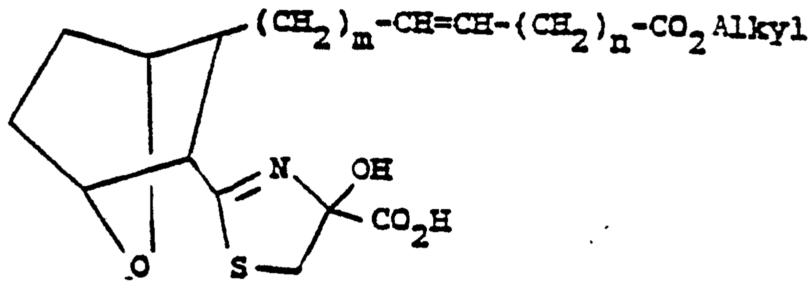

(XIX)

is then dehydrated and
the obtained thiazol acid of the general formula XX

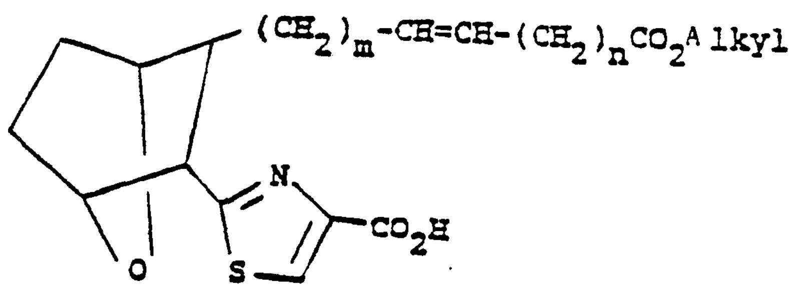

(XX)

is reacted by way of a carbodiimide coupling reaction with an amine of the general formula A'''

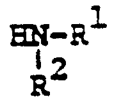

(A''')

to form an amide of the general formula $IB_a$

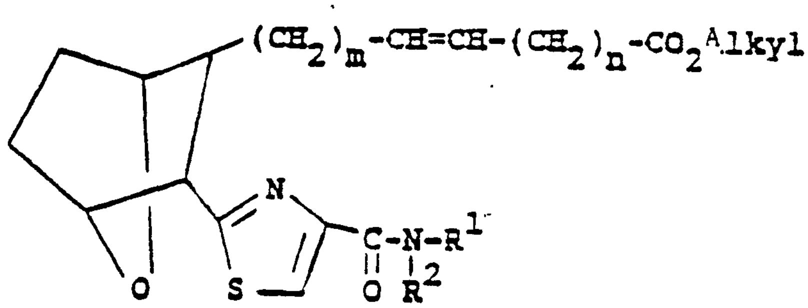

($IB_a$)

e) for the production of the compounds of the general formula IC

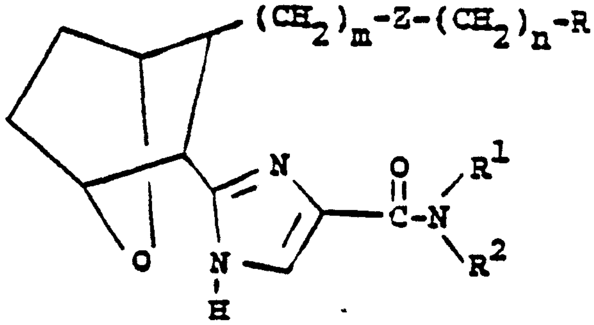

(IC)

wherein X represent NH, an acid of the general formulae III or IIa is subjected to a coupling reaction with an amine of the general formula B

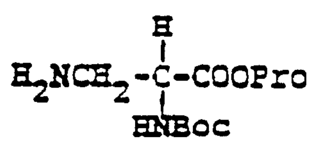

(B)

wherein Boc is t.-butyloxycarbonyl and Pro is a protecting group, and the obtained amide is subjected to a thionation reaction by treating with Lawesson's reagent,
the obtained ester of the general formula XXI

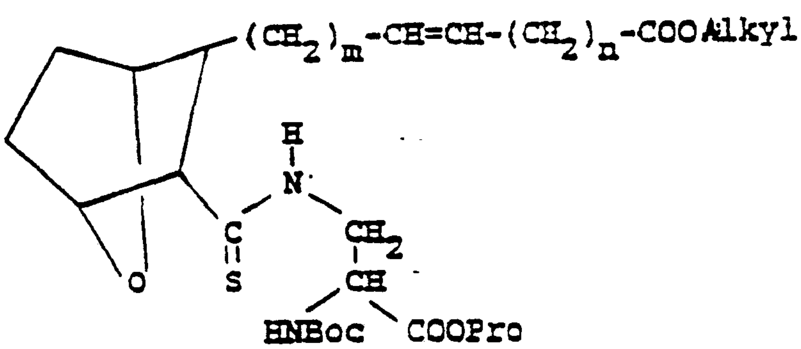

(XXI)

is subjected to a cyclisation reaction,
the obtained imidazoline of the general formula XXII

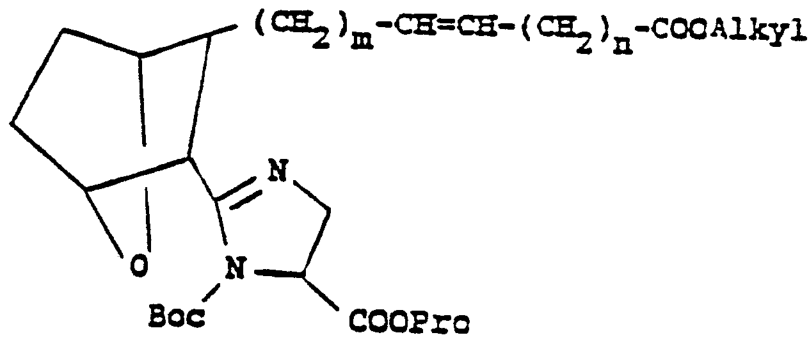

(XXII)

is subjected to a treatment to remove the protecting group, the obtained acid of the general formula XXIII

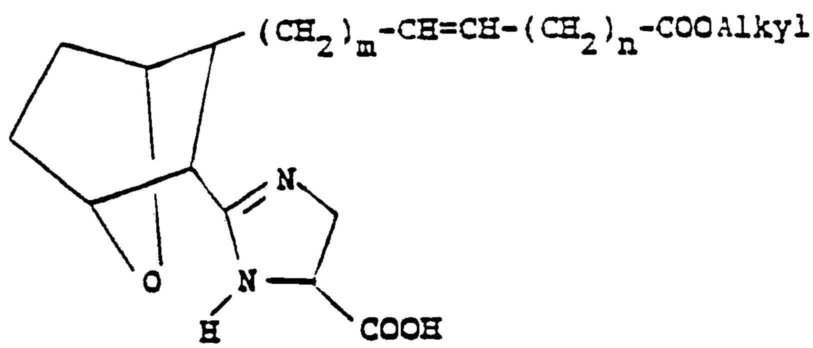

(XXIII)

is subjected to a coupling reaction with an amine of the general formula A‴ and the obtained amide of the general formula XXIV

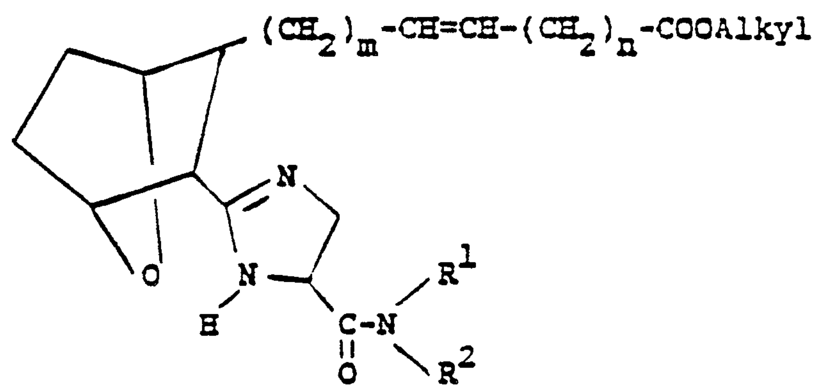

(XXIV)

is oxidized to form an ester of the general formula ICa

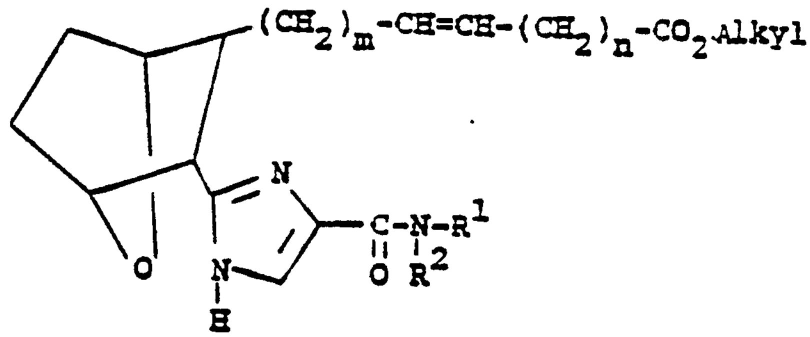

(ICa)

f) the esters of the general formulae IA′, IA″, IBa and ICa may be converted to the corresponding acids of the formula $I^1$

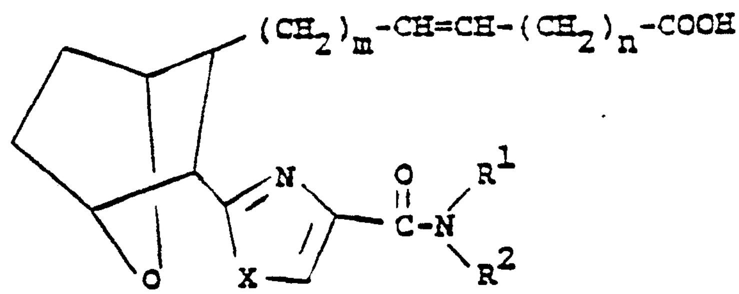

($I^1$)

g) for the production of compounds of the general formula I wherein Z represents $-(CH_2)-$, an acid of the general formula $I^1$ is hydrogenated to form an acid of the general formula $I^2$

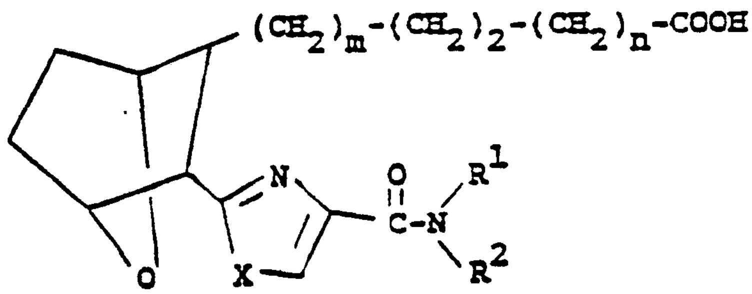

($I^2$)

h) for the production of compounds wherein R represents $CONHSO_2R^3$, i.e. compounds of the general formula $I^3$

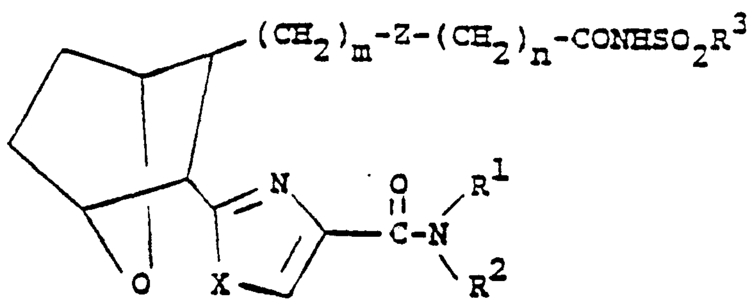

($I^3$)

an acid of the general formulae $I^1$ or $I^2$ is treated with a sulfonamide of the general formula C

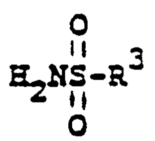

(C)

i) the acids of the general formulae $I^1$ or $I^2$ may be converted to the corresponding alkyl esters,

j) for the production of compounds wherein R represents $-CH_2$-5-tetrazolyl and Z is $-(CH_2)_2-$, i.e. compounds of the general formula $I^4$

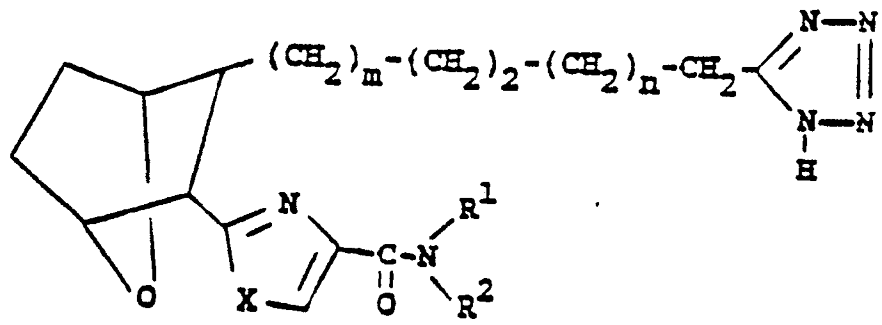

$(I^4)$

an ester of the general formulae IA′, IA″, IBa or ICa or an ester of the general formula $I^1$ is subjected to a reduction with a hydrid reagent,
the obtained alcohol of the general formula XXV

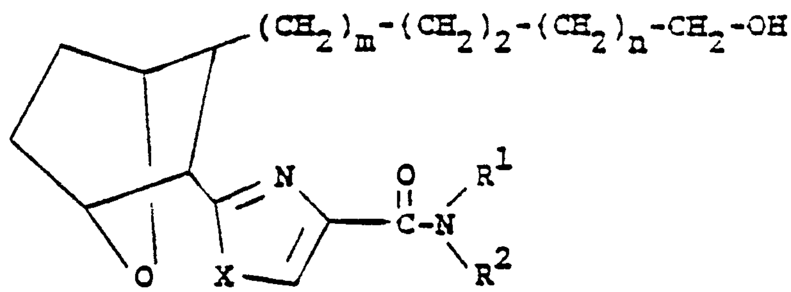

(XXV)

is subjected to a bromation reaction and the obtained bromide is converted to a nitril, the obtained nitril of the general formula XXVI

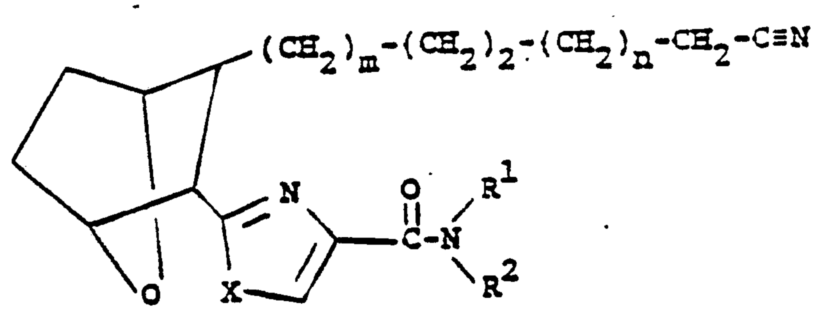

(XXVI)

is subjected to a cycloaddition reaction to form a compound of the general formula $I^4$,

k) for the production of compounds wherein R represents $-CH_2$-5-tetrazolyl and Z is -CH=CH-, i.e. compounds of the general formula $I^5$

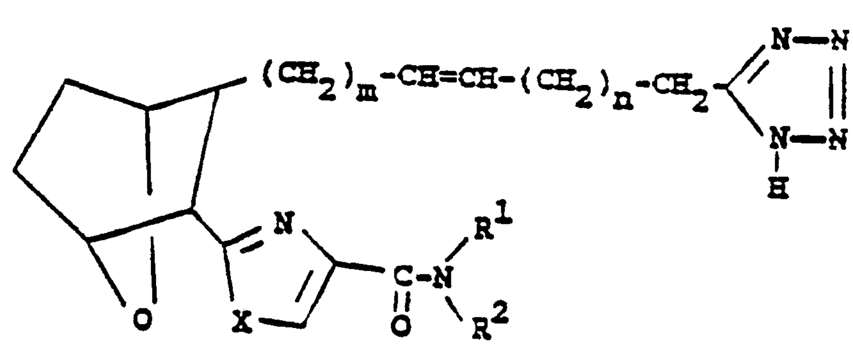

$(I^5)$

a hemiacetal of the formula D

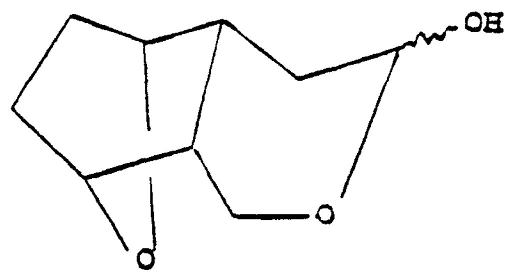

(D)

is reacted with a Wittig reagent of the general formula E

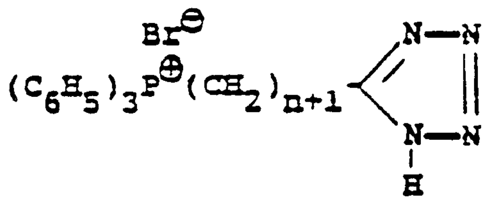

(E)

the obtained hydroxymethyl compound of the general formula XXVII

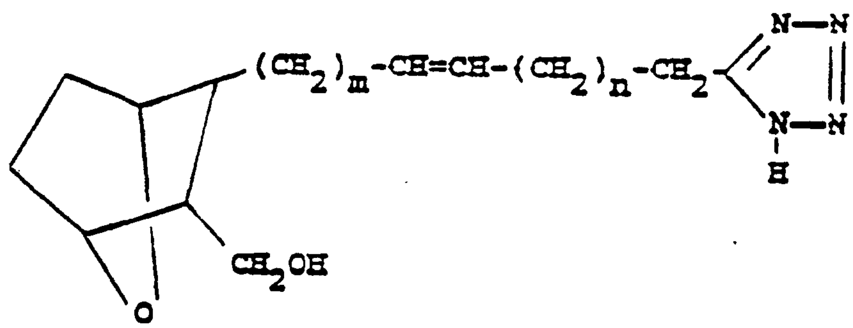

(XXVII)

is treated with a protecting compound F
Pro-Halogenid (F)
the protected tetrazol of the general formula XXVIII

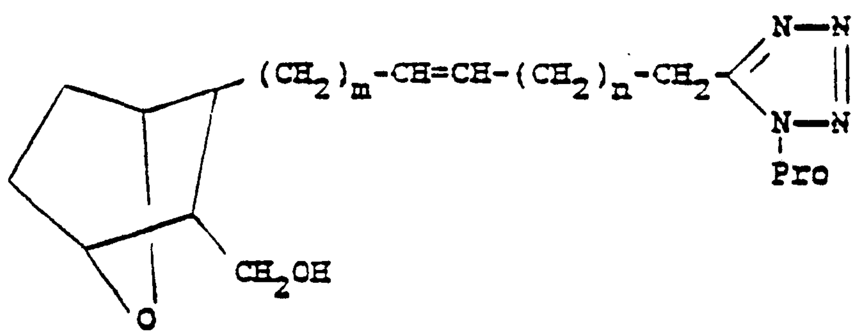

(XXVIII)

may be used in place of the hydroxymethyl compound of the general formula II to form the various compounds of the formula XXIX wherein X represents O, S or NH

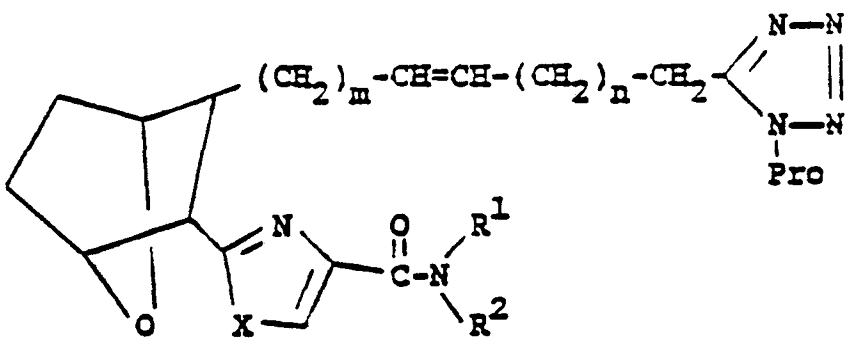

(XXIX)

and the compound of the general formula XXIX is reacted to form a compound of the general formula $I^5$,

l) for the production of compounds of the general formula I wherein R represents $CONHR^{3a}$ wherein $R^{3a}$ has another meaning than hydrogen, a corresponding acid of the general formula $I^6$

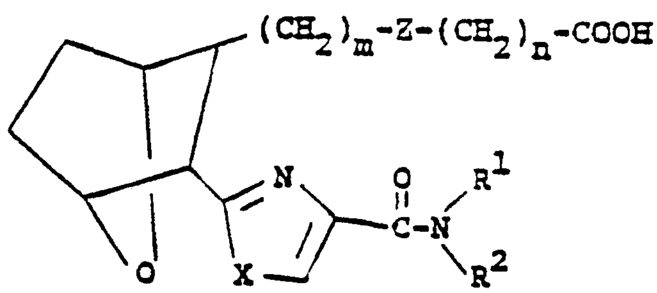

($I^6$)

is reacted with an amine of the general formula G

$HNHR^{3a}$(G)

to form an amide of the general formula $I^7$

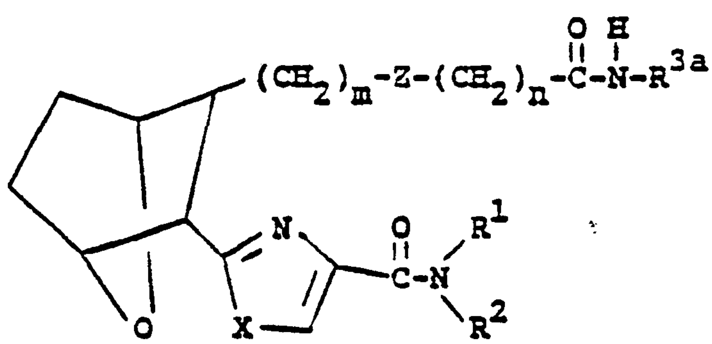

($I^7$)

wherein $R^{3a}$ represents lower alkyl, aryl or aralkyl,

m) for the production of compounds of the general formula I wherein R represents $CONH_2$, the corresponding acid of the general formula $I^6$ may be treated employing the procedure as described above for producing amide $I^7$ except that ammoniachloride is employed in the place of the amine G to form the amide of the invention $I^8$

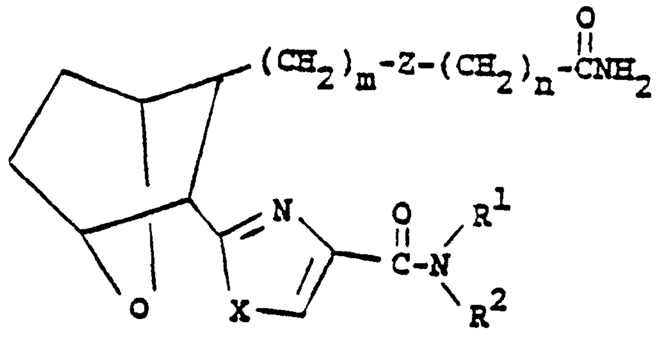

$(I^8)$

or

n) for the production of the compounds of the general formula I wherein R represents $CH_2OH$, the corresponding ester of the general formula $I^9$

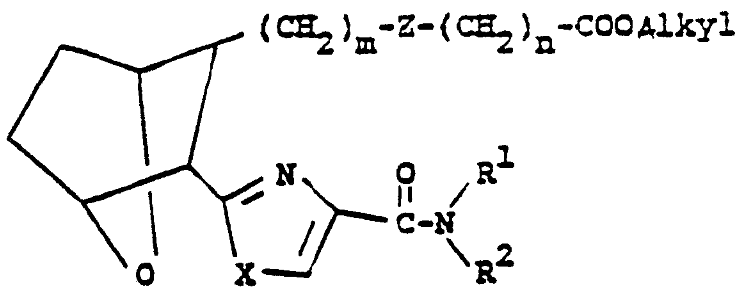

$(I^9)$

is treated with a reducing agent to form an alcohol of the general formula $I^{10}$

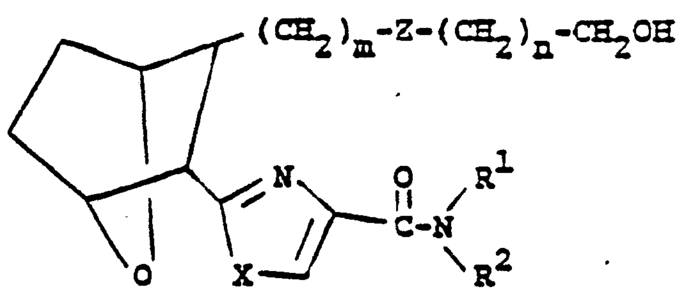

$(I^{10})$

2. The method for the production of a compound according to Claim 1 having the formula

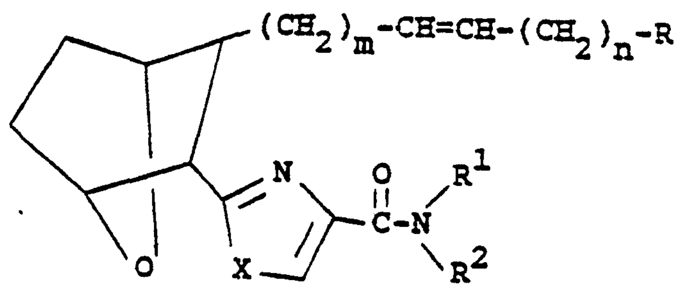

3. The method for the production of a compound according to Claim 2 having the formula

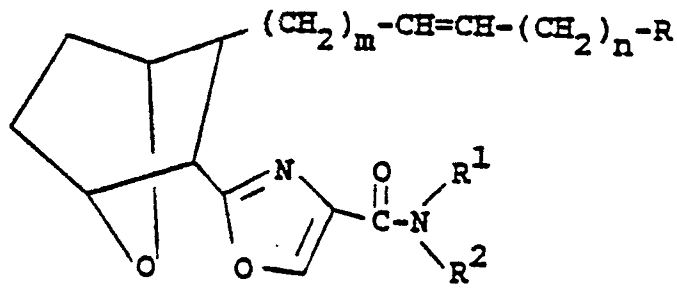

4. The method for the production of a compound according to Claim 2 where m = 1 and n = 2.

5. The method for the production of a compound according to Claim 1 having the formula

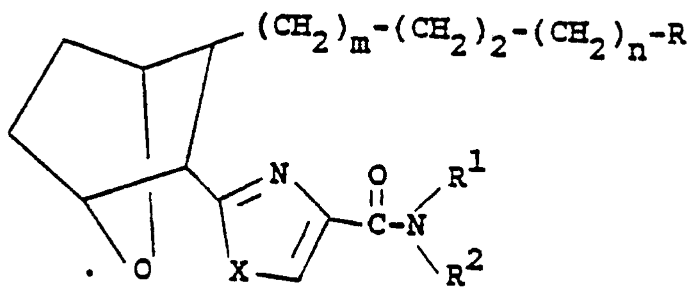

6. The method for the production of a compound according to Claim 5 where m = 1 and n = 2.

7. The method for the production of a compound according to Claim 5 having the formula

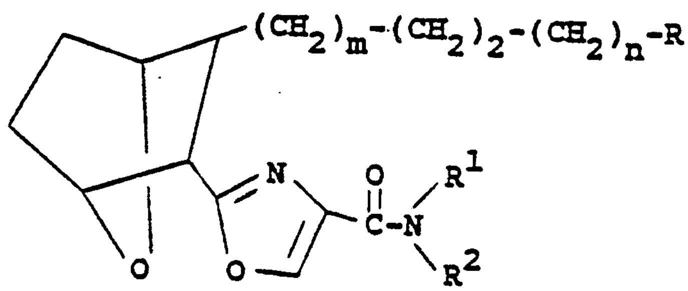

8. The method for the production of a compound according to Claim 1 having the formula

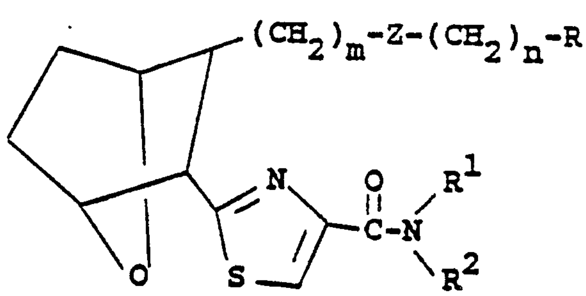

9. The method for the production of a compound according to Claim 1 having the formula

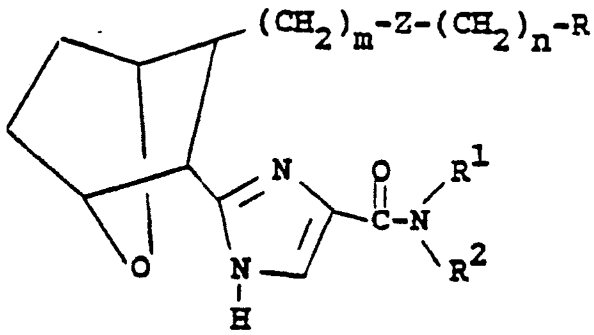

10. The method for the production of a compound according to Claim 1 wherein R is COH, $CONHSO_2R^3$ or $-CH_2$-5-tetrazolyl.

11. The method for the production of a compound according to Claim 1 having the formula

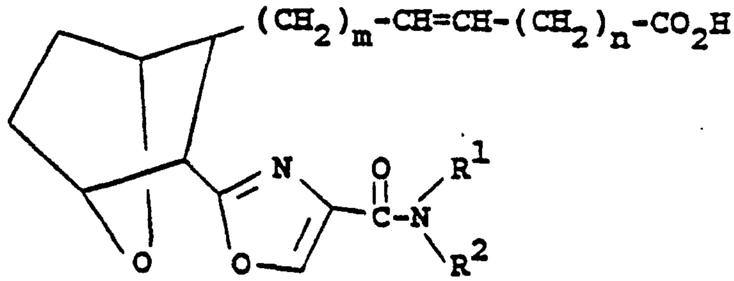

12. The method for the production of a compound according to Claim 1 having the name [1S-[1α,2α(Z),3α,4α]]-6-[3-[4-[[(4-cyclohexylbutyl)amino]carbonyl]-2-oxazolyl]-7-oxabicyclo-[2.2.1]hept-2-yl]-4-hexenoic acid, or esters or salts thereof; [1S-[1α,2α(Z),3α,4α]]-6-[3-[4[[(4-cyclohexylbutyl)-amino]carbonyl]-2-thiazolyl]-7-oxabicyclo[2.2.1]hept-2-yl-4-hexenoic acid, or esters or salts thereof; [1S-[1α,2α(Z),3α,4α]]-6-[3-[4[[(4-cyclohexylbutyl)methylamino]carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[(1-pyrrolidinyl)carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[-(cyclohexylamino)carbonyl]-2-oxazolyl]-7-oxabicyclo-[2.2.1]hept-2-yl]-4-hexenoic acid or esters or salts thereof; [1S-[1α,2α(Z),3α,4α]]-6-[3-[4-[[(2-cyclohexylethyl)amino]carbonyl-2-oxazolyl]-7-oxabicyclo[2.2.1]-hept-2-yl]-4-hexenoic aicd, or esters or salts thereof; [1S-[1α,2α(Z),3α,4α]]-6-[3-[4-[[[2-(4-chlorophenyl)-ethyl]amino]carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[[(4-chlorophenyl]amino]carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[[[4-(4-chlorophenyl)butyl]amino]-carbonyl]-2-oxazolyl]-7-oxabicyclo[2.2.1]hept-2-yl]-4-hexenoic acid, or esters or salts thereof; [1S-[1α,2α(Z)-,3α,4α]]-6-[3-[4a-[[(6-cyclohexylhexyl)amino]carbonyl]-2-oxazolyl]-7-oxabicyclo[2.2.1]hept-2-yl]-4-hexenoic acid, or esters, or salts thereof; [1S-[1α,2α(Z),3β,4α]]-6-[3- [4-[[(6-cyclohexylhexyl)amino]carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[(propylamino)carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[[(4-butylphenyl)amino]carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[(2,3-dihydro-1H-indol-1-yl)carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[[(4-cyclohexylbutyl)amino]carbonyl]-2-oxazolyl]-7-oxabicyclo[2.2.1]hept-2-yl]-N-(phenylsulfonyl)-4-hexenamide; [1S-[1α,2α(Z),3α,4α]]-6-[3-[4-[[(4-cyclohexylbutyl)amino]carbonyl]-2-oxazolyl]-N-(methylsulfonyl)-7-oxabicyclo[2.2.1]hept-2-yl]-4-hexenamide; [1S-[1α,2α(Z),3α,4α]]-7-[3-[4-[[(4-cyclohexylbutyl)amino]carbonyl]-2-oxazolyl]-7-oxabicyclo[2.2.1]hept-2-yl]-5-heptenoic acid, or esters or salts thereof; [1S[1α,2α(Z),3α,4α]]-6-[3-[4-[[(4-cyclohexylbutyl)amino]carbonyl]-1H-imidazol-2-yl]-7-oxabicyclo[2.2.1]hept-2-yl]-4-hexenoic acid or esters or salts thereof; [1S-[1α,2α,3α,4α]-6-[3-[4-[[(7,7-dimethyloctyl)amino]-carbonyl]-2-oxazolyl]-7-oxabicyclo[2.2.1]hept-2-yl]-4-hexenoic acid, or esters or salts thereof; [1S-[1α,2α(E)-,3α,4α]]-6-[3-[4[[(4-cyclohexylbutyl)amino]carbonyl]-2-oxazolyl]-7-oxabicyclo[2.2.1]hept-2-yl]-4-hexenoic acid; [1S-[1α,2α,3α,4α]]-3-[4-[[(4- cyclohexylbutyl]amino] carbonyl]-2-oxazolyl]-7-oxabicyclo[2.2.1]heptane-2-hexanoic acid or esters or salts thereof.

13. The method for the production of a compound according to Claim 1

having the name [1S-[1α,2α(Z),3α,4α]]-6-[3-[4-[[(4-cyclohexylbutyl)amino]carbonyl]-2-oxazolyl]-7-oxabicyclo-[2.2.1]hept-2-yl]-4-hexenoic acid, or esters or salts thereof.

Claims for the following Contracting State: GR

1. A compound having the formula I

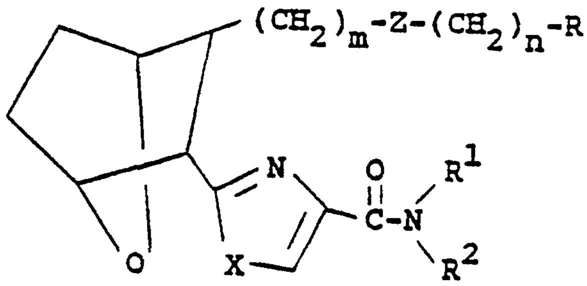

(I)

including all stereoisomers thereof, wherein
m is 1, 2 or 3;
n is 0, 1, 2, 3 or 4;
Z is $-(CH_2)_2-$ or -CH = CH-
with the proviso when Z is -CH = CH-, n is 1, 2, 3 or 4;
R is $CO_2H$, $CO_2$alkali metal, $CO_2$lower alkyl, $CH_2OH$, $CONHSO_2R^3$, $CONHR^{3a}$ or $-CH_2$-5-tetrazolyl;
X is O, S or NH;
$R^1$ is lower alkyl, lower alkenyl, lower alkynyl, aryl, aralkyl, cycloalkyl or cycloalkylalkyl;
$R^2$ is hydrogen, lower alkyl, aryl, or aralkyl;
or $R^1$ and $R^2$ together with the N to which they are linked form a 5- to 8- membered ring;
$R^3$ is lower alkyl, aryl or aralkyl; and
$R^{3a}$ is hydrogen, lower alkyl, aryl or aralkyl.

2. The compound according to Claim 1 having the formula

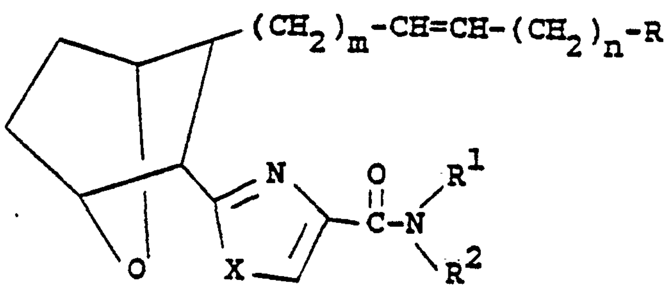

3. The compound according to Claim 2 having the formula

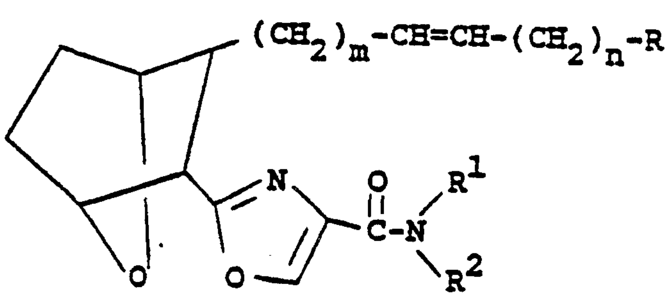

4. The compound according to Claim 2 where m = 1 and n = 2.

5. The compound according to Claim 1 having the formula

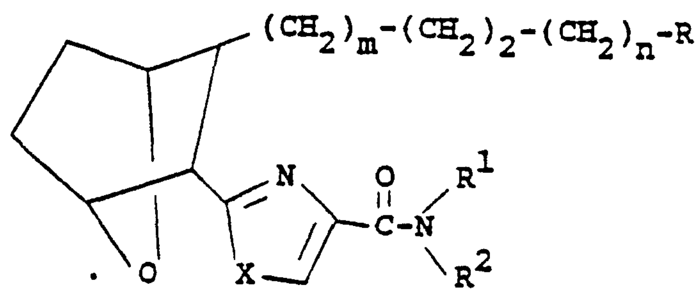

6. The compound according to Claim 5 where m = 1 and n = 2.

7. The compound according to Claim 5 having the formula

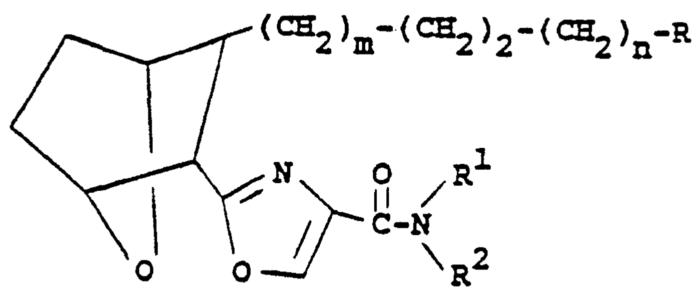

8. The compound according to Claim 1 having the formula

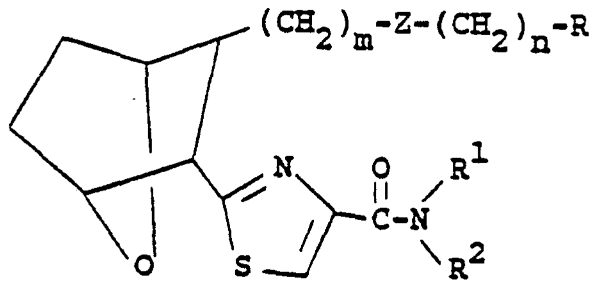

9. The compound according to Claim 1 having the formula

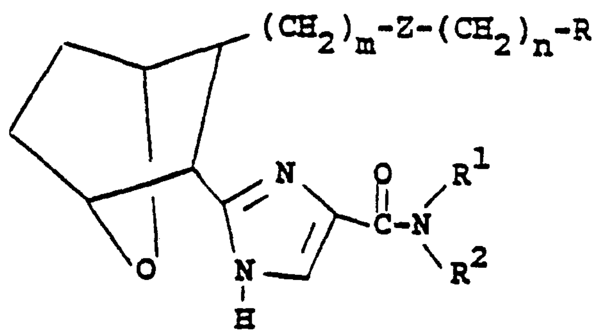

10. The compound according to Claim 1 wherein R is $CO_2H$, $CONHSO_2R^3$ or $-CH_2$-5-tetrazolyl.

11. The compound according to Claim 1 having the formula

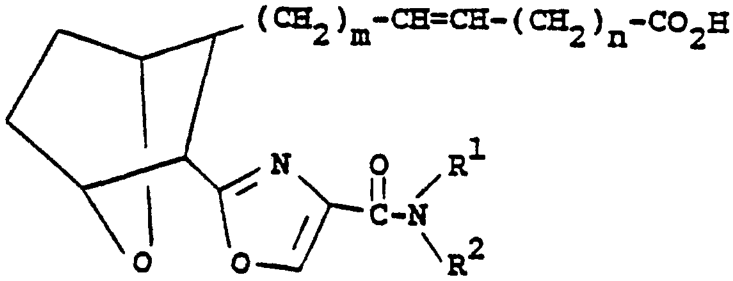

12. The compound according to Claim 1 having the name [1S-[1α,2α(Z),3α,4α]]-6-[3-[4-[[(4-cyclohexylbutyl)amino]carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[[(4-cyclohexylbutyl)amino]carbonyl]-2-thiazolyl]-7-oxabicyclo[2.2.1]hept-2-yl]-4-hexenoic acid, or esters or salts thereof; [1S[1α,2α(Z),3α,4α]]-6-[3-[4-[[(4-cyclohexylbutyl)methylamino]-carbonyl]-2-oxazolyl]-7-oxabicyclo[2.2.1]hept-2-yl]-4-hexenoic acid, or esters or salts thereof; [1S-[1α,2α(Z)-,3α,4α]]-6-[3-[4-[(1-pyrrolidinyl)carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[(cyclohexylamino)carbonyl]-2-oxazolyl]-7-oxabicyclo[2.2.1]-hept-2-yl]-4-hexenoic acid or esters or salts thereof; [1S-[1α,2α(Z),3α,4α]]-6-[3-[4-[[(2-cyclohexylethyl)-amino]carbonyl]-2-oxazolyl]-7-oxabicyclo[2.2.1]hept-2-yl]-4-hexenoic aicd, or esters or salts thereof; [1S-[1α,2α(Z),3α,4α]]-6-[3-[4-[[[2-(4-chlorophenyl)ethyl]amino]carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[[(4-chlorophenyl]amino]carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[[[4-(4-chlorophenyl)butyl]amino]carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4a-[[(6-cyclohexylhexyl)amino]carbonyl]-2-oxazolyl]-7-oxabicyclo[2.2.1]hept-2-yl]-4-hexenoic acid, or esters, or salts thereof; [1S-[1α,2α(Z),3β,4α]]-6-[3- [4-[[(6-cyclohexylhexyl)amino]carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[(propylamino)carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[[(4-butylphenyl)amino]carbonyl]-2-oxazolyl]-7-oxabicyclo[2.2.1]hept-2-yl]-4-hexenoic acid, or esters or salts thereof; [1S-[1α,2α(Z),3α,4α]]-6[3-[4-[(2,3-dihydro-1H-indol-1-yl)carbonyl]-2-oxazolyl]-7-oxabicyclo[2.2.1]hept-2-yl]-4-hexenoic acid, or esters or salts thereof; [1S-[1α,2α(Z),3α,4α]]-6-[3-[4-[[(4-cyclohexylbutyl)amino]carbonyl]-2-oxazolyl]-7-oxabicyclo-[2.2.1]hept-2-yl]-N-(phenylsulfonyl)-4-hexenamide; [1S-[1α,2α(Z),3α,4α]]-6-[3-[4-[[(4-cyclohexylbutyl)amino]-carbonyl]-2-oxazolyl]-N-(methylsulfonyl-7-oxabicyclo[2.2.1]hept-2-yl]-4-hexenamide; [1S-[1α,2α(Z),3α,4α]]-7-[3-[4-[[(4-cyclohexylbutyl)amino]carbonyl]-2-oxazolyl]-7-oxabicyclo[2.2.1]hept-2-yl]-5-heptenoic acid, or esters or salts thereof; [1S-[1α,2α(Z),3α,4α]]-6-[3-[4-[[(4-cyclohexylbutyl)amino]carbonyl]-1H-imidazol-2-yl]-7-oxabicyclo[2.2.1]hept-2-yl-4-hexenoic acid or esters or salts thereof; [1S-[1α,2α,3α,4α]-6-[3-[4-[[(7,7-dimethyloctyl)amino]carbonyl]-2-oxazolyl]-7-oxabicyclo[2.2.1]hept-2-yl]-4-hexenoic acid, or esters or salts thereof; [1S-[1α,2α(E),3α,4α]]-6-[3-[4-[[(4-cyclohexylbutyl)amino]carbonyl]-2-oxazolyl]-7-oxabicyclo[2.2.1]-hept-2-yl]-4-hexenoic acid; [1S-[1α,2α,3α,4α]]-3-[4-[[(4- cyclohexylbutyl)amino] carbonyl]-2-oxazolyl-7-oxabicyclo[2.2.1]heptane-2-hexanoic acid or esters or salts thereof.

13. The compound according to Claim 1 having the name [1S-[1α,2α(Z),3α,4α]]-6-[3-[4-[[(4-cyclohexylbutyl)amino]carbonyl]-2-oxazolyl]-7-oxabicyclo[2.2.1]hept-2-yl]-4-hexenoic acid, or esters or salts thereof.

14. A compound of the general formula I according to any one of claims 1 to 13 for use in a method of inhibiting platelet aggregation and bronchoconstriction.

15. A compound of the general formula I according to any one of claims 1 to 13 for use in a method for improving post-ischemic myocardial function.

16. A compound of the general formula I according to any one of claims 1 to 13 for use in a method for treating toxemia during pregnancy.

17. A compound of the general formula I according to any one of claims 1 to 13 for use in a method for preventing or reducing venous thrombosis.

18. A compound of the general formula I according to any one of claims 1 to 13 for use in a method for preventing or reducing platelet loss during extracorporeal circulation.

19. A compound of the general formula I according to any one of claims 1 to 13 for use in a method for treating burn injuries and/or promoting wound healing.

20. A compound of the general formula I according to any one of claims 1 to 13 for use in a method for reducing post-ischemic myocardial injury together with an effective amount of a thrombolytic agent within 6 hours of a myocardial infarction.

21. A compound of the general formula I according to any one of claims 1 to 13 for use in a method

according to claim 20 wherein said thrombolytic agent is t-PA, streptokinase, urokinase, prourokinase or anisoylated plasminogen-streptokinase activator complex.